# EUROPEAN PATENT APPLICATION

(11) **EP 3 778 646 A1**
(43) Date of publication of application: **17.02.2021**
(21) Application number: 20195355.1
(22) Date of filing: 01.03.2018
(51) Int. Cl.: C07K 16/30, C07K 16/32, A61K 39/395, G01N 33/53, A61P 35/00, A61K 39/00, C07K 14/725, C07K 16/18, C07K 16/28, G01N 33/574

(54) **T CELL RECEPTOR LIKE ANTIBODIES THAT BIND TO P53-MHC CLASS I COMPLEX**

(30) Priority: 08.03.2017 SG 10201701883R
(62) Divisional of application: 18763437.3
(71) Applicant: Agency for Science, Technology and Research, Singapore 138632 (SG)
(72) Inventor: WANG, Cheng-I, 138648 Singapore (SG); LOW, Lionel, 138648 Singapore (SG); GOH, Angeline, 138648 Singapore (SG); WANG, Bei, 138648 Singapore (SG); LEE, Wen-Hsin, 138648 Singapore (SG); HUANG, Ching-Wen, 138648 Singapore (SG)
(74) Representative: Mewburn Ellis LLP

(57) **Abstract**

Antibodies and fragments thereof which bind to peptide-MHC complexes are disclosed, in particular peptide-MHC complex comprising a peptide of p53 and MHC class I molecule. Also disclosed are compositions comprising such antibodies and fragments, and uses and methods using the same.

SEQUENCE LISTING

<110>  Agency for Science, Technology and Research

<120>  T Cell Receptor Like Antibodies

<130>  RIC/FP7336001

<150>  SG 10201701883R
<151>  2017-08-03

<160>  77

<170>  PatentIn version 3.5

<210>  1
<211>  217
<212>  PRT
<213>  Artificial Sequence

<220>
<223>  P1C1

<400>  1

Gln Ala Val Leu Thr Gln Pro Ser Ser Val Ser Gly Ala Pro Gly Gln
1               5                   10                  15

Arg Val Thr Ile Ser Cys Thr Gly Ser Ser Ser Asn Ile Gly Ala Asp
            20                  25                  30

Tyr Glu Thr His Trp Tyr Gln His Leu Pro Gly Thr Ala Pro Lys Leu
        35                  40                  45

Leu Ile Tyr Gly Asn Thr Asn Arg Pro Ser Gly Val Pro His Arg Phe
    50                  55                  60

Ser Gly Ser Lys Ser Gly Thr Ser Ala Ser Leu Ala Ile Thr Gly Leu
65                  70                  75                  80

Gln Ala Glu Asp Glu Ala Asp Tyr Tyr Cys Gln Ser Tyr Asp Ser Asn
                85                  90                  95

Leu Ser Ala Trp Val Phe Gly Gly Gly Thr Lys Leu Thr Ile Leu Ser
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Lys Ser Tyr Ser Cys Gln Val Thr His Glu Gly Ser Thr Val Glu
        195                 200                 205

Lys Thr Val Ala Pro Ala Glu Cys Ser
    210                 215

<210>  2
<211>  217
<212>  PRT
<213>  Artificial Sequence

<220>
<223>  P1C1_gl; P1C1_dm; 2E3; 1E11

<400>  2

Gln Ser Val Leu Thr Gln Pro Pro Ser Val Ser Gly Ala Pro Gly Gln
1               5                   10                  15

Arg Val Thr Ile Ser Cys Thr Gly Ser Ser Ser Asn Ile Gly Ala Asp
            20                  25                  30

Tyr Glu Thr His Trp Tyr Gln Gln Leu Pro Gly Thr Ala Pro Lys Leu
        35                  40                  45

Leu Ile Tyr Gly Asn Thr Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Glu Ala Asp Tyr Tyr Cys Gln Ser Tyr Asp Ser Asn
                85                  90                  95

Leu Ser Ala Trp Val Phe Gly Gly Gly Thr Lys Leu Thr Ile Leu Ser
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Lys Ser Tyr Ser Cys Gln Val Thr His Glu Gly Ser Thr Val Glu
        195                 200                 205

Lys Thr Val Ala Pro Ala Glu Cys Ser
    210                 215

<210>  3
<211>  217
<212>  PRT
<213>  Artificial Sequence

<220>
<223>  1G7; P1C1_tm

<400>  3

Gln Ser Val Leu Thr Gln Pro Pro Ser Val Ser Gly Ala Pro Gly Gln
1               5                   10                  15

Arg Val Thr Ile Ser Cys Ala Gly Ser Tyr Ser Asn Ile Gly Asp Asp
            20                  25                  30

Tyr Glu Thr His Trp Tyr Gln Gln Leu Pro Gly Thr Ala Pro Lys Leu
        35                  40                  45

Leu Ile Tyr Gly Asn Thr Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Glu Ala Asp Tyr Tyr Cys Gln Ser Tyr Asp Ser Asn
                85                  90                  95

Leu Ser Ala Trp Val Phe Gly Gly Gly Thr Lys Leu Thr Ile Leu Ser
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Lys Ser Tyr Ser Cys Gln Val Thr His Glu Gly Ser Thr Val Glu
        195                 200                 205

Lys Thr Val Ala Pro Ala Glu Cys Ser
    210                 215

<210>  4
<211>  214
<212>  PRT
<213>  Artificial Sequence

<220>
<223>  P1H4

<400>  4

Glu Ile Val Met Thr Gln Ser Pro Gly Thr Leu Ser Leu Ser Pro Gly
1               5                   10                  15

Glu Arg Ala Thr Leu Ser Cys Arg Ala Ser Gln Ser Ile Gly Thr Asp
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Pro
                85                  90                  95

Thr Phe Gly Gly Ala Thr Lys Val Glu Ile Lys Arg Thr Val Ala Ala
            100                 105                 110

Pro Ser Val Phe Ile Phe Pro Pro Ser Asp Glu Gln Leu Lys Ser Gly

115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  5
<211>  218
<212>  PRT
<213>  Artificial Sequence

<220>
<223>  P1B11

<400>  5

Gln Ala Val Leu Thr Gln Pro Ser Ser Val Ser Gly Ala Pro Gly Gln
1               5                   10                  15

Arg Val Thr Ile Ser Cys Thr Gly Ser Ser Ser Asn Ile Gly Ala Gly
            20                  25                  30

Tyr Asp Val His Trp Tyr Gln Gln Leu Pro Gly Thr Ala Pro Lys Leu
        35                  40                  45

Leu Ile Tyr Gly Asn Asn Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Glu Ala Asp Tyr Tyr Cys Gln Ser Tyr Asp Ser Asn
                85                  90                  95

Leu Ser Asp Thr Trp Val Phe Gly Gly Gly Thr Lys Leu Thr Val Leu
            100                 105                 110

Gly Gln Pro Lys Ala Ala Pro Ser Val Thr Leu Phe Pro Pro Ser Ser
        115                 120                 125

Glu Glu Leu Gln Ala Asn Lys Ala Thr Leu Val Cys Leu Ile Ser Asp
    130                 135                 140

Phe Tyr Pro Gly Ala Val Thr Val Ala Trp Lys Ala Asp Ser Ser Pro
145                 150                 155                 160

Val Lys Ala Gly Val Glu Thr Thr Thr Pro Ser Lys Gln Ser Asn Asn
                165                 170                 175

Lys Tyr Ala Ala Ser Ser Tyr Leu Ser Leu Thr Pro Glu Gln Trp Lys
            180                 185                 190

Ser His Lys Ser Tyr Ser Cys Gln Val Thr His Glu Gly Ser Thr Val
        195                 200                 205

Glu Lys Thr Val Ala Pro Thr Glu Cys Ser
    210                 215

<210> 6
<211> 217
<212> PRT
<213> Artificial Sequence

<220>
<223> P1A8

<400> 6

Gln Ser Val Leu Thr Gln Pro Pro Ser Val Ser Gly Ala Pro Gly Gln
1               5                   10                  15

Arg Val Thr Ile Ser Cys Thr Gly Ser Ser Ser Asn Ile Gly Ala Gly
            20                  25                  30

Tyr Asp Val Asn Trp Tyr Gln Gln Leu Pro Gly Thr Ala Pro Lys Leu
        35                  40                  45

Leu Ile Tyr Gly Asn Asn Asn Arg Pro Ser Gly Val Pro Asp Arg Phe
    50                  55                  60

Ser Gly Ser Asn Ser Gly Thr Ser Val Ser Leu Ala Ile Thr Gly Leu
65                  70                  75                  80

Gln Pro Glu Asp Glu Ala Asp Tyr Tyr Cys Gln Ser Tyr Asp Ser Ser
                85                  90                  95

Leu Ser Ala Trp Val Phe Gly Gly Gly Thr Lys Leu Ala Val Leu Gly
                100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Gly Ser Pro Val
145                 150                 155                 160

Lys Ala Gly Val Glu Thr Thr Lys Pro Ser Lys Gln Ser Asn Asn Lys
                165                 170                 175

Tyr Ala Ala Ser Ser Tyr Leu Ser Leu Thr Pro Glu Gln Trp Lys Ser
                180                 185                 190

His Lys Ser Tyr Ser Cys Gln Val Thr His Glu Gly Ser Thr Val Glu
        195                 200                 205

Lys Thr Val Ala Pro Ala Glu Cys Ser
    210                 215

<210>  7
<211>  216
<212>  PRT
<213>  Artificial Sequence

<220>
<223>  P2B4

<400>  7

Gln Ser Ala Leu Thr Gln Pro Ala Ser Val Ser Gly Ser Leu Gly Gln
1               5                   10                  15

Ser Ile Thr Ile Ser Cys Thr Gly Thr Ser Ser Asp Val Gly Gly Tyr
            20                  25                  30

Asn Tyr Val Ser Trp Tyr Gln Gln Gln Ala Gly Lys Ala Pro Lys Leu
        35                  40                  45

Met Ile Tyr Asp Val Ser Ser Arg Pro Ser Gly Val Ser Asp Arg Phe
    50                  55                  60

Ser Gly Ser Lys Ser Gly Asn Thr Ala Ser Leu Thr Ile Ser Gly Leu
65                  70                  75                  80

Gln Ala Asp Asp Glu Ala Asp Tyr Tyr Cys Ser Ser Tyr Thr Val Phe
                85                  90                  95

Ser Thr Leu Val Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Gly Ser Pro Val Lys
145                 150                 155                 160

Ala Gly Val Glu Thr Thr Lys Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Ala Glu Cys Ser
    210                 215

<210>  8
<211>  118
<212>  PRT
<213>  Artificial Sequence

<220>
<223>  P1C1

<400>  8

Gln Leu Gln Leu Gln Glu Ser Gly Pro Gly Leu Val Lys Pro Ser Gln
1               5                   10                  15

Thr Leu Ser Leu Thr Cys Ala Val Ser Gly Gly Ser Ile Ser Ser Gly
            20                  25                  30

Gly Tyr Tyr Trp Ser Trp Ile Arg Gln Arg Pro Gly Lys Gly Leu Glu
        35                  40                  45

Trp Ile Gly Tyr Ile Tyr Tyr Ser Gly Ser Thr Tyr Tyr Asn Pro Ser
    50                  55                  60

Leu Lys Ser Arg Leu Thr Ile Ser Val Asp Thr Ser Lys Asn Gln Phe

65                  70                  75                  80

Ser Leu Lys Leu Ser Ser Val Thr Ala Ala Asp Thr Ala Val Tyr Tyr
                85                  90                  95

Cys Ala Arg Glu Asn Phe Gly Ala Phe Asp His Trp Gly Gln Gly Thr
            100                 105                 110

Leu Val Thr Val Ser Ser
        115

<210>  9
<211>  118
<212>  PRT
<213>  Artificial Sequence

<220>
<223>  P1C1_gl; 1G7

<400>  9

Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Gly Tyr Tyr Trp Ser Trp Ile Arg Gln His Pro Gly Lys Gly Leu Glu
        35                  40                  45

Trp Ile Gly Tyr Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Glu Asn Phe Gly Ala Phe Asp His Trp Gly Gln Gly Thr
            100                 105                 110

Leu Val Thr Val Ser Ser
        115

<210>  10
<211>  118
<212>  PRT
<213>  Artificial Sequence

<220>

<223>  2E3

<400>  10

Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Gly Tyr Tyr Trp Ala Trp Ile Arg Gln His Pro Gly Lys Gly Leu Glu
        35                  40                  45

Trp Ile Gly Tyr Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Glu Asn Phe Gly Ala Phe Asp His Trp Gly Gln Gly Thr
            100                 105                 110

Leu Val Thr Val Ser Ser
        115


<210>  11
<211>  118
<212>  PRT
<213>  Artificial Sequence

<220>
<223>  1E11

<400>  11

Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Gly Tyr Tyr Trp Ser Trp Ile Arg Gln His Pro Gly Lys Gly Leu Glu
        35                  40                  45

Trp Ile Gly Tyr Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe

65                  70                  75                  80

Ser Leu Lys Leu Ser Ser Val Thr Ala Ala Asp Thr Ala Val Tyr Tyr
                85                  90                  95

Cys Ala Arg Glu Asn Phe Gly Ser Tyr Asp Tyr Trp Gly Gln Gly Thr
            100                 105                 110

Leu Val Thr Val Ser Ser
        115

<210>  12
<211>  118
<212>  PRT
<213>  Artificial Sequence

<220>
<223>  P1C1_dm; P1C1_tm

<400>  12

Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Gly Tyr Tyr Trp Ala Trp Ile Arg Gln His Pro Gly Lys Gly Leu Glu
        35                  40                  45

Trp Ile Gly Tyr Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Glu Asn Phe Gly Ser Tyr Asp Tyr Trp Gly Gln Gly Thr
            100                 105                 110

Leu Val Thr Val Ser Ser
        115

<210>  13
<211>  120
<212>  PRT
<213>  Artificial Sequence

<220>

<223>  P1H4

<400>  13

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Ala Gly Thr Asn Tyr Ala Gln Lys Phe
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Glu Asp Thr Ala Val Tyr Tyr Cys
                85                  90                  95

Ala Arg Glu Gly Ala Asp Gly Ile Tyr Tyr Phe Asp Tyr Trp Gly Gln
            100                 105                 110

Gly Thr Leu Val Thr Val Ser Ser
        115                 120


<210>  14
<211>  118
<212>  PRT
<213>  Artificial Sequence

<220>
<223>  P1B11

<400>  14

Gln Val Gln Leu Gln Glu Ser Gly Pro Gly Leu Val Lys Pro Ser Glu
1               5                   10                  15

Thr Leu Ser Leu Thr Cys Ser Val Ser Gly Asp Ser Ile Ser Ser Gly
            20                  25                  30

Gly Tyr Tyr Trp Ser Trp Ile Arg Gln His Pro Glu Lys Gly Leu Glu
        35                  40                  45

Trp Ile Gly Tyr Ile Tyr Tyr Ser Gly Thr Thr Tyr Tyr Asn Pro Ser
    50                  55                  60

Leu Lys Ser Arg Ile Thr Met Ser Ile Asp Thr Ser Lys Asn Gln Phe

65                  70                  75                  80

Ser Leu Lys Val Ser Ser Val Thr Ala Ala Asp Thr Ala Val Tyr Tyr
                85                  90                  95

Cys Ala Arg Glu Asn Phe Gly Ala Phe Asp Tyr Trp Gly Gln Gly Thr
            100                 105                 110

Leu Val Thr Val Ser Ser
        115

<210>  15
<211>  118
<212>  PRT
<213>  Artificial Sequence

<220>
<223>  P1A8

<400>  15

Gln Leu Gln Leu Gln Glu Ser Gly Pro Gly Leu Val Lys Pro Ser Gln
1               5                   10                  15

Thr Leu Ser Leu Thr Cys Ser Val Ser Gly Gly Ser Ile Ser Ser Gly
            20                  25                  30

Gly Tyr Tyr Trp Ser Trp Ile Arg Gln Arg Pro Gly Lys Gly Leu Glu
        35                  40                  45

Trp Ile Gly Tyr Ile Tyr Tyr Ser Gly Ser Thr Tyr Tyr Asn Pro Ser
    50                  55                  60

Leu Lys Ser Arg Leu Thr Ile Ser Val Asp Thr Ser Lys Asn Gln Leu
65                  70                  75                  80

Ser Leu Lys Leu Ser Ser Val Thr Ala Ala Asp Thr Ala Val Tyr Tyr
                85                  90                  95

Cys Ala Arg Glu Asn Phe Gly Ala Phe Asp His Trp Gly Gln Gly Thr
            100                 105                 110

Leu Val Thr Val Ser Ser
        115

<210>  16
<211>  116
<212>  PRT
<213>  Artificial Sequence

<220>

<223>  P2B4

<400>  16

Gln Val Gln Leu Val Gln Ser Gly Ala Glu Val Lys Lys Pro Gly Thr
1               5                   10                  15

Ser Val Thr Val Ser Cys Arg Ala Ser Gly Phe Thr Phe Ser Asp Tyr
            20                  25                  30

Tyr Ile His Trp Val Arg Gln Ala Pro Gly Gln Gly Leu Glu Trp Met
        35                  40                  45

Gly Trp Met Ser Pro Asp Ser Gly Ala Thr Tyr Tyr Ala Gln Lys Phe
    50                  55                  60

Gln Gly Arg Val Thr Met Thr Arg Asp Thr Ser Thr Asn Thr Ala Tyr
65                  70                  75                  80

Met Glu Leu Ser Arg Leu Thr Ser Asp Asp Thr Ala Val Tyr Tyr Cys
                85                  90                  95

Val Arg Asp Thr Tyr Gly His Asp Tyr Trp Gly Gln Gly Thr Leu Val
            100                 105                 110

Thr Val Ser Ser
        115

<210>  17
<211>  14
<212>  PRT
<213>  Artificial Sequence

<220>
<223>  P1C1; P1C1_gl; P1C1_dm; 2E3; 1E11 LC-CDR1

<400>  17

Thr Gly Ser Ser Ser Asn Ile Gly Ala Asp Tyr Glu Thr His
1               5                   10

<210>  18
<211>  7
<212>  PRT
<213>  Artificial Sequence

<220>
<223>  P1C1; P1C1_gl; P1C1_dm; 2E3; 1E11 LC-CDR2

<400>  18

Gly Asn Thr Asn Arg Pro Ser
1               5

<210>  19
<211>  11
<212>  PRT
<213>  Artificial Sequence

<220>
<223>  P1C1; P1C1_gl; P1C1_dm; 2E3; 1E11 LC-CDR3

<400>  19

Gln Ser Tyr Asp Ser Asn Leu Ser Ala Trp Val
1               5                   10


<210>  20
<211>  14
<212>  PRT
<213>  Artificial Sequence

<220>
<223>  1G7; P1C1_tm LC-CDR1

<400>  20

Ala Gly Ser Tyr Ser Asn Ile Gly Asp Asp Tyr Glu Thr His
1               5                   10


<210>  21
<211>  11
<212>  PRT
<213>  Artificial Sequence

<220>
<223>  P1H4 LC-CDR1

<400>  21

Arg Ala Ser Gln Ser Ile Gly Thr Asp Leu Ala
1               5                   10


<210>  22
<211>  7
<212>  PRT
<213>  Artificial Sequence

<220>
<223>  P1H4 LC-CDR2

<400>  22

Asp Ala Ser Asn Arg Ala Thr
1               5


<210>  23
<211>  9
<212>  PRT
<213>  Artificial Sequence

<220>

<223>  P1H4 LC-CDR3

<400>  23

Gln Gln Arg Ser Asn Trp Pro Pro Thr
1               5


<210>  24
<211>  14
<212>  PRT
<213>  Artificial Sequence

<220>
<223>  P1B11 LC-CDR1

<400>  24

Thr Gly Ser Ser Ser Asn Ile Gly Ala Gly Tyr Asp Val His
1               5                   10


<210>  25
<211>  7
<212>  PRT
<213>  Artificial Sequence

<220>
<223>  P1B11 LC-CDR2

<400>  25

Gly Asn Asn Asn Arg Pro Ser
1               5


<210>  26
<211>  12
<212>  PRT
<213>  Artificial Sequence

<220>
<223>  P1B11 LC-CDR3

<400>  26

Gln Ser Tyr Asp Ser Asn Leu Ser Asp Thr Trp Val
1               5                   10


<210>  27
<211>  14
<212>  PRT
<213>  Artificial Sequence

<220>
<223>  P1A8 LC-CDR1

<400>  27

Thr Gly Ser Ser Ser Asn Ile Gly Ala Gly Tyr Asp Val Asn
1               5                   10

<210>  28
<211>  11
<212>  PRT
<213>  Artificial Sequence

<220>
<223>  P1A8 LC-CDR3

<400>  28

Gln Ser Tyr Asp Ser Ser Leu Ser Ala Trp Val
1               5                   10


<210>  29
<211>  14
<212>  PRT
<213>  Artificial Sequence

<220>
<223>  P2B4 LC1-CDR1

<400>  29

Thr Gly Thr Ser Ser Asp Val Gly Gly Tyr Asn Tyr Val Ser
1               5                   10


<210>  30
<211>  7
<212>  PRT
<213>  Artificial Sequence

<220>
<223>  P2B4 LC-CDR2

<400>  30

Asp Val Ser Ser Arg Pro Ser
1               5


<210>  31
<211>  10
<212>  PRT
<213>  Artificial Sequence

<220>
<223>  P2B4 LC-CDR3

<400>  31

Ser Ser Tyr Thr Val Phe Ser Thr Leu Val
1               5                   10


<210>  32
<211>  7
<212>  PRT
<213>  Artificial Sequence

<220>

<223>  P1C1; P1C1_gl; 1G7; P1A8 HC-CDR1

<400>  32

Ser Gly Gly Tyr Tyr Trp Ser
1               5


<210>  33
<211>  16
<212>  PRT
<213>  Artificial Sequence

<220>
<223>  P1C1; P1C1_gl; 1G7; P1A8 HC-CDR2

<400>  33

Tyr Ile Tyr Tyr Ser Gly Ser Thr Tyr Tyr Asn Pro Ser Leu Lys Ser
1               5                   10                  15


<210>  34
<211>  8
<212>  PRT
<213>  Artificial Sequence

<220>
<223>  P1C1; P1C1_gl; 1G7; P1A8 HC-CDR3

<400>  34

Glu Asn Phe Gly Ala Phe Asp His
1               5


<210>  35
<211>  7
<212>  PRT
<213>  Artificial Sequence

<220>
<223>  2E3 HC-CDR1

<400>  35

Ser Gly Gly Tyr Tyr Trp Ala
1               5


<210>  36
<211>  8
<212>  PRT
<213>  Artificial Sequence

<220>
<223>  1E11 HC-CDR3

<400>  36

Glu Asn Phe Gly Ser Tyr Asp Tyr
1               5

<210>  37
<211>  5
<212>  PRT
<213>  Artificial Sequence

<220>
<223>  P1H4 HC-CDR1

<400>  37

Gly Tyr Tyr Met His
1               5


<210>  38
<211>  17
<212>  PRT
<213>  Artificial Sequence

<220>
<223>  P1H4 HC-CDR2

<400>  38

Trp Ile Asn Pro Asn Ser Ala Gly Thr Asn Tyr Ala Gln Lys Phe Gln
1               5                   10                  15


Gly




<210>  39
<211>  11
<212>  PRT
<213>  Artificial Sequence

<220>
<223>  P1H4 HC-CDR3

<400>  39

Glu Gly Ala Asp Gly Ile Tyr Tyr Phe Asp Tyr
1               5                   10


<210>  40
<211>  7
<212>  PRT
<213>  Artificial Sequence

<220>
<223>  P1B11 HC-CDR1

<400>  40

Ser Gly Gly Tyr Tyr Trp Ser
1               5


<210>  41
<211>  16

<212>  PRT
<213>  Artificial Sequence

<220>
<223>  P1B11 HC-CDR2

<400>  41

Tyr Ile Tyr Tyr Ser Gly Thr Thr Tyr Tyr Asn Pro Ser Leu Lys Ser
1               5                   10                  15


<210>  42
<211>  8
<212>  PRT
<213>  Artificial Sequence

<220>
<223>  P1B11 HC-CDR3

<400>  42

Glu Asn Phe Gly Ala Phe Asp Tyr
1               5


<210>  43
<211>  5
<212>  PRT
<213>  Artificial Sequence

<220>
<223>  P2B4 HC-CDR1

<400>  43

Asp Tyr Tyr Ile His
1               5


<210>  44
<211>  17
<212>  PRT
<213>  Artificial Sequence

<220>
<223>  P2B4 HC-CDR2

<400>  44

Trp Met Ser Pro Asp Ser Gly Ala Thr Tyr Tyr Ala Gln Lys Phe Gln
1               5                   10                  15


Gly



<210>  45
<211>  7
<212>  PRT
<213>  Artificial Sequence

<220>
<223>  P2B4 HC-CDR3

<400>  45

Asp Thr Tyr Gly His Asp Tyr
1               5


<210>  46
<211>  14
<212>  PRT
<213>  Artificial Sequence

<220>
<223>  Sequence family LC-CDR1-1 Family consensus


<220>
<221>  MISC_FEATURE
<222>  (1)..(1)
<223>  Xaa = T or A

<220>
<221>  MISC_FEATURE
<222>  (4)..(4)
<223>  Xaa = S or Y

<220>
<221>  MISC_FEATURE
<222>  (9)..(9)
<223>  Xaa = A or D

<220>
<221>  MISC_FEATURE
<222>  (10)..(10)
<223>  Xaa = G or D

<220>
<221>  MISC_FEATURE
<222>  (12)..(12)
<223>  Xaa = D or E

<220>
<221>  MISC_FEATURE
<222>  (13)..(13)
<223>  Xaa = V or T

<220>
<221>  MISC_FEATURE
<222>  (14)..(14)
<223>  Xaa = H or N

<400>  46

Xaa Gly Ser Xaa Ser Asn Ile Gly Xaa Xaa Tyr Xaa Xaa Xaa
1               5                   10


<210>  47
<211>  7
<212>  PRT
<213>  Artificial Sequence

<220>
<223>  Sequence Family LC-CDR2-1 family consensus


<220>
<221>  MISC_FEATURE
<222>  (3)..(3)
<223>  Xaa = N or T

<400>  47

Gly Asn Xaa Asn Arg Pro Ser
1               5


<210>  48
<211>  12
<212>  PRT
<213>  Artificial Sequence

<220>
<223>  Sequence family LC-CDR3-1 family consenus


<220>
<221>  MISC_FEATURE
<222>  (6)..(6)
<223>  Xaa = N or S

<220>
<221>  MISC_FEATURE
<222>  (9)..(9)
<223>  Xaa = Absent or D

<220>
<221>  MISC_FEATURE
<222>  (10)..(10)
<223>  Xaa = A or T

<400>  48

Gln Ser Tyr Asp Ser Xaa Leu Ser Xaa Xaa Trp Val
1               5                   10


<210>  49
<211>  7
<212>  PRT
<213>  Artificial Sequence

<220>
<223>  Sequence family HC-CDR1-1 family consensus


<220>
<221>  MISC_FEATURE
<222>  (7)..(7)
<223>  Xaa = S or A

<400>  49

Ser Gly Gly Tyr Tyr Trp Xaa

1                 5


<210>  50
<211>  5
<212>  PRT
<213>  Artificial Sequence

<220>
<223>  Sequence family HC-CDR1-2 family consensus


<220>
<221>  MISC_FEATURE
<222>  (1)..(1)
<223>  Xaa = G or D

<220>
<221>  MISC_FEATURE
<222>  (4)..(4)
<223>  Xaa = M or I

<400>  50

Xaa Tyr Tyr Xaa His
1               5


<210>  51
<211>  16
<212>  PRT
<213>  Artificial Sequence

<220>
<223>  Sequence family HC-CDR2-1 family consensus


<220>
<221>  MISC_FEATURE
<222>  (7)..(7)
<223>  Xaa = S or T

<400>  51

Tyr Ile Tyr Tyr Ser Gly Xaa Thr Tyr Tyr Asn Pro Ser Leu Lys Ser
1               5                   10                  15


<210>  52
<211>  17
<212>  PRT
<213>  Artificial Sequence

<220>
<223>  Sequence family HC-CDR2-2 family consensus


<220>
<221>  MISC_FEATURE
<222>  (2)..(2)
<223>  Xaa = I or M

<220>

<221>  MISC_FEATURE
<222>  (3)..(3)
<223>  Xaa = N or S

<220>
<221>  MISC_FEATURE
<222>  (5)..(5)
<223>  Xaa = N or D

<220>
<221>  MISC_FEATURE
<222>  (7)..(7)
<223>  Xaa = A or G

<220>
<221>  MISC_FEATURE
<222>  (8)..(8)
<223>  Xaa = G or A

<220>
<221>  MISC_FEATURE
<222>  (10)..(10)
<223>  Xaa = N or Y

<400>  52

Trp Xaa Xaa Pro Xaa Ser Xaa Xaa Thr Xaa Tyr Ala Gln Lys Phe Gln
1               5                   10                  15

Gly

<210>  53
<211>  8
<212>  PRT
<213>  Artificial Sequence

<220>
<223>  Sequence family HC-CDR3-1 family consensus

<220>
<221>  MISC_FEATURE
<222>  (5)..(5)
<223>  Xaa = A or S

<220>
<221>  MISC_FEATURE
<222>  (6)..(6)
<223>  Xaa = F or Y

<220>
<221>  MISC_FEATURE
<222>  (8)..(8)
<223>  Xaa = H or Y

<400>  53

Glu Asn Phe Gly Xaa Xaa Asp Xaa
1               5

<210>  54
<211>  651
<212>  DNA
<213>  Artificial Sequence

<220>
<223>  P1C1

<400>  54
caggctgtgc tgactcagcc gtcctcagtg tctggggccc cagggcagag ggtcaccatc      60

tcctgcactg ggagcagctc caacatcggg gcagattatg agacacactg gtaccagcac     120

cttccaggaa cggcccccaa actcctcatt tatggtaaca ccaatcggcc ctcaggggtc     180

cctcaccgat tctctggctc caagtctggc acctcagcct ccctggccat cactgggctc     240

caggctgagg atgaggctga ttattattgc cagtcctatg acagcaatct gagtgcttgg     300

gtgttcggcg gagggaccaa gctgaccatc ctaagtcagc ccaaggctgc cccctcggtc     360

actctgttcc caccctcctc tgaggagctt caagccaaca aggccacact ggtgtgtctc     420

ataagtgact tctacccggg agccgtgaca gtggcctgga aggcagatag cagccccgtc     480

aaggcgggag tggagaccac cacaccctcc aaacaaagca acaacaagta cgcggccagc     540

agctacctga gcctgacgcc tgagcagtgg aagtcccaca aaagctacag ctgccaggtc     600

acgcatgaag ggagcaccgt ggagaagaca gtggcccctg cagaatgttc a              651


<210>  55
<211>  651
<212>  DNA
<213>  Artificial Sequence

<220>
<223>  P1C1_gl; P1C1_dm; 2E3; 1E11

<400>  55
cagtctgtgc tgactcagcc gccctcagtg tctggggccc cagggcagag ggtcaccatc      60

tcctgcactg ggagcagctc caacatcggg gcagattatg agacacactg gtaccagcaa     120

cttccaggaa cggcccccaa actcctcatt tatggtaaca ccaatcggcc ctcaggggtc     180

cctgaccgat tctctggctc caagtctggc acctcagcct ccctggccat cactgggctc     240

caggctgagg atgaggctga ttattattgc cagtcctatg acagcaatct gagtgcttgg     300

gtgttcggcg gagggaccaa gctgaccatc ctaagtcagc ccaaggctgc cccctcggtc     360

actctgttcc caccctcctc tgaggagctt caagccaaca aggccacact ggtgtgtctc     420

ataagtgact tctacccggg agccgtgaca gtggcctgga aggcagatag cagccccgtc     480

aaggcgggag tggagaccac cacaccctcc aaacaaagca acaacaagta cgcggccagc     540

agctacctga gcctgacgcc tgagcagtgg aagtcccaca aaagctacag ctgccaggtc     600

acgcatgaag ggagcaccgt ggagaagaca gtggcccctg cagaatgttc a              651

<210> 56
<211> 651
<212> DNA
<213> Artificial Sequence

<220>
<223> 1G7; P1C1_tm

<400> 56
cagtctgtgc tgactcagcc gccctcagtg tctggggccc cagggcagag ggtcaccatc 60

tcctgcgctg ggtcttattc taatattggg gatgattatg aaactcattg gtaccagcaa 120

cttccaggaa cggcccccaa actcctcatt tatggtaaca ccaatcggcc ctcaggggtc 180

cctgaccgat tctctggctc caagtctggc acctcagcct ccctggccat cactgggctc 240

caggctgagg atgaggctga ttattattgc cagtcctatg acagcaatct gagtgcttgg 300

gtgttcggcg gagggaccaa gctgaccatc ctaagtcagc ccaaggctgc cccctcggtc 360

actctgttcc caccctcctc tgaggagctt caagccaaca aggccacact ggtgtgtctc 420

ataagtgact tctacccggg agccgtgaca gtggcctgga aggcagatag cagccccgtc 480

aaggcgggag tggagaccac cacaccctcc aaacaaagca acaacaagta cgcggccagc 540

agctacctga gcctgacgcc tgagcagtgg aagtcccaca aaagctacag ctgccaggtc 600

acgcatgaag ggagcaccgt ggagaagaca gtggcccctg cagaatgttc a 651


<210> 57
<211> 642
<212> DNA
<213> Artificial Sequence

<220>
<223> P1H4

<400> 57
gaaattgtga tgacgcagtc tcctggcacc ctgtctttgt ctccagggga aagagccacc 60

ctctcctgca gggccagtca gagtattggg accgacttag cctggtacca gcagaaacct 120

ggccaggctc ccaggctcct catctatgat gcatccaaca gggccactgg catcccagcc 180

aggttcagtg gcagtgggtc tgggacagac ttcactctca ccatcagcag cctagagcct 240

gaagattttg cagtttatta ctgtcagcag cgtagtaact ggcctcccac tttcggcgga 300

gcgaccaagg tggagatcaa acgaactgtg gctgcaccat ctgtcttcat cttcccgcca 360

tctgatgagc agttgaaatc tggaactgcc tctgttgtgt gcctgctgaa taacttctat 420

cccagagagg ccaaagtaca gtggaaggtg gataacgccc tccaatcggg taactcccag 480

gagagtgtca cagagcagga cagcaaggac agcacctaca gcctcagcag caccctgacg 540

ctgagcaaag cagactacga gaaacacaaa gtctacgcct gcgaagtcac ccatcagggc 600

ctgagctcgc ccgtcacaaa gagcttcaac aggggagagt gt 642

<210> 58
<211> 654
<212> DNA
<213> Artificial Sequence

<220>
<223> P1B11

<400> 58
caggctgtgc tgactcagcc gtcctcagtg tctggggccc cagggcagag ggtcaccatc 60

tcctgcactg ggagcagctc caacatcggg gcaggttatg atgtacactg gtaccaacag 120

cttccaggaa cagcccccaa actcctcatt tatggtaaca acaatcgacc ctcaggggtc 180

cctgaccgat tctctggctc caagtctggc acctcagcct ccctggccat cactgggctc 240

caggctgagg atgaggctga ttattactgc cagtcctatg acagcaacct gagtgacacc 300

tgggtattcg gcggagggac caagctgacc gtcctaggtc agcccaaggc tgccccctcg 360

gtcactctgt tcccgccctc ctctgaggag cttcaagcca acaaggccac actggtgtgt 420

ctcataagtg acttctaccc gggagccgtg acagtggcct ggaaggcaga tagcagcccc 480

gtcaaggcgg gagtggagac caccacaccc tccaaacaaa gcaacaacaa gtacgcggcc 540

agcagctacc tgagcctgac gcctgagcag tggaagtccc acaaaagcta cagctgccag 600

gtcacgcatg aagggagcac cgtggagaag acagtggccc ctacagaatg ttca 654

<210> 59
<211> 651
<212> DNA
<213> Artificial Sequence

<220>
<223> P1A8

<400> 59
cagtctgtgc tgacgcagcc gccctcagtg tctggggccc cagggcagag ggtcaccatc 60

tcctgcactg ggagtagctc caacatcggg gcaggttatg atgtaaactg gtaccagcaa 120

cttccaggaa cagcccccaa actcctcatc tatggaaaca acaatcggcc ctcaggggtc 180

cctgaccgat tctctggctc caactctggc acctcagtct ccctggccat cactgggctc 240

cagcctgagg atgaggctga ttactactgc cagtcctacg acagcagcct gagtgcctgg 300

gtgttcggcg gagggacgaa gctggccgtc ctaggtcagc ccaaggctgc cccctcggtc 360

actctgttcc cgccctcctc tgaggagctc caagccaaca aggccacact agtgtgtctg 420

atcagtgact tctacccggg agctgtgaca gtggcctgga aggcagatgg cagccccgtc 480

aaggcgggag tggagaccac caaaccctcc aaacagagca acaacaagta cgcggccagc 540

agctacctga gcctgacgcc tgagcagtgg aagtcccaca aaagctacag ctgccaggtc 600

acgcatgaag ggagcaccgt ggagaagaca gtggcccctg cagaatgctc t 651

<210> 60
<211> 648
<212> DNA
<213> Artificial Sequence

<220>
<223> P2B4

<400> 60
cagtctgccc tgactcagcc tgcctccgtg tctgggtctc ttggacagtc gatcaccatc 60

tcctgcactg ggaccagcag tgacgttggt ggttataact atgtctcctg gtaccaacag 120

caggcaggca aagcccccaa actcatgatt tatgatgtca gtagtcggcc ctcaggggtt 180

tctgatcgct tctctggctc caagtctggc aacacggcct ccctgaccat ctctgggctc 240

caggctgacg acgaggctga ttattactgc agctcatata cagtcttcag cactctagta 300

ttcggcggag ggaccaagtt gaccgtccta ggtcagccca aggctgcccc ctcggtcact 360

ctgttcccac cctcctctga ggaactccaa gccaacaagg ccacactagt gtgtctgatc 420

agtgacttct acccgggagc tgtgacagtg gcctggaagg cagatggcag ccccgtcaag 480

gcgggagtgg agaccaccaa accctccaaa cagagcaaca acaagtacgc ggccagcagc 540

tacctgagcc tgacgcctga gcagtggaag tcccacagaa gctacagctg ccaggtcacg 600

catgaaggga gcaccgtgga gaagacagtg gcccctgcag aatgctca 648

<210> 61
<211> 354
<212> DNA
<213> Artificial Sequence

<220>
<223> P1C1

<400> 61
cagctgcagc tgcaggagtc gggcccagga ctggtgaagc cttcacagac cctgtccctc 60

acctgcgctg tctctggtgg ctccatcagc agtggtggtt actactggag ctggatccgc 120

cagcgcccag ggaagggcct ggagtggatt gggtacatct attacagtgg gagcacctac 180

tacaacccgt ccctcaagag tcgacttacc atatcagtag acacgtctaa gaaccagttc 240

tccctgaaac tgagctctgt gactgccgcg gacacggccg tgtattactg tgcgagagaa 300

aattttggag cgtttgacca ctggggccag ggcaccctgg tcaccgtctc aagc 354

<210> 62
<211> 354
<212> DNA
<213> Artificial Sequence

<220>
<223>  P1C1_gl; 1G7

<400>  62
caggtgcagc tgcaggagtc gggcccagga ctggtgaagc cttcacagac cctgtccctc      60

acctgcactg tctctggtgg ctccatcagc agtggtggtt actactggag ctggatccgc     120

cagcacccag ggaagggcct ggagtggatt gggtacatct attacagtgg gagcacctac     180

tacaacccgt ccctcaagag tcgagttacc atatcagtag acacgtctaa gaaccagttc     240

tccctgaaac tgagctctgt gactgccgcg gacacggccg tgtattactg tgcgagagaa     300

aattttggag cgtttgacca ctggggccag ggcaccctgg tcaccgtctc aagc           354


<210>  63
<211>  354
<212>  DNA
<213>  Artificial Sequence

<220>
<223>  2E3

<400>  63
caggtgcagc tgcaggagtc gggcccagga ctggtgaagc cttcacagac cctgtccctc      60

acctgcactg tctctggtgg ctccatcagc tctggtggtt attattgggc ttggatccgc     120

cagcacccag ggaagggcct ggagtggatt gggtacatct attacagtgg gagcacctac     180

tacaacccgt ccctcaagag tcgagttacc atatcagtag acacgtctaa gaaccagttc     240

tccctgaaac tgagctctgt gactgccgcg gacacggccg tgtattactg tgcgagagaa     300

aattttggag cgtttgacca ctggggccag ggcaccctgg tcaccgtctc aagc           354


<210>  64
<211>  354
<212>  DNA
<213>  Artificial Sequence

<220>
<223>  1E11

<400>  64
caggtgcagc tgcaggagtc gggcccagga ctggtgaagc cttcacagac cctgtccctc      60

acctgcactg tctctggtgg ctccatcagc agtggtggtt actactggag ctggatccgc     120

cagcacccag ggaagggcct ggagtggatt gggtacatct attacagtgg gagcacctac     180

tacaacccgt ccctcaagag tcgagttacc atatcagtag acacgtctaa gaaccagttc     240

tccctgaaac tgagctctgt gactgccgcg gacacggccg tgtattactg tgcgagagaa     300

aattttggat cttatgatta ttggggccag ggcaccctgg tcaccgtctc aagc           354


<210>  65
<211>  354

<212>  DNA
<213>  Artificial Sequence

<220>
<223>  P1C1_dm; P1C1_tm

<400>  65
caggtgcagc tgcaggagtc gggcccagga ctggtgaagc cttcacagac cctgtccctc      60

acctgcactg tctctggtgg ctccatcagc agtggtggtt actactgggc ctggatccgc     120

cagcacccag ggaagggcct ggagtggatt gggtacatct attacagtgg gagcacctac     180

tacaacccgt ccctcaagag tcgagttacc atatcagtag acacgtctaa gaaccagttc     240

tccctgaaac tgagctctgt gactgccgcg gacacggccg tgtattactg tgcgagagaa     300

aattttggat cttatgatta ttggggccag ggcaccctgg tcaccgtctc aagc           354


<210>  66
<211>  360
<212>  DNA
<213>  Artificial Sequence

<220>
<223>  P1H4

<400>  66
caggtccagc tggtgcagtc tggggctgag gtgaagaagc ctggggcctc agtgaaggtc      60

tcctgcaagg cttctggata caccttcacc ggctactata tgcactgggt gcgacaggcc     120

cctggacaag ggcttgagtg gatgggatgg atcaatccta acagtgctgg cacaaactat     180

gcacagaagt tccagggcag agtcaccatg accacagaca catccacgag cacagcctac     240

atggagctga ggagcctgag atctgaggac acggctgtgt attactgtgc gagagagggc     300

gcggacggga tttactactt tgactactgg ggccagggca ccctggtcac cgtctcaagc     360


<210>  67
<211>  354
<212>  DNA
<213>  Artificial Sequence

<220>
<223>  P1B11

<400>  67
caggtgcagc tgcaggagtc gggcccagga ctggtgaagc cttcggagac cctgtccctc      60

acgtgcagtg tctctggtga ctccattagt agtggtggtt actactggag ctggatccgc     120

cagcacccag agaagggcct ggagtggatt gggtacatct attacagtgg gaccacctac     180

tacaacccgt ccctcaagag tcgaataacc atgtcaatag acacgtctaa gaaccagttc     240

tccctgaaag tgagctctgt gactgccgcg gacacggccg tttattactg tgcgagagaa     300

aattttggag cgtttgacta ctggggccag ggcaccctgg tcaccgtctc aagc           354

<210>  68
<211>  354
<212>  DNA
<213>  Artificial Sequence

<220>
<223>  P1A8

<400>  68
cagctgcagc tgcaggagtc cggcccagga ctggtgaagc cttcacagac cctgtccctc      60

acctgcagtg tctctggtgg ctccatcagc agtggtggtt actactggag ctggatccgc     120

cagcgcccag ggaagggcct ggagtggatt gggtacatct attacagtgg gagcacctac     180

tacaacccgt ccctcaagag tcgacttacc atatcagtag acacgtctaa gaaccagctc     240

tccctgaaac tgagctctgt gactgccgcg gacacggccg tgtattactg tgcgagagaa     300

aattttgggg cgtttgacca ctggggccag ggcaccctgg tcaccgtctc aagc           354


<210>  69
<211>  348
<212>  DNA
<213>  Artificial Sequence

<220>
<223>  P2B4

<400>  69
caggtccagc tggtgcagtc tggggctgag gtgaagaagc ctgggacctc agtgacggtc      60

tcctgcaggg cttctggatt caccttcagc gactactata tacactgggt gcgacaggcc     120

cctggacaag gacttgagtg gatgggatgg atgagccctg acagtggtgc cacatactat     180

gcacagaagt ttcagggcag ggtcaccatg accagggaca cgtccaccaa cacagcctac     240

atggaactga gcagactcac atctgacgac acggccgtgt attattgtgt gagagatact     300

tatggccatg actactgggg ccagggaacc ctggtcaccg tctcaagc                  348


<210>  70
<211>  393
<212>  PRT
<213>  Homo sapiens

<400>  70

<210>  71
<211>  9
<212>  PRT
<213>  Artificial Sequence

<220>
<223>  p53 peptide

<400>  71

Arg Met Pro Glu Ala Ala Pro Pro Val
1               5

<210>  72
<211>  9
<212>  PRT
<213>  Artificial Sequence

<220>
<223>  p53 peptide

<400>  72

Ser Thr Pro Pro Pro Gly Thr Arg Val
1               5

<210>  73
<211>  9
<212>  PRT
<213>  Artificial Sequence

<220>
<223>  p53 peptide

<400>  73

Leu Leu Gly Arg Asn Ser Phe Glu Val
1               5

<210>  74
<211>  9
<212>  PRT
<213>  Artificial Sequence

<220>
<223>  p53 peptide

<400>  74

Val Val Pro Tyr Glu Pro Pro Glu Val
1               5

<210>  75
<211>  10
<212>  PRT
<213>  Artificial Sequence

<220>
<223>  p53 peptide

<400>  75

Thr Tyr Ser Pro Ala Leu Asn Lys Met Phe
1               5                   10

<210>  76
<211>  9
<212>  PRT
<213>  Artificial Sequence

<220>
<223>  p53 peptide

<400>  76

Ala Ile Tyr Lys Gln Ser Gln His Met
1               5

<210>  77
<211>  9
<212>  PRT
<213>  Artificial Sequence

<220>
<223>  p53 peptide

<400>  77

Glu Tyr Leu Asp Asp Arg Asn Thr Phe
1               5

## Description

This application claims priority from SG Application No. 10201701883R filed 8 March 2017, the contents and elements of which are herein incorporated by reference for all purposes.

### Field of the Invention

The present invention relates to antibodies which bind to peptide-MHC complexes, in particular peptide-MHC complex comprising a peptide of p53 and MHC class I molecule.

### Background to the Invention

The tumour suppressor p53 is one of the most commonly mutated genes found in malignancies. The p53 transcription factor plays an important role in tumour suppression by regulating genes involved in apoptosis, senescence, cell-cycle arrest and genomic stability. Mutations of p53 that result in a loss of function therefore increase susceptibility to oncogenesis.

A crucial component of the immune system in anti-tumour immunity is the cytotoxicity of CD8+ T cell response to tumour cells. Cytotoxic CD8+ T cells distinguishes tumour cells from normal healthy cells by recognising peptides derived from intracellular processing, which are presented on the cell surface by MHC class I molecules, e.g. HLA*A24.

However, for an effective immune response to be mounted by the CD8+ T cells, the peptide-MHC complex has to be foreign or "non-self" for tolerance to be broken. Mutations of p53 may potentially result in the generation of such distinct peptides that are distinct from normal healthy cells and thus can be recognized by T cells to trigger robust anti-tumour immune responses. In addition, the accumulation of mutant p53 in tumour cells can result in changes to the processes in the degradation of p53, which in turn lead to the generation of a different repertoire of antigenic peptides that distinguishes the tumour cell from normal cells.

Although therapeutic monoclonal antibodies have proven successful in the field of cancer treatment, they are limited by the availability of target molecules on surface of cancer cells. Intracellular proteins such as p53 are inaccessible to classical antibody approaches. Presentation of antigenic epitopes derived from intracellular proteins as peptide-MHC complexes expressed on the surface of cells provides an opportunity to target such antigens with monoclonal antibodies.

Antibodies which target intracellular targets abnormally expressed by MHC class I molecules during cancer have been developed by different groups. However, these antibodies focus mostly on HLA*A02 which is the predominant allele in Caucasians, for instance among the 14 most common HLA*A types in Europe HLA*A02 represent ∼30%. HLA-A distribution in Asia is more scattered, and HLA*A24 and HLA*A11 are common alleles too.

### Summary of the Invention

In one aspect, the present invention provides an antibody or antigen binding fragment, optionally isolated, which is capable of binding to a complex of a peptide of an intracellular protein, and an MHC class I molecule. In some embodiments, the intracellular protein is p53.

In another aspect, the present invention provides an antibody or antigen binding fragment, optionally isolated, which is capable of binding to a peptide-MHC complex comprising a peptide of p53 and an MHC class I molecule.

In some embodiments in accordance with various aspects of the invention, the MHC class I molecule comprises an MHC class I α-chain encoded by an HLA-A*24 allele. In some embodiments, the peptide of p53 comprises, or consists of, the amino acid sequence of SEQ ID NO:75, or a variant having thereof having one or two or three amino acid substitutions in the amino acid sequence. In some embodiments, the antibody or antigen binding fragment comprises the amino acid sequences i) to vi):

| | |
|---|---|
| i) LC-CDR1: | X₁GSX₂SNIGX₃X₄YX₅X₆X₇ (SEQ ID NO:46); |
| | TGTSSDVGGYNYVS (SEQ ID NO:29); or |
| | RASQSIGTDLA (SEQ ID NO:21); |
| ii) LC-CDR2: | GNX₈NRPS (SEQ ID NO:47); |
| | DASNRAT (SEQ ID NO:22); or |
| | DVSSRPS (SEQ ID NO:30) |
| iii) LC-CDR3: | QSYDSX₉LSX₁₀X₁₁WV (SEQ ID NO:48); |
| | QQRSNWPPT (SEQ ID NO:23); or |
| | SSYTVFSTLV (SEQ ID NO:31); |
| iv) HC-CDR1: | SGGYYWX₁₂ (SEQ ID NO:49); or |
| | X₁₃YYX₁₄H (SEQ ID NO:50); |
| v) HC-CDR2: | YIYYSGX₁₅TYYNPSLKS (SEQ ID NO:51); or |
| | WX₁₆X₁₇PX₁₈SX₁₉X₂₀TX_{2'}YAQKFQG (SEQ ID NO:52); |
| vi) HC-CDR3: | ENFGX₂₂X₂₃DX₂₄ (SEQ ID NO:53); |
| | EGADGIYYFDY (SEQ ID NO:39); or |
| | DTYGHDY (SEQ ID NO:45); |

or a variant thereof in which one or two or three amino acids in one or more of the sequences i) to vi) are replaced with another amino acid;
wherein X₁ = T or A, X₂ = S or Y, X₃ = A or D, X₄ = G or D, X₅ = D or E, X₆ = V or T, X₇ = H or N, X₈ = N or T, X₉ = N or S, X₁₀ = Absent or D, X₁₁ = A or T, X₁₂ = S or A, X₁₃ = G or D, X₁₄ = M or I, X₁₅ = S or T, X₁₆ = I or M, X₁₇ = N or S, X₁₈ = N or D, X₁₉ = A or G, X₂₀ = G or A, X₂₁ = N or Y, X₂₂ = A or S, X₂₃ = F or Y, and X₂₄ = H or Y.

In some embodiments, LC-CDR1 is one of TGSSSNIGADYETH (SEQ ID NO:17), AGSYSNIGDDYETH (SEQ ID NO:20), TGSSSNIGAGYDVH (SEQ ID NO:24), TGSSSNIGAGYDVN (SEQ ID NO:27), TGTSSDVGGYNYVS (SEQ ID NO:29) or RASQSIGTDLA (SEQ ID NO:21). In some embodiments, LC-CDR2 is one of GNTNRPS (SEQ ID NO:18), GNNNRPS (SEQ ID NO:25), DASNRAT (SEQ ID NO:22) or DVSSRPS (SEQ ID NO:30). In some embodiments, LC-CDR3 is one of QSYDSNLSAWV (SEQ ID NO:19), QSYDSNLSDTWV (SEQ ID NO:26), QSYDSSLSAWV (SEQ ID NO:28), QQRSNWPPT (SEQ ID NO:23) or SSYTVFSTLV (SEQ ID NO:31). In some embodiments, HC-CDR1 is one of SGGYYWS (SEQ ID NO:32), SGGYYWA (SEQ ID NO:35), SGGYYWS (SEQ ID NO:40), GYYMH (SEQ ID NO:37), or DYYIH (SEQ ID NO:43). In some embodiments, HC-CDR2 is one of YIYYSGSTYYNPSLKS (SEQ ID NO:33), YIYYSGTTYYNPSLKS (SEQ ID NO:41), WINPNSAGTNYAQKFQG (SEQ ID NO:38) or WMSPDSGATYYAQKFQG (SEQ ID NO:44). In some embodiments, HC-CDR3 is one of ENFGAFDH (SEQ ID NO:34), ENFGSYDY (SEQ ID NO:36), EGADGIYYFDY (SEQ ID NO:39), or DTYGHDY (SEQ ID NO:45).

In some embodiments, the antibody or antigen binding fragment has at least one light chain variable region incorporating the following CDRs:

| | |
|---|---|
| LC-CDR1: | TGSSSNIGADYETH (SEQ ID NO:17) |
| LC-CDR2: | GNTNRPS (SEQ ID NO:18) |
| LC-CDR3: | QSYDSNLSAWV (SEQ ID NO:19); |

or

| | |
|---|---|
| LC-CDR1: | AGSYSNIGDDYETH (SEQ ID NO:20) |
| LC-CDR2: | GNTNRPS (SEQ ID NO:18) |
| LC-CDR3: | QSYDSNLSAWV (SEQ ID NO:19); |

or

| | |
|---|---|
| LC-CDR1: | RASQSIGTDLA (SEQ ID NO:21) |
| LC-CDR2: | DASNRAT (SEQ ID NO:22) |
| LC-CDR3: | QQRSNWPPT (SEQ ID NO:23); |

or

| | |
|---|---|
| LC-CDR1: | TGSSSNIGAGYDVH (SEQ ID NO:24) |
| LC-CDR2: | GNNNRPS (SEQ ID NO:25) |
| LC-CDR3: | QSYDSNLSDTWV (SEQ ID NO:26); |

or

| | |
|---|---|
| LC-CDR1: | TGSSSNIGAGYDVN (SEQ ID NO:27) |
| LC-CDR2: | GNNNRPS (SEQ ID NO:25) |
| LC-CDR3: | QSYDSSLSAWV (SEQ ID NO:28); |

or

| | |
|---|---|
| LC-CDR1: | TGTSSDVGGYNYVS (SEQ ID NO:29) |
| LC-CDR2: | DVSSRPS (SEQ ID NO:30) |
| LC-CDR3: | SSYTVFSTLV (SEQ ID NO:31). |

In some embodiments, the antibody or antigen binding fragment has at least one heavy chain variable region incorporating the following CDRs:

| | |
|---|---|
| HC-CDR1: | SGGYYWS (SEQ ID NO:32) |
| HC-CDR2: | YIYYSGSTYYNPSLKS (SEQ ID NO:33) |
| HC-CDR3: | ENFGAFDH (SEQ ID NO:34 |

or

| | |
|---|---|
| HC-CDR1: | SGGYYWA (SEQ ID NO:35) |
| HC-CDR2: | YIYYSGSTYYNPSLKS (SEQ ID NO:33) |
| HC-CDR3: | ENFGAFDH (SEQ ID NO:34); |

or

| | |
|---|---|
| HC-CDR1: | SGGYYWS (SEQ ID NO:32) |
| HC-CDR2: | YIYYSGSTYYNPSLKS (SEQ ID NO:33) |
| HC-CDR3: | ENFGSYDY (SEQ ID NO:36); |

or

| | |
|---|---|
| HC-CDR1: | SGGYYWA (SEQ ID NO:35) |
| HC-CDR2: | YIYYSGSTYYNPSLKS (SEQ ID NO:33) |
| HC-CDR3: | ENFGSYDY (SEQ ID NO:36); |

or

| | |
|---|---|
| HC-CDR1: | GYYMH (SEQ ID NO:37) |
| HC-CDR2: | WINPNSAGTNYAQKFQG (SEQ ID NO:38) |
| HC-CDR3: | EGAOGIYYFOY (SEQ ID NO:39): |

or

| | |
|---|---|
| HC-CDR1: | SGGYYWS (SEQ ID NO:40) |
| HC-CDR2: | YIYYSGTTYYNPSLKS (SEQ ID NO:41) |
| HC-CDR3: | ENFGAFDY (SEQ ID NO:42); |

or

| | |
|---|---|
| HC-CDR1: | DYYIH (SEQ ID NO:43) |
| HC-CDR2: | WMSPDSGATYYAQKFQG (SEQ ID NO:44) |
| HC-CDR3: | DTYGHDY (SEQ ID NO:45). |

In another aspect, the present invention provides an antibody or antigen binding fragment, optionally isolated, which is capable of binding to a peptide-MHC complex comprising a peptide of p53 and an MHC class I molecule, comprising a light chain and a heavy chain variable region sequence, wherein:
the light chain comprises a LC-CDR1, LC-CDR2, LC-CDR3, having at least 85% overall sequence identity to LC-CDR1: one of X₁GSX₂SNIGX₃X₄YX₅X₆X₇ (SEQ ID NO:46), TGTSSDVGGYNYVS (SEQ ID NO:29) or RASQSIGTDLA (SEQ ID NO:21); LC-CDR2: one of GNX₈NRPS (SEQ ID NO:47), DASNRAT (SEQ ID NO:22) or DVSSRPS (SEQ ID NO:30); LC-CDR3: one of QSYDSX₉LSX₁₀X₁₁WV (SEQ ID NO:48), QQRSNWPPT (SEQ ID NO:23) or SSYTVFSTLV (SEQ ID NO:31); and
the heavy chain comprises a HC-CDR1, HC-CDR2, HC-CDR3, having at least 85% overall sequence identity to HC-CDR1: one of SGGYYWX₁₂ (SEQ ID NO:49) or X₁₃YYX₁₄H (SEQ ID NO:50); HC-CDR2: one of YIYYSGX₁₅TYYNPSLKS (SEQ ID NO:51) or WX₁₆X₁₇PX₁₈SX₁₉X₂₀TX_{2'}YAQKFQG (SEQ ID NO:52); HC-CDR2: one of ENFGX₂₂X₂₃DX₂₄ (SEQ ID NO:53), EGADGIYYFDY (SEQ ID NO:39) or DTYGHDY (SEQ ID NO:45);
wherein X₁ = T or A, X₂ = S or Y, X₃ = A or D, X₄ = G or D, X₅ = D or E, X₆ = V or T, X₇ = H or N, X₈ = N or T, X₉ = N or S, X₁₀ = Absent or D, X₁₁ = A or T, X₁₂ = S or A, X₁₃ = G or D, X₁₄ = M or I, X₁₅ = S or T, X₁₆ = I or M, X₁₇ = N or S, X₁₈ = N or D, X₁₉ = A or G, X₂₀ = G or A, X₂₁ = N or Y, X₂₂ = A or S, X₂₃ = F or Y, and X₂₄ = H or Y.

In another aspect, the present invention provides an antibody or antigen binding fragment, optionally isolated, which is capable of binding to a peptide-MHC complex comprising a peptide of p53 and an MHC class I molecule, comprising a light chain and a heavy chain variable region sequence, wherein:
the light chain sequence has at least 85% sequence identity to the light chain sequence of one of SEQ ID NOs:1 to 7, and;
the heavy chain sequence has at least 85% sequence identity to the heavy chain sequence of one of SEQ ID NOs:8 to 16.

In some embodiments in accordance with various aspects of the invention, the antibody or antigen binding fragment displays antibody-dependent cell-mediated cytotoxicity (ADCC) to cells comprising or expressing peptide-MHC complex comprising a peptide of p53 and an MHC class I molecule. In some embodiments, the antibody or antigen binding fragment is internalised by cells comprising or expressing peptide-MHC complex comprising a peptide of p53 and an MHC class I molecule. In some embodiments, the antibody or antigen binding fragment is a fully human antibody or a fully human antibody fragment. In some embodiments, the antibody or antigen binding fragment is conjugated to a drug moiety or a detectable moiety.

In some embodiments in accordance with various aspects of the invention, the antibody or antigen binding fragment further comprises an antibody or antigen binding fragment specific for a target other than a peptide-MHC complex. In some embodiments, the antibody or antigen binding fragment specific for a target other than a peptide-MHC complex is an antibody or antigen binding fragment capable of binding to an immune cell surface molecule, i.e. the target other than a peptide-MHC complex is an immune cell surface molecule.

In another aspect, the present invention provides a chimeric antigen receptor (CAR) comprising an antigen binding fragment according to the present invention.

In another aspect, the present invention provides an *in vitro* complex, optionally isolated, comprising an antibody, antigen binding fragment or CAR according to the present invention bound to a peptide-MHC complex comprising a peptide of p53 and an MHC class I molecule.

In another aspect, the present invention provides a composition comprising the antibody, antigen binding fragment or CAR according to the present invention and at least one pharmaceutically-acceptable carrier.

In another aspect, the present invention provides an isolated nucleic acid encoding the antibody, antigen binding fragment or CAR according to the present invention.

In another aspect, the present invention provides a vector comprising the nucleic acid according to the present invention.

In another aspect, the present invention provides a cell comprising the nucleic acid according to the present invention or the vector according to the present invention.

In another aspect, the present invention provides a method for making an antibody, antigen binding fragment or CAR according to the present invention, comprising culturing the cell of the present invention under conditions suitable for the expression of the antibody or antigen binding fragment or CAR.

In another aspect, the present invention provides an antibody, antigen binding fragment, CAR, composition, nucleic acid, vector or cell according to the present invention for use in therapy, or in a method of medical treatment.

In another aspect, the present invention provides an antibody, antigen binding fragment, CAR, composition, nucleic acid, vector or cell according to the present invention for use in the treatment or prevention of a cancer.

In another aspect, the present invention provides the use of an antibody, antigen binding fragment, CAR, composition, nucleic acid, vector or cell according to the present invention in the manufacture of a medicament for treating or preventing a cancer.

In another aspect, the present invention provides a method of treating or preventing a cancer, comprising administering to a subject a therapeutically or prophylactically effective amount of the antibody, antigen binding fragment, CAR, composition, nucleic acid, vector or cell according to the present invention.

In another aspect, the present invention provides a method of treating or preventing a cancer in a subject, comprising:
(a) isolating at least one cell from a subject;
(b) modifying the at least one cell to express or comprise the antibody, antigen binding fragment, CAR, nucleic acid or vector according to the present invention and;
(c) administering the modified at least one cell to a subject.

In another aspect, the present invention provides a method of treating or preventing a cancer in a subject, comprising:
(a) isolating at least one cell from a subject;
(b) introducing into the at least one cell the nucleic acid according to the present invention or the vector according to the present invention, thereby modifying the at least one cell and;
(c) administering the modified at least one cell to a subject.

In another aspect, the present invention provides a kit of parts comprising a predetermined quantity of the antibody, antigen binding fragment, CAR, composition, nucleic acid, vector or cell according to the present invention.

In another aspect, the present invention provides a method of diagnosing a disease or a condition in a subject, the method comprising contacting a sample containing, or suspected to contain, peptide-MHC complex with an antibody or antigen binding fragment according to any one of claims 1 to 21 and detecting the formation of a complex of antibody, or antigen binding fragment, and the peptide-MHC complex.

### Description

The present invention relates to antibodies and antigen binding fragments which bind to peptide-MHC complexes, in particular antibodies capable of binding to peptide-MHC complex comprising a p53 peptide bound to (i.e. presented by) an MHC class I molecule.

Antibodies/fragments that mimic the TCR's specificity for a tumour associated peptide-MHC complex, e.g. p53/HLA*A24, are useful as tumour-specific targeting agents. Such species are effective as a therapeutics on their own, or as a targeting tool for specific delivery of payloads such as drugs and toxins, and also as a diagnostic tools.

### Peptide-MHC complexes

T cell receptors (TCRs) recognise antigen peptides presented by MHC molecules.
Antigens are processed by the molecular machinery of antigen presenting cells (APCs) to peptides, which then become associated with MHC molecules and presented as peptide-MHC complexes at the cell surface. Different TCRs display different ability to bind to, and therefore different reactivity to, different peptide-MHC complexes. Antigen processing, loading and presentation on MHC is described in detail in, for example, Immunobiology, 5th Edn. Janeway CA Jr, Travers P, Walport M, et al. New York: Garland Science (2001), Chapter 5, hereby incorporated by reference in entirety.

The present invention is particularly concerned with TCR-like antibodies, which are capable of specific binding to peptide-MHC complexes. In particular, the present invention is concerned with antibodies capable of binding to peptide-MHC complexes comprising a peptide of an intracellular protein presented by an MHC class I molecule.

An 'intracellular protein' may be a protein which is not expressed to a significant degree at the surface of a cell expressing the protein. The subcellular localization of an intracellular protein may be primarily to the cytoplasm or nucleus of a cell expressing the protein, or to an organelle of a cell expressing the protein e.g. the endoplasmic reticulum, Golgi apparatus, etc.

In some embodiments, the antibodies of the present invention are capable of binding to peptide-MHC complex comprising a peptide of an intracellular protein whose presentation by MHC class I is upregulated in a disease, e.g. a cancer. In some embodiments, the antibodies of the present invention are capable of binding to peptide-MHC complex comprising a cancer-associated peptide.

In some embodiments, the antibodies of the present invention are capable of binding to peptide-MHC complex comprising a peptide of p53.

The p53 tumour suppressor is a protein which is encoded in humans by the TP53 gene. P53 plays a crucial role in cellular response to DNA damage and other genomic aberrations. Activation of p53 leads to cell cycle arrest, DNA repair or apoptosis.
In this specification "p53" refers to p53 from any species and includes isoforms, fragments, variants or homologues of a p53 from any species. In some embodiments, the p53 is human p53, primate p53, non-human primate p53, rodent p53, murine p53, or mammalian p53.

In some embodiments p53 may comprise or consist of the amino acid sequence of UniProtKB - P04637 (P53_HUMAN):







 (SEQ ID NO:70)

In some embodiments, the p53 may comprise or consist of the amino acid sequence having at least 60%, e.g. one of at least 65%, 70%, 75%, 80%, 85%, 90%, 91%, 92%, 93%, 94%, 95%, 96%, 97%, 98% or 99% sequence identity to the amino acid sequence of SEQ ID NO:70.

Mutations of the gene encoding p53 have been implicated in the development and progression of a variety of cancers (Muller and Vousden, Cancer Cell (2014) 25:304-317). As explained e.g. in Sakakura et al. 2007, Clin Immunol. 125(1):43-51, loss of p53 function is the most common abnormality in human cancer, and more than 80% tumors showing defects in p53, and these alterations lead to increased presentation of wildtype p53 epitopes (non-mutated peptide sequences derived from p53 protein) by HLA class I molecules on tumor cells, for recognition by HLA class I-restricted T cells.

CD8+ T cells have been identified which recognize MHC class I presenting different p53 epitopes. These include the HLA-A*02 (HLA-A2)-restricted epitopes p53₆₅₋₇₃, p53₁₄₉₋₁₅₇, p53₂₆₄₋₂₇₂ and p53₂₁₇₋₂₂₅, and the HLA-A*24 (HLA-A24)-restricted epitopes p53₁₂₅₋₁₃₄, p53₁₆₁₋₁₆₉, and p53₂₀₄₋₂₁₂ (see e.g. Chikamatsu et al., Oral Oncol. 2008 44(9): 870-877). The amino acid sequences of these peptides of p53 are shown below.
p53₆₅₋₇₃: RMPEAAPPV (SEQ ID NO:71)
p53₁₄₉₋₁₅₇: STPPPGTRV (SEQ ID NO:72)
p53₂₆₄₋₂₇₂: LLGRNSFEV (SEQ ID NO:73)
p53₂₁₇₋₂₂₅: VVPYEPPEV (SEQ ID NO:74)
p53₁₂₅₋₁₃₄: TYSPALNKMF (SEQ ID NO:75)
p53₁₆₁₋₁₆₉: AIYKQSQHM (SEQ ID NO:76)
p53₂₀₄₋₂₁₂: EYLDDRNTF (SEQ ID NO:77)

As used herein a "peptide" refers to a chain of two or more amino acid monomers linked by peptide bonds. In some embodiments a peptide may be 50 amino acids or fewer in length. A "polypeptide" as used herein refers to a chain of two or more peptides linked by peptide bonds.

In some embodiments, a peptide of p53 may comprise or consist of a contiguous sequence of 5-20, 5-18, 5-15, 5-14, 5-13, 5-12, 5-11, or 5-10 amino acids. In some embodiments, the peptide may be one of 5, 6, 7, 8, 9, 10, 11, 12, 13, 14, or 15 amino acids in length.

In some embodiments, the p53 peptide comprises, or consists of, the sequence of one of SEQ ID NOs:71 to 77, or variant thereof having one or two or three amino acid substitutions in the amino acid sequence. In some embodiments, the peptide additionally comprises 1, 2, 3, 4, 5 amino acids at one or both ends of the amino acid sequence. In some embodiments, the peptide additionally comprises 1-2, 1-3, 1-4, or 1-5 amino acids at one or both ends of the amino acid sequence.

In some embodiments, the p53 peptide does not consist of SEQ ID NO:73.

In some embodiments, the p53 peptide comprises, or consists of, the sequence of SEQ ID NO:75, or variant thereof having one or two or three amino acid substitutions in the amino acid sequence.

The peptide is presented by an MHC molecule, e.g. an MHC class I molecule. MHC class I molecules are heterodimers of an α-chain and a β2-microglobulin. The α-chain has three domains designated α1, α2 and α3. The α1 and α2 domains together form the groove to which the peptide presented by the MHC class I molecule binds, to form the peptide-MHC complex. MHC class I α-chains are polymorphic, and different α-chains are capable of binding and presenting different peptides. In humans MHC class I α-chains are encoded by human leukocyte antigen (HLA) genes.

In this specification MHC class I α/HLA may be from any species and includes isoforms, fragments, variants or homologues from any species. In some embodiments, the MHC class I α/HLA is human, primate, non-human primate, rodent, murine, or mammalian.

In some embodiments, the MHC class I molecule comprises an α-chain encoded at the HLA-A locus. In some embodiments, the α-chain is encoded by an HLA-A allele which is a member of allele group HLA-A2 or HLA-A24. In some embodiments, the α-chain is encoded by an HLA-A allele which is a member of allele group HLA-A24 (e.g. HLA-A2402 or HLA-A2403). In some embodiments, the α-chain is encoded by HLA-A2402.

In some embodiments, the MHC class I molecule comprises an α-chain which is not encoded by HLA-A201. In some embodiments, the MHC class I molecule comprises an α-chain which is not encoded by a member of allele group HLA-A2.

In particular embodiments, the present invention is directed to an antibody or antigen binding fragment which is capable of binding to a peptide:MHC complex comprising a peptide comprising or consisting of the amino acid sequence of SEQ ID NO:75, and an MHC class I molecule comprising an MHC class I α-chain encoded by an HLA-A*24 allele.

### Antibodies/antigen-binding fragments

Antibodies and antigen-binding fragments according to the present invention bind to a peptide-MHC complex, of a peptide of p53 and an MHC class I molecule.

By "antibody" we include fragments and derivatives thereof, or a synthetic antibody or synthetic antibody fragment. As used herein, an antibody is a polypeptide capable of binding specifically to the relevant target molecule (i.e. the antigen for which the antibody is specific). Antibodies according to the present invention may be provided in isolated form.

In view of contemporary techniques in relation to monoclonal antibody technology, antibodies can be prepared to most antigens. The antigen-binding portion may be a part of an antibody (for example a Fab fragment) or a synthetic antibody fragment (for example a single chain Fv fragment [ScFv]). Suitable monoclonal antibodies to selected antigens may be prepared by known techniques, for example those disclosed in "Monoclonal Antibodies: A manual of techniques ", H Zola (CRC Press, 1988) and in "Monoclonal Hybridoma Antibodies: Techniques and Applications ", J G R Hurrell (CRC Press, 1982). Chimeric antibodies are discussed by Neuberger et al (1988, 8th International Biotechnology Symposium Part 2, 792-799).

Monoclonal antibodies (mAbs) are useful in the methods of the invention and are a homogenous population of antibodies specifically targeting a single epitope on an antigen.

Antigen binding fragments of antibodies, such as Fab and Fab₂ fragments may also be used/provided as can genetically engineered antibodies and antibody fragments. The variable heavy (V_{H}) and variable light (V_{L}) domains of the antibody are involved in antigen recognition, a fact first recognised by early protease digestion experiments. Further confirmation was found by "humanisation" of rodent antibodies. Variable domains of rodent origin may be fused to constant domains of human origin such that the resultant antibody retains the antigenic specificity of the rodent parent antibody (Morrison et al (1984) Proc. Natl. Acad. Sd. USA 81, 6851-6855).

In some embodiments, the antibody/fragment is a fully human antibody/fragment. A fully human antibody/fragment is encoded by human nucleic acid sequence(s). Fully human antibodies/fragments are devoid of non-human amino acid sequences.

The two most commonly employed techniques to the production of fully human antibodies are (i) phage display, in which human antibody genes are expressed in phage display libraries, and (ii) production of antibodies in transgenic mice engineered to have human antibody genes (described in Park and Smolen Advances in Protein Chemistry (2001) 56: 369-421). Briefly, in the human antibody gene-phage display technique, genes encoding the VH and VL chains are generated by PCR amplification and cloning from "naive" human lymphocytes, and assembled into a library from which they can be expressed either as disulfide-linked Fab fragments or as single-chain Fv (scFv) fragments. The Fab-or scFv-encoding genes are fused to a surface coat protein of filamentous bacteriophage and Fab or scFv capable of binding to the target of interest can then be identified by screening the library with antigen. Molecular evolution or affinity maturation procedures can be employed to enhance the affinity of the Fab/scFv fragment. In the transgenic mouse technique, mice in which the endogenous murine Ig gene loci have been replaced by homologous recombination with their human homologues are immunized with antigen, and monoclonal antibody is prepared by conventional hybridoma technology, to yield fully human monoclonal antibody.

That antigenic specificity is conferred by variable domains and is independent of the constant domains is known from experiments involving the bacterial expression of antibody fragments, all containing one or more variable domains. These molecules include Fab-like molecules (Better et al (1988) Science 240, 1041); Fv molecules (Skerra et al (1988) Science 240, 1038); single-chain Fv (ScFv) molecules where the V_{H} and V_{L} partner domains are linked via a flexible oligopeptide (Bird et al (1988) Science 242, 423; Huston et al (1988) Proc. Natl. Acad. Sd. USA 85, 5879) and single domain antibodies (dAbs) comprising isolated V domains (Ward et al (1989) Nature 341, 544). A general review of the techniques involved in the synthesis of antibody fragments which retain their specific binding sites is to be found in Winter & Milstein (1991) Nature 349, 293- 299.

By "ScFv molecules" we mean molecules wherein the V_{H} and V_{L} partner domains are covalently linked, e.g. by a flexible oligopeptide.

Fab, Fv, ScFv and dAb antibody fragments can all be expressed in and secreted from E. coli, thus allowing the facile production of large amounts of the said fragments.

Whole antibodies, and F(ab')₂ fragments are "bivalent". By "bivalent" we mean that the said antibodies and F(ab')₂ fragments have two antigen combining sites. In contrast, Fab, Fv, ScFv and dAb fragments are monovalent, having only one antigen combining site.

An antigen-binding fragment according to the present invention may be any fragment of a polypeptide which is capable of binding to target antigen.

In some embodiments, an antigen binding fragment comprises at least three light chain CDRs (i.e. LC-CDR1, LC-CDR2 and LC-CDR3; also referred to herein as LC-CDRs 1-3) and three heavy chain CDRs (i.e. HC-CDR1, HC-CDR2 and HC-CDR3; also referred to herein as HC-CDRs 1-3) which together define the antigen binding region of an antibody or antigen binding fragment. In some embodiments, an antigen binding fragment may comprise the light chain variable domain and heavy chain variable domain of an antibody or antigen binding fragment. In some embodiments, an antigen binding fragment may comprise the light chain polypeptide and heavy chain polypeptide of an antibody or antigen binding fragment.

The present invention also provides a chimeric antigen receptor (CAR) comprising one or more antigen binding fragments or polypeptides according to the present invention.

Chimeric Antigen Receptors (CARs) are recombinant receptors that provide both antigen-binding and T cell activating functions. CAR structure and engineering is reviewed, for example, in Dotti et al., Immunol Rev (2014) 257(1), hereby incorporated by reference in its entirety. A CAR typically comprises an extracellular antigen-binding region linked to a cell membrane anchor region, e.g. a transmembrane region, and a signalling region. An optional hinge or spacer region may provide separation between the antigen-binding region and cell membrane anchor region, and may act as a flexible linker.

Antigen-binding fragments or polypeptides according to the present invention are provided herein as the antigen-binding domain of a chimeric antigen receptor (CAR). In some embodiments, the CAR comprises a VL domain and a VH domain according to any embodiment of an antibody, antigen binding fragment or polypeptide described herein. The antigen binding region of a CAR according to the present invention may be provided with any suitable format, e.g. scFv, scFab, etc.

The cell membrane anchor region, or transmembrane region, is provided between the antigen-binding region and the signalling region of the CAR and provides for anchoring the CAR to the cell membrane of a cell expressing a CAR, with the antigen-binding region in the extracellular space, and signalling region inside the cell. In some embodiments, the CAR comprises a cell membrane anchor region comprising or consisting of an amino acid sequence which comprises, consists of, or is derived from, the transmembrane region amino acid sequence for one of CDS-ζ, CD4, CD8 or CD28. As used herein, a region which is 'derived from' a reference amino acid sequence comprises an amino acid sequence having at least 60%, e.g. one of at least 65%, 70%, 75%, 80%, 85%, 90%, 91%, 92%, 93%, 94%, 95%, 96%, 97%, 98%, 99% or 100% sequence identity to the reference sequence.

The cell membrane anchor region, or transmembrane region, may be hydrophobic alpha helix that spans the cell membrane. The transmembrane region associated with the signalling region is commonly used, or the transmembrane region can be selected or modified by amino acid substitution to avoid binding of such domains to the transmembrane domains of the same or different surface membrane proteins to minimize interactions with other members of the receptor complex.

CARs may be combined with costimulatory ligands, chimeric costimulatory receptors or cytokines to further enhance T cell potency, specificity and safety (Sadelain et al., The basic principles of chimeric antigen receptor (CAR) design. Cancer Discov. 2013 April; 3(4): 388-398. doi:10.1158/2159-8290.CD-12-0548, specifically incorporated herein by reference).

Also provided is a cell comprising a CAR according to the invention. The CAR according to the present invention may be used to generate CAR-expressing immune cells, e.g. T cells. These cells may target cancer cells, e.g. tumour cells, which express the antigen(s) for which the CAR is specific. Engineering of CARs into T cells may be performed during culture, *in vitro,* for transduction and expansion, such as happens during expansion of T cells for adoptive T cell therapy.

Also provided in the present invention are bispecific antibodies and bispecific antigen binding fragments comprising an antibody or antigen binding fragment according to the present invention. The bispecific antibodies or bispecific antigen binding fragments may comprise (i) an antibody or antigen binding fragment according to the present invention, and (ii) an antibody or antigen binding fragment specific for a target other than a peptide-MHC complex.

In some embodiments the bispecific antibodies and bispecific antigen binding fragments comprise an antigen or antigen binding fragment according to the present invention, and an antibody or antigen binding fragment which is capable of binding to an immune cell surface molecule. Herein, an immune cell surface molecule is a molecule which is expressed at the cell surface of an immune cell, and may be any peptide/polypeptide, glycoprotein, lipoprotein, glycan, glycolipid, lipid, or fragment thereof.

The immune cell surface molecule may be expressed at the cell surface of any immune cell. In some embodiments, the immune cell may be a cell of hematopoietic origin, e.g. a neutrophil, eosinophil, basophil, dendritic cell, lymphocyte, or monocyte. The lymphocyte may be e.g. a T cell, B cell, natural killer (NK) cell, NKT cell or innate lymphoid cell (ILC), or a precursor thereof (e.g. a thymocyte or pre-B cell). The cell may express one or more CD3 polypeptides (e.g. CD3ε, CD3y, CD3δ, CD3ζ and/or CD3η), TCR polypeptides (TCRα, TCRβ, TCRγ and/or TCRδ), CD27, CD28, CD4, CD8, CD16, CCR5, CCR7, CD2, CD7, PD-1, and/or CTLA4.

In some embodiments, the immune cell is a T cell. In some embodiments, the T cell is a CD3+ T cell. In some embodiments, the T cell is a CD3+, CD8+ T cell. In some embodiments, the T cell is a CD3+, CD4+ T cell. In some embodiments, the T cell is a cytotoxic T cell (e.g. a cytotoxic T lymphocyte (CTL)), a T helper cell (e.g. a Th1, Th2, Th9, Th17, Th22 or Tfh cell), a regulatory T cell (Treg), a central memory cell (Tcm), or an effector memory cell (Tem).

In some embodiments, the immune cell is an NK cell. In some embodiments, the T cell is a CD16+ NK cell.

In some embodiments, the immune cell surface molecule is selected from one or more CD3 polypeptides (e.g. CD3ε, CD3γ, CD3δ, CD3ζ or CD3η), TCR polypeptides (TCRα, TCRβ, TCRy and TCRδ), CD27, CD28, CD4, CD8, CCR5, CCR7, CD2, CD7, PD-1, and CTLA4.

CD3 is a complex of polypeptides expressed at the cell surface of T lymphocytes. In mammals, the CD3 complex contains a CD3γ chain, a CD3δ chain and two CD3ε chains. These chains associate with the TCR polypeptides (TCRα and TCRβ) and CD3ζ/CD3η chains to form the CD3-TCR complex, which generates the activation signal in T lymphocytes.

In some embodiments, the immune cell surface molecule is a molecule expressed at the cell surface of a T cell. In some embodiments, the immune cell surface molecule is a polypeptide of the CD3-TCR complex. In some embodiments, the immune cell surface molecule is a CD3 polypeptide (e.g. CD3ε, CD3γ, CD3δ, CD3ζ or CD3η), a complex containing a CD3 polypeptide, a TCR polypeptide (TCRα, TCRβ, TCRγ or TCRδ) or a complex containing a TCR polypeptide.

In some embodiments, the bispecific antibodies and bispecific antigen binding fragments comprise an antibody or antigen binding fragment according to the present invention and a CD3 binding domain. In some embodiments, the bispecific antibodies and bispecific antigen binding fragments comprise an antibody or antigen binding fragment according to the present invention and a CD3-TCR complex polypeptide binding domain. In some embodiments the CD3-TCR complex polypeptide binding domain is a CD3 polypeptide binding domain. In some embodiments, the CD3-TCR complex polypeptide binding domain is a CD3ε binding domain.

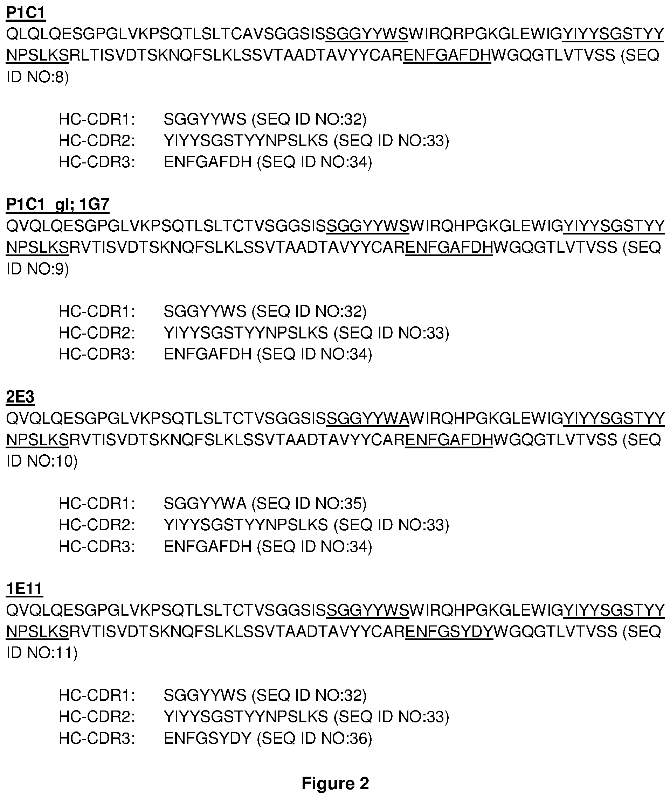

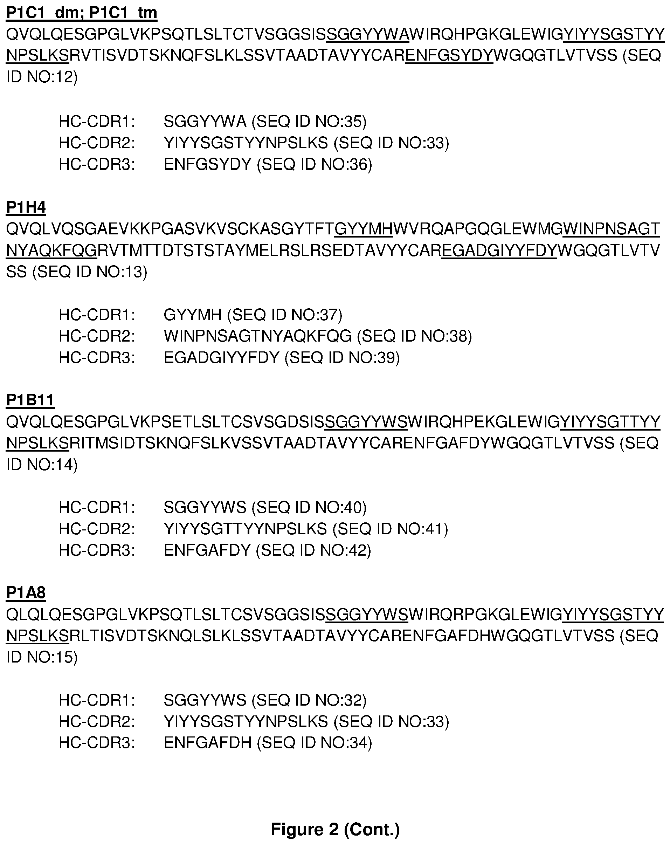

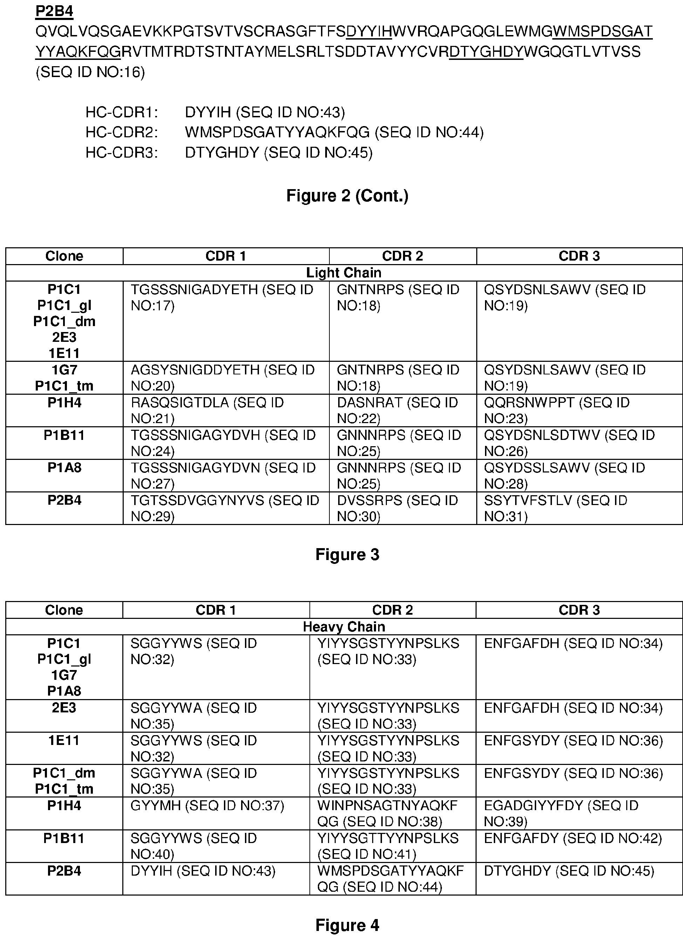

Bispecific antibodies/fragments may be provided in any suitable format, such as those formats described in Kontermann MAbs 2012, 4(2): 182-197, which is hereby incorporated by reference in its entirety. For example, a bispecific antibody or bispecific antigen binding fragment may be a bispecific antibody conjugate (e.g. an IgG2, F(ab')₂ or CovX-Body), a bispecific IgG or IgG-like molecule (e.g. an IgG, scFv₄-Ig, IgG-scFv, scFv-IgG, DVD-Ig, IgG-sVD, sVD-IgG, 2 in 1 -IgG, mAb², or Tandemab common LC), an asymmetric bispecific IgG or IgG-like molecule (e.g. a kih IgG, kih IgG common LC, CrossMab, kih IgG-scFab, mAb-Fv, charge pair or SEED-body), a small bispecific antibody molecule (e.g. a Diabody (Db), dsDb, DART, scDb, tandAbs, tandem scFv (taFv), tandem dAb/VHH, triple body, triple head, Fab-scFv, or F(ab')₂-scFv₂), a bispecific Fc and C_{H}3 fusion protein (e.g. a taFv-Fc, Di-diabody, scDb-C_{H}3, scFv-Fc-scFv, HCAb-VHH, scFv-kih-Fc, or scFv-kih-C_{H}3), or a bispecific fusion protein (e.g. a scFv₂-albumin, scDb-albumin, taFv-toxin, DNL-Fab₃, DNL-Fab₄-IgG, DNL-Fab₄-IgG-cytokine₂). See in particular Figure 2 of Kontermann MAbs 2012, 4(2): 182-19. The bispecific antibody or antigen binding fragment may be a bispecific T cell engager (BiTE).

In some embodiments the antibody or antigen binding fragment specific for a target other than a peptide-MHC complex is located on the fragment crystallisable region (Fc region) of a bispecific antibody or bispecific antigen binding fragment described herein. In some embodiments the Fab region of the bispecific antibody or bispecific antigen binding fragment comprises the antibody or antigen binding fragment specific for a target other than a peptide-MHC complex. In some embodiments the antibody or antigen binding fragment specific for a target other than a peptide-MHC complex replaces a Fab region of a bispecific antibody or bispecific antigen binding fragment described herein.

Methods for producing bispecific antibodies include chemically crosslinking of antibodies or antibody fragments, e.g. with reducible disulphide or non-reducible thioether bonds, for example as described in Segal and Bast, 2001. Production of Bispecific Antibodies. Current Protocols in Immunology. 14:IV:2.13:2.13.1-2.13.16, which is hereby incorporated by reference in its entirety. For example, *N*-succinimidyl-3-(-2-pyridyldithio)-propionate (SPDP) can be used to chemically crosslink e.g. Fab fragments via hinge region SH- groups, to create disulfide-linked bispecific F(ab)₂ heterodimers. Other methods include fusing antibody-producing hybridomas e.g. with polyethylene glycol, to produce a quadroma cell capable of secreting bispecific antibody, for example as described in D. M. and Bast, B. J. 2001. Production of Bispecific Antibodies. Current Protocols in Immunology. 14:IV:2.13:2.13.1-2.13.16. Bispecific antibodies and bispecific antigen binding fragments can also be produced recombinantly, by expression from e.g. a nucleic acid construct encoding polypeptides for the antigen binding molecules, for example as described in Antibody Engineering: Methods and Protocols, Second Edition (Humana Press, 2012), at Chapter 40: Production of Bispecific Antibodies: Diabodies and Tandem scFv (Hornig and Farber-Schwarz), or French, How to make bispecific antibodies, Methods Mol. Med. 2000; 40:333-339, the entire contents of both of which are hereby incorporated by reference.

Antibodies may be produced by a process of affinity maturation in which a modified antibody is generated that has an improvement in the affinity of the antibody for antigen, compared to an unmodified parent antibody. Affinity-matured antibodies may be produced by procedures known in the art, e.g., Marks et al.,Rio/Technology 10:779-783 (1992); Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):331 0-15 9 (1995); and Hawkins et al, J. Mol. Biol. 226:889-896 (1992).

The present invention provides antibodies described herein which have further undergone the process of chain shuffling, e.g. light chain shuffling and/or heavy chain shuffling. Chain shuffling to improve antibody affinity is described in detail in Marks, Antibody Affinity Maturation by Chain Shuffling, Antibody Engineering Methods and Protocols, Humana Press (2004) Vol. 248, pp327-343, which is hereby incorporated by reference in its entirety - in particular, light chain shuffling is described in detail at sections 3.1 and 3.2 thereof. In light chain shuffling, heavy chain variable regions of antibodies are combined with a repertoire of light chain variable region partners to identify new VL/VH combinations having high affinity for the target protein of interest. In this way, the antibody/fragment is optimised for very high binding affinity.

In some aspects, the antibody/fragment of the present invention comprises the CDRs (i.e. CDRs 1-3) of the VH and/or VL domains of an antibody clone described herein, or a variant thereof. In some embodiments, the antibody/fragment of the present invention comprises HC-CDRs 1-3 of an antibody clone described herein, or a variant thereof. In some embodiments, the antibody/fragment of the present invention comprises LC-CDRs 1-3 of an antibody clone described herein, or a variant thereof.

HC-CDRs 1-3 and LC-CDRs 1-3 of the antibody clones of the present disclosure are defined according to the Kabat definition (Kabat, et al., NIH Publication, 91-3242 (1991), which is hereby incorporated by reference in its entirety.

As used herein, a variant of a CDR may comprise e.g. 1 or 2 or 3 substitutions in the amino acid sequence of the CDR. As used herein, a variant of a VL or VH domain may comprise e.g. 1, 2, 3, 4, 5, 6, 7, 8, 9 or 10 substitutions in the amino acid sequence of the domain.

In some embodiments, the antibody/fragment of the present invention comprises HC-CDRs 1-3 of an antibody clone described herein, or a variant thereof, and LC-CDRs 1-3 of an antibody clone described herein, or a variant thereof.

In some aspects, the antibody/fragment of the present invention comprises the CDRs of the VH and/or VL domains of an antibody clone described herein, or a variant thereof. In some aspects, the antibody/fragment of the present invention comprises the VH and/or VL domains of an antibody clone described herein, or a variant thereof.

In some aspects, the antibody/fragment of the present invention comprises the CDRs of the VH and/or VL domains of a clone, or a variant thereof, selected from P1C1, P1C1_gl, P1C1_dm, P1C1_tm, 1G7, 2E3, 1E11, P1H4, P1B11, P1A8 or P2B4.

In some aspects, the antibody/fragment of the present invention comprises the VH and/or VL domains of a clone, or a variant thereof, selected from P1C1, P1C1_gl, P1C1_dm, P1C1_tm, 1G7, 2E3, 1E11, P1H4, P1B11, P1A8 or P2B4.

In some aspects, the antibody/fragment of the present invention comprises HC-CDRs 1-3 of the VH domain of an antibody clone described herein, or a variant thereof. In some aspects, the antibody/fragment of the present invention comprises the VH domain of a clone, or a variant thereof.

In some aspects, the antibody/fragment of the present invention comprises LC-CDRs 1-3 of the VL domain of an antibody clone described herein, or a variant thereof. In some aspects, the antibody/fragment of the present invention comprises the VL domain of a clone, or a variant thereof.

In some embodiments the antibody/fragment of the present invention comprises HC-CDRs 1-3 of the VH domain, or the VH domain, of an antibody clone selected from P1C1, P1C1_gl, P1C1_dm, P1C1_tm, 1G7, 2E3, 1E11, P1H4, P1B11, P1A8 or P2B4.

In some embodiments the antibody/fragment of the present invention comprises LC-CDRs 1-3 of the VL domain, or the VL domain, of an antibody clone selected from P1C1, P1C1_gl, P1C1_dm, P1C1_tm, 1G7, 2E3, 1E11, P1H4, P1B11, P1A8 or P2B4.

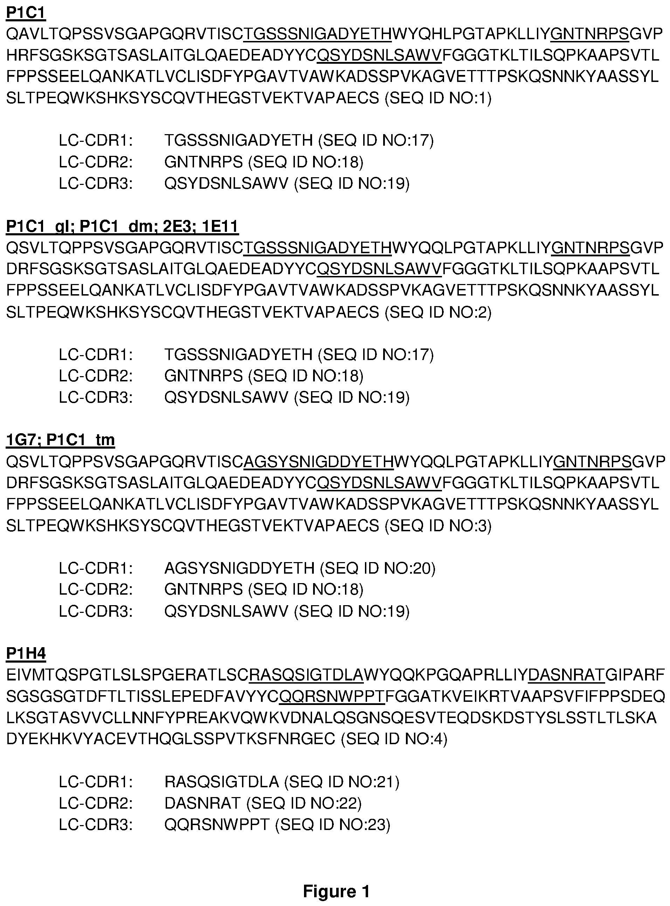

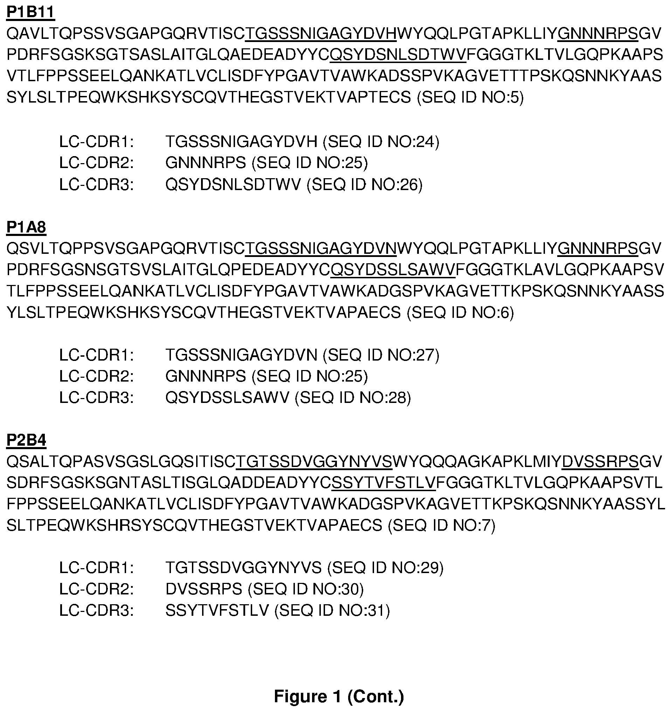

The amino acid sequences of the VL domains of antibody clones P1C1, P1C1_gl, P1C1_dm, P1C1_tm, 1G7, 2E3, 1E11, P1H4, P1B11, P1A8 or P2B4 are shown in Figure 1, as are the LC-CDRs 1-3, defined according to the Kabat system (Kabat, et al., NIH Publication, 91-3242 (1991). The amino acid sequences of the VH domains for P1C1, P1C1_gl, P1C1_dm, P1C1_tm, 1G7, 2E3, 1E11, P1H4, P1B11, P1A8 or P2B4 are shown in Figure 2, as are the HC-CDRs 1-3, defined according to the Kabat system.

Antibodies/fragments according to the present invention may comprise VL and/or VH chains comprising an amino acid sequence that has a high percentage sequence identity to one or more of the VL and/or VH amino acid sequences described herein. For example, antibodies according to the present invention include antibodies having a VL chain that comprises an amino acid sequence having at least 70%, more preferably one of at least 75%, 80%, 85%, 86%, 87%, 88%, 89%, 90%, 91%, 92%, 93%, 94%, 95%, 96%, 97%, 98%, 99%, or 100%, sequence identity to the VL chain amino acid sequence of one of SEQ ID NOs:1 to 7. Antibodies/fragments according to the present invention include antibodies having a VH chain that comprises an amino acid sequence having at least 70%, more preferably one of at least 75%, 80%, 85%, 86%, 87%, 88%, 89%, 90%, 91%, 92%, 93%, 94%, 95%, 96%, 97%, 98%, 99%, or 100%, sequence identity to the VH chain amino acid sequence of one of SEQ ID NOs:8 to 16.

Antibodies/fragments according to the present invention may comprise VL and/or VH chains encoded by a nucleic acid sequence that has a high percentage sequence identity to one or more of the VL and/or VH nucleic acid sequences described herein, or nucleic acid sequence encoding the same amino acid sequence as a result of codon degeneracy. For example, antibodies according to the present invention include antibodies having a VL chain encoded by a nucleic acid sequence having at least 70%, more preferably one of at least 75%, 80%, 85%, 86%, 87%, 88%, 89%, 90%, 91%, 92%, 93%, 94%, 95%, 96%, 97%, 98%, 99%, or 100%, sequence identity to the VL chain nucleic acid sequence of one of SEQ ID NOs:54 to 60 or nucleic acid sequence encoding the same amino acid sequence as one of SEQ ID NOs:54 to 60 as a result of codon degeneracy. Antibodies/fragments according to the present invention include antibodies having a VH chain encoded by a nucleic acid sequence having at least 70%, more preferably one of at least 75%, 80%, 85%, 86%, 87%, 88%, 89%, 90%, 91%, 92%, 93%, 94%, 95%, 96%, 97%, 98%, 99%, or 100%, sequence identity to the VH chain nucleic acid sequence of one of SEQ ID NOs:61 to 69 or nucleic acid sequence encoding the same amino acid sequence as one of SEQ ID NOs:61 to 69 as a result of codon degeneracy.

The light and heavy chain CDRs disclosed herein may also be particularly useful in conjunction with a number of different framework regions. Accordingly, light and/or heavy chains having LC-CDR1-3 or HC-CDR1-3 may possess an alternative framework region. Suitable framework regions are well known in the art and are described for example in M. Lefranc & G. Lefranc (2001) "The Immunoglobulin FactsBook", Academic Press, incorporated herein by reference.

Antibodies/fragments according to the present invention may be detectably labelled or, at least, capable of detection. For example, the antibody may be labelled with a radioactive atom or a coloured molecule or a fluorescent molecule or a molecule which can be readily detected in any other way. Suitable detectable molecules include fluorescent proteins, luciferase, enzyme substrates, radiolabels and binding moieties. Labelling may be by conjugation to the antibody/fragment. The antigen binding molecule may be directly labelled with a detectable label or it may be indirectly labelled. In some embodiments, the label may be selected from: a radio-nucleotide, positron-emitting radionuclide (e.g. for positron emission tomography (PET)), MRI contrast agent or fluorescent label.

Antibodies and antigen binding fragments according to the present invention may be conjugated to an effector moiety, e.g. drug moiety such as a cytotoxic small molecule. Such conjugates are useful for the targeted killing of cells expressing the target for which the antibody/antigen binding fragments is specific, through the targeted delivery of the effector moiety.

Alternatively, in some embodiments the antibodies and antigen binding fragments according to the present invention may be useful in conjunction with a secondary binding agent (e.g. secondary antibody) capable of binding to the antibody/fragment, which binding agent is conjugated to a drug moiety, e.g. a cytotoxic small molecule.

In some embodiments, the moiety is selected from a moiety described e.g. in Vankemmelbeke and Durrant Ther. Deliv. (2016) 7(3), 141-144 or Diamantis and Banjeri, British Journal of Cancer (2016) 114, 362-367, both of which are hereby incorporated by reference in their entirety. In some embodiments the drug moiety is selected from PNU159682 (PNU) and pyrrolobenzodiazepine (PBD). In some embodiments, the drug moiety is an anthracycline derivative.

Also provided by the present invention are isolated heavy chain variable region polypeptides, and isolated light chain variable region polypeptides.

In some aspects an isolated light chain variable region polypeptide is provided, comprising the LC-CDRs 1-3 of any one of the antibody clones described herein. In some aspects an isolated light chain variable region polypeptide is provided, comprising an amino acid sequence having at least 70%, more preferably one of at least 75%, 80%, 85%, 86%, 87%, 88%, 89%, 90%, 91%, 92%, 93%, 94%, 95%, 96%, 97%, 98%, 99%, or 100% sequence identity to the amino acid sequence of the light chain variable region of any one of the antibody clones described herein.

In some aspects an isolated heavy chain variable region polypeptide is provided, comprising the HC-CDRs 1-3 of any one of the antibody clones described herein. In some aspects an isolated heavy chain variable region polypeptide is provided, comprising an amino acid sequence having at least 70%, more preferably one of at least 75%, 80%, 85%, 86%, 87%, 88%, 89%, 90%, 91%, 92%, 93%, 94%, 95%, 96%, 97%, 98%, 99%, or 100% sequence identity to the amino acid sequence of the heavy chain variable region of any one of the antibody clones described herein.

### Functional properties of the antibodies/fragments

The antibodies and fragments of the present invention may be characterised by reference to certain functional properties. In particular, an antibody or antigen binding fragment according to the present invention may possess one or more of the following properties:
a) Specifically binds to peptide-MHC complex comprising a peptide of p53 and an MHC class I molecule, e.g. peptide-MHC complex comprising p53₁₂₅₋₁₃₄ and an MHC class I molecule comprising an MHC class I α-chain encoded by an HLA-A*24 allele;
b) Binds to peptide-MHC complex comprising a peptide of p53 and an MHC class I molecule (e.g. peptide-MHC complex comprising p53₁₂₅₋₁₃₄ and an MHC class I molecule comprising an MHC class I α-chain encoded by an HLA-A*24 allele) with an affinity of binding of ≤5 µM, e.g. as determined by surface plasmon resonance (SPR);
c) Does not specifically bind to MHC class I molecule (e.g. MHC class I molecule comprising an MHC class I α-chain encoded by an HLA-A*24 allele) in the absence of a peptide of p53 (e.g. in the absence of p53₁₂₅₋₁₃₄);
d) Does not specifically bind to a peptide-MHC complex (e.g. comprising an MHC class I molecule comprising an MHC class I α-chain encoded by an HLA-A*24 allele) which does not comprise a peptide of p53 (e.g. p53₁₂₅₋₁₃₄);
e) Does not specifically bind to a peptide of p53 (e.g. p53₁₂₅₋₁₃₄) in the absence of an MHC class I molecule (e.g. in the absence of MHC class I molecule comprising an MHC class I α-chain encoded by an HLA-A*24 allele);
f) Displays antibody-dependent cell-mediated cytotoxicity (ADCC) to cells comprising/expressing peptide-MHC complex comprising a peptide of p53 and an MHC class I molecule (e.g. peptide-MHC complex comprising p53₁₂₅₋₁₃₄ and an MHC class I molecule comprising an MHC class I α-chain encoded by an HLA-A*24 allele);
g) Does not display ADCC to cells comprising/expressing MHC class I molecule (e.g. MHC class I molecule comprising an MHC class I α-chain encoded by an HLA-A*24 allele) in the absence of a peptide of p53 (e.g. in the absence of p53₁₂₅₋₁₃₄);
h) Does not display ADCC to cells comprising/expressing peptide-MHC complex (e.g. comprising an MHC class I molecule comprising an MHC class I α-chain encoded by an HLA-A*24 allele) which does not comprise a peptide of p53 (e.g. p53₁₂₅₋₁₃₄);
i) Displays internalisation by cells comprising/expressing peptide-MHC complex comprising a peptide of p53 and an MHC class I molecule (e.g. peptide-MHC complex comprising p53₁₂₅₋₁₃₄ and an MHC class I molecule comprising an MHC class I α-chain encoded by an HLA-A*24 allele);
j) Does not display internalisation by cells comprising/expressing MHC class I molecule (e.g. MHC class I molecule comprising an MHC class I α-chain encoded by an HLA-A*24 allele) in the absence of a peptide of p53 (e.g. in the absence of p53₁₂₅₋₁₃₄);
k) Does not display internalisation by cells comprising/expressing peptide-MHC complex (e.g. comprising an MHC class I molecule comprising an MHC class I α-chain encoded by an HLA-A*24 allele) which does not comprise a peptide of p53 (e.g. p53₁₂₅₋₁₃₄);

An antibody/fragment that specifically binds to peptide-MHC complex comprising a peptide of p53 and an MHC class I molecule (e.g. peptide-MHC complex comprising p53₁₂₅₋₁₃₄ and an MHC class I molecule comprising an MHC class I α-chain encoded by an HLA-A*24 allele) binds with greater affinity, and/or with greater duration than it binds to other, non-target molecules/complexes.

In some embodiments the present antibodies/fragments may bind peptide-MHC complex comprising a peptide of p53 and an MHC class I molecule (e.g. peptide-MHC complex comprising p53₁₂₅₋₁₃₄ and an MHC class I molecule comprising an MHC class I α-chain encoded by an HLA-A*24 allele) with greater affinity than the affinity of binding to an MHC class I molecule (e.g. MHC class I molecule comprising an MHC class I α-chain encoded by an HLA-A*24 allele) in the absence of a peptide of p53 (e.g. in the absence of p53₁₂₅₋₁₃₄), and/or with greater affinity than the affinity of binding to a peptide-MHC complex (e.g. comprising an MHC class I molecule comprising an MHC class I α-chain encoded by an HLA-A*24 allele) which does not comprise a peptide of p53 (e.g. p53₁₂₅₋₁₃₄).

In some embodiments, the extent of binding of an antibody to a non-target is less than about 10% of the binding of the antibody to the target as measured, e.g., by ELISA, SPR, Bio-Layer Interferometry (BLI), MicroScale Thermophoresis (MST), or by a radioimmunoassay (RIA). Alternatively, the binding specificity may be reflected in terms of binding affinity, where the antibody/fragment of the present invention binds to peptide-MHC complex comprising a peptide of p53 and an MHC class I molecule (e.g. peptide-MHC complex comprising p53₁₂₅₋₁₃₄ and an MHC class I molecule comprising an MHC class I α-chain encoded by an HLA-A*24 allele) with a K_{D} that is at least 0.1 order of magnitude (i.e. 0.1 x 10ⁿ, where n is an integer representing the order of magnitude) greater than the K_{D} towards a non-target molecule/complex. This may optionally be one of at least 0.2, 0.3, 0.4, 0.5, 0.6, 0.7, 0.8, 0.9, 1.0, 1.5, or 2.0.

Binding affinity of an antibody or antigen-binding fragment for its target is often described in terms of its dissociation constant (K_{D}). Binding affinity can be measured by methods known in the art, such as by ELISA, Surface Plasmon Resonance (SPR; see e.g. Hearty et al., Methods Mol Biol (2012) 907:411-442; or Rich et al., Anal Biochem. 2008 Feb 1; 373(1):112-20), Bio-Layer Interferometry (see e.g. Lad et al., (2015) J Biomol Screen 20(4): 498-507; or Concepcion et al., Comb Chem High Throughput Screen. 2009 Sep; 12(8):791-800), MicroScale Thermophoresis (MST) analysis (see e.g. Jerabek-Willemsen et al., Assay Drug Dev Technol. 2011 Aug; 9(4): 342-353), or by a radiolabelled antigen binding assay (RIA) performed with the Fab version of the antibody and antigen molecule.

In some embodiments, the antibody/fragment according to the present invention binds to peptide-MHC complex comprising a peptide of p53 and an MHC class I molecule (e.g. peptide-MHC complex comprising p53₁₂₅₋₁₃₄ and an MHC class I molecule comprising an MHC class I α-chain encoded by an HLA-A*24 allele) with a K_{D} of 10 µM or less, preferably one of ≤5 µM, ≤1 µM, ≤900 nM, ≤800 nM, ≤700 nM, ≤600 nM, ≤500 nM, ≤400 nM, ≤300 nM, ≤200 nM, ≤100 nM, ≤75 nM, ≤50 nM, ≤40 nM, ≤30 nM, ≤20 nM, ≤15 nM, ≤12.5 nM, ≤10 nM, ≤9 nM, ≤8 nM, ≤7 nM, ≤6 nM, ≤5 nM, ≤4 nM ≤3 nM, ≤2 nM, ≤1 nM, ≤500 pM, e.g. as determined by analysis by SPR.

In some embodiments, the antibody/fragment according to the present invention binds to peptide-MHC complex comprising a peptide of p53 and an MHC class I molecule (e.g. peptide-MHC complex comprising p53₁₂₅₋₁₃₄ and an MHC class I molecule comprising an MHC class I α-chain encoded by an HLA-A*24 allele) with an affinity of binding (e.g. as determined by ELISA) of EC50 = 1000 ng/ml or less, preferably one of ≤900 ng/ml, ≤800 ng/ml, ≤700 ng/ml, ≤600 ng/ml, ≤500 ng/ml, ≤400 ng/ml, ≤300 ng/ml, ≤200 ng/ml, ≤100 ng/ml, ≤90 ng/ml, ≤80 ng/ml, ≤70 ng/ml, ≤60 ng/ml, ≤50 ng/ml, ≤40 ng/ml, ≤30 ng/ml, ≤20 ng/ml, ≤15 ng/ml, ≤10 ng/ml, ≤7.5 ng/ml, ≤5 ng/ml, ≤2.5 ng/ml, or ≤1 ng/ml.

Affinity of binding to by an antibody/fragment may be analysed *in vitro* by ELISA assay. Suitable assays are well known in the art and can be performed by the skilled person, for example, as described in Antibody Engineering, vol. 1 (2nd Edn), Springer Protocols, Springer (2010), Part V, pp657-665.

In some embodiments, the antibody/antigen binding fragment displays antibody-dependent cell-mediated cytotoxicity (ADCC) to cells comprising/expressing peptide-MHC complex comprising a peptide of p53 and an MHC class I molecule (e.g. peptide-MHC complex comprising p53₁₂₅₋₁₃₄ and an MHC class I molecule comprising an MHC class I α-chain encoded by an HLA-A*24 allele). An antibody/antigen binding fragment which displays ADCC to cells comprising/expressing peptide-MHC complex comprising a peptide of p53 and an MHC class I molecule (e.g. peptide-MHC complex comprising p53₁₂₅₋₁₃₄ and an MHC class I molecule comprising an MHC class I α-chain encoded by an HLA-A*24 allele) typically comprises an Fc region.

Antibody-dependent cell-mediated cytotoxicity (ADCC) refers to a cell-mediated immune response in which an effector immune cell lyses a target cell coated with antibody bound to antigen expressed by the target cell. Effector immune cells including e.g. NK cells typically recognise antibody-coated cells through binding of the Fc region of the antibody to Fc receptors expressed by the effector cells. Cross-linking of the Fc receptors results in release from the effector cell of cytotoxic factors such as perforin and granzymes, causing lysis of the target cell.

The ability of a given antibody/antigen binding fragment to elicit ADCC can be measured e.g. by analysis using an *in vitro* cell killing assay. Such assays usually involve *in vitro* culture of cells expressing the target antigen in the presence of the antibody and effector immune cells, and measuring the amount of cell killing after a period of time. ADCC assays include e.g. ⁵¹Cr release assay, e.g. as described in Nelson et al. 2001, Curr Protoc Immunol. Chapter 7:Unit 7.27, which is hereby incorporated by reference in its entirety. Briefly, target cells are treated with ⁵¹Cr, which they internalise. Lysis of the target cells by ADCC results in the release of the radioactive ⁵¹Cr into the cell culture supernatant, which can be detected and quantified. An antibody/antigen binding fragment which displays ADCC to a given target cell causes more lysis of cells than the level of cell lysis observed in the absence of the antibody/antigen binding fragment, or in the presence of an antibody/fragment specific for a target antigen which is not expressed by the cell.

In some embodiments, the antibody/antigen binding fragment according to the present invention results in a level of cell killing of cells comprising/expressing peptide-MHC complex comprising a peptide of p53 and an MHC class I molecule (e.g. peptide-MHC complex comprising p53₁₂₅₋₁₃₄ and an MHC class I molecule comprising an MHC class I α-chain encoded by an HLA-A*24 allele) which is more than 1 times, e.g. ≥1.01 times, ≥1.02 times, ≥1.03 times, ≥1.04 times, ≥1.05 times, ≥1.1 times, ≥1.2 times, ≥1.3 times, ≥1.4 times, ≥1.5 times, ≥1.6 times, ≥1.7 times, ≥1.8 times, ≥1.9 times, ≥2 times, ≥3 times, ≥4 times, ≥5 times, ≥6 times, ≥7 times, ≥8 times, ≥9 times, or ≥10 times the level of cell killing in the absence of the antibody/antigen binding fragment, or in the presence of an antibody/fragment specific for a target antigen which is not expressed by the cells, in a comparable assay.

In some embodiments, the antigen/antigen binding fragment according to the present invention displays internalisation by cells comprising/expressing peptide-MHC complex comprising a peptide of p53 and an MHC class I molecule (e.g. peptide-MHC complex comprising p53₁₂₅₋₁₃₄ and an MHC class I molecule comprising an MHC class I α-chain encoded by an HLA-A*24 allele).

'Internalisation' refers to uptake of antibody into the cell, e.g. by endocytosis of antibody bound to antigen expressed at the cell surface. Antibody internalisation can be analysed by methods well known to the skilled person, including analysis of antibody localisation/trafficking, e.g. of antibody labelled with a detectable moiety. For example, antibody internalisation can be measured as described in the experimental examples herein, using antibody labelled with a pH-sensitive dye producing a detectable signal when trafficked to the endosome. Assay for analysis of antibody internalisation are described for example in Nath et al., J Immunol Methods. 2016; 431:11-21, and Liao-Chan et al., PLoS One. 2015; 10(4): e0124708, both of which are hereby incorporated by reference in their entirety.

In some embodiments, the antibody/antigen binding fragment according to the present invention is internalised by cells comprising/expressing peptide-MHC complex comprising a peptide of p53 and an MHC class I molecule (e.g. peptide-MHC complex comprising p53₁₂₅₋₁₃₄ and an MHC class I molecule comprising an MHC class I α-chain encoded by an HLA-A*24 allele) to an extent which is more than 1 times, e.g. ≥1.01 times, ≥1.02 times, ≥1.03 times, ≥1.04 times, ≥1.05 times, ≥1.1 times, ≥1.2 times, ≥1.3 times, ≥1.4 times, ≥1.5 times, ≥1.6 times, ≥1.7 times, ≥1.8 times, ≥1.9 times, ≥2 times, ≥3 times, ≥4 times, ≥5 times, ≥6 times, ≥7 times, ≥8 times, ≥9 times, or ≥10 times the level of internalisation by those cells of an appropriate control antibody/fragment (e.g. isotype control), in a comparable assay.

In some embodiments, internalisation of the antibody/antigen binding fragment according to the present invention by cells comprising/expressing peptide-MHC complex comprising a peptide of p53 and an MHC class I molecule (e.g. peptide-MHC complex comprising p53₁₂₅₋₁₃₄ and an MHC class I molecule comprising an MHC class I α-chain encoded by an HLA-A*24 allele) is more than 1 times, e.g. ≥1.01 times, ≥1.02 times, ≥1.03 times, ≥1.04 times, ≥1.05 times, ≥1.1 times, ≥1.2 times, ≥1.3 times, ≥1.4 times, ≥1.5 times, ≥1.6 times, ≥1.7 times, ≥1.8 times, ≥1.9 times, ≥2 times, ≥3 times, ≥4 times, ≥5 times, ≥6 times, ≥7 times, ≥8 times, ≥9 times, or ≥10 times the level of internalisation by cells not comprising/expressing peptide-MHC complex comprising a peptide of p53 and an MHC class I molecule (e.g. cells not comprising/expressing peptide-MHC complex comprising p53₁₂₅₋₁₃₄ and an MHC class I molecule comprising an MHC class I α-chain encoded by an HLA-A*24 allele), in a comparable assay.

### Nucleic acids/vectors

The present invention provides a nucleic acid encoding an antibody, antigen binding fragment or CAR according to the present invention. In some embodiments, the nucleic acid is purified or isolated, e.g. from other nucleic acid, or naturally-occurring biological material.

The present invention also provides a vector comprising nucleic acid encoding an antibody, antigen binding fragment or CAR according to the present invention.

A "vector" as used herein is a nucleic acid (DNA or RNA) used as a vehicle to transfer exogenous nucleic acid into a cell. The vector may be an expression vector for expression of the nucleic acid in the cell. Such vectors may include a promoter sequence operably linked to the nucleic acid encoding the sequence to be expressed. A vector may also include a termination codon and expression enhancers. Any suitable vectors, promoters, enhancers and termination codons known in the art may be used to express an antibody, antigen binding fragment or CAR according to the invention from a vector according to the invention.

In this specification the term "operably linked" may include the situation where a selected nucleic acid sequence and regulatory nucleic acid sequence (e.g. promoter and/or enhancer) are covalently linked in such a way as to place the expression of the nucleotide sequence under the influence or control of the regulatory sequence (thereby forming an expression cassette). Thus a regulatory sequence is operably linked to the selected nucleic acid sequence if the regulatory sequence is capable of effecting transcription of the nucleic acid sequence. Where appropriate, the resulting transcript may then be translated into a desired polypeptide.

The nucleic acid and/or vector according to the present invention may be provided for introduction into a cell, e.g. a primary human immune cell. Suitable vectors include plasmids, binary vectors, DNA vectors, mRNA vectors, viral vectors (e.g. gammaretroviral vectors (e.g. murine Leukemia virus (MLV)-derived vectors), lentiviral vectors, adenovirus vectors, adeno-associated virus vectors, vaccinia virus vectors and herpesvirus vectors), transposon-based vectors, and artificial chromosomes (e.g. yeast artificial chromosomes), e.g. as described in Maus et al., Annu Rev Immunol (2014) 32:189-225 or Morgan and Boyerinas, Biomedicines 2016 4, 9, which are both hereby incorporated by reference in its entirety. In some embodiments, the viral vector may be a lentiviral, retroviral, adenoviral, or Herpes Simplex Virus vector. In some embodiments, the lentiviral vector may be pELNS, or may be derived from pELNS. In some embodiments, the vector may be a vector encoding CRISPR/Cas9.

### Cells comprising/expressing the antibodies/fragments/CARs

The present invention also provides a cell comprising or expressing an antibody, antigen binding fragment or CAR, according to the present invention. Also provided is a cell comprising or expressing a nucleic acid or vector according to the invention.

The cell may be a eukaryotic cell, e.g. a mammalian cell. The mammal may be a human, or a non-human mammal (e.g. rabbit, guinea pig, rat, mouse or other rodent (including any animal in the order *Rodentia*), cat, dog, pig, sheep, goat, cattle (including cows, e.g. dairy cows, or any animal in the order *Bos*), horse (including any animal in the order *Equidae*), donkey, and non-human primate).

In some embodiments, the cell may be from, or may have been obtained from, a human subject.

The cell may be an immune cell. The cell may be a cell of hematopoietic origin, e.g. a neutrophil, eosinophil, basophil, dendritic cell, lymphocyte, or monocyte. The lymphocyte may be e.g. a T cell, B cell, NK cell, NKT cell or innate lymphoid cell (ILC), or a precursor thereof. The cell may express e.g. CD3 polypeptides (e.g. CD3γ CD3ε CD3ζ or CD3δ), TCR polypeptides (TCRα or TCRβ), CD27, CD28, CD4 or CD8. In some embodiments, the cell is a T cell. In some embodiments, the T cell is a CD3+ T cell. In some embodiments, the T cell is a CD3+, CD8+ T cell. In some embodiments, the T cell is a cytotoxic T cell (e.g. a cytotoxic T lymphocyte (CTL)).

In some embodiments, the cell is an antigen-specific T cell. In embodiments herein, a "antigen-specific" T cell is a cell which displays certain functional properties of a T cell in response to the antigen for which the T cell is specific, or a cell expressing said antigen. In some embodiments, the properties are functional properties associated with effector T cells, e.g. cytotoxic T cells. In some embodiments, an antigen-specific T cell may display one or more of the following properties: cytotoxicity, e.g. to a cell comprising/expressing antigen for which the T cell is specific; proliferation, IFNγ expression, CD107a expression, IL-2 expression, TNFα expression, perforin expression, granzyme expression, granulysin expression, and/or FAS ligand (FASL) expression, e.g. in response to antigen for which the T cell is specific or a cell comprising/expressing antigen for which the T cell is specific. In some embodiments, the antigen for which the T cell is specific may be a peptide or polypeptide of a virus, e.g. Epstein-Barr virus (EBV), influenza virus, measles virus, hepatitis B virus (HBV), hepatitis C virus (HCV), human immunodeficiency virus (HIV), lymphocytic choriomeningitis virus (LCMV), Herpes simplex virus (HSV) or human papilloma virus (HPV).

The present invention also provides a method for producing a cell comprising a nucleic acid or vector according to the present invention, comprising introducing a nucleic acid or vector according to the present invention into a cell. The present invention also provides a method for producing a cell expressing an antibody, antigen binding fragment or CAR, according to the present invention, comprising introducing a nucleic acid or vector according to the present invention in a cell. In some embodiments, the methods additionally comprise culturing the cell under conditions suitable for expression of the nucleic acid or vector by the cell. In some embodiments, the methods are performed *in vitro.*

In some embodiments, introducing an isolated nucleic acid or vector according to the invention into a cell comprises transduction, e.g. retroviral transduction. Accordingly, in some embodiments the isolated nucleic acid or vector is comprised in a viral vector, or the vector is a viral vector. In some embodiments, the method comprises introducing a nucleic acid or vector according to the invention by electroporation, e.g. as described in Koh et al., Molecular Therapy - Nucleic Acids (2013) 2, e114, which is hereby incorporated by reference in its entirety.

The present invention also provides cells obtained or obtainable by the methods for producing a cell according to the present invention.

### Therapeutic applications

Antibodies, antigen binding fragments, CARs, nucleic acids, vectors, cells and compositions according to the present invention may be provided for use in methods of medical treatment or prevention of diseases/conditions, or for the alleviation of the symptoms of a disease/condition. They may be administered to subjects having a disease/condition in need of treatment, and/or to subjects at risk of such developing or contracting the disease/condition.

The subject may be any animal or human. The subject is preferably mammalian, more preferably human. The subject may be a non-human mammal, but is more preferably human. The subject may be male or female. The subject may be a patient. A subject may have been diagnosed with a disease or condition requiring treatment, or be suspected of having such a disease or condition.

Treatment or alleviation of a disease/disorder may be effective to prevent progression of the disease/disorder, e.g. to prevent worsening of the condition or to slow the rate of development. In some embodiments treatment or alleviation may lead to an improvement in the disease/disorder, e.g. a reduction in the symptoms of the disease/disorder or reduction in some other correlate of the severity/activity of the disease/disorder.

Prevention of a disease/disorder may refer to prevention of a worsening of the condition or prevention of the development of the disease/disorder, e.g. preventing an early stage disease/disorder developing to a later, chronic, stage. For example, in a cancer, prevention may e.g. be prevention of development of the cancer, or prevention of metastasis.

In some embodiments, the disease/condition may be a disease/condition associated with p53, e.g. a disease/condition associated with mutation in TP53. In some embodiments, a disease/condition associated with mutation in TP53 may be a disease/condition for which mutation in TP53 is a risk factor for the development or progression of the disease/condition.

The disease/condition may be associated with expression of a peptide of p53 in complex with MHC class I. In some embodiments, effector cells of the disease/condition (e.g. cells directly or indirectly implicated in the pathology of the disease/condition) express a peptide of p53 in complex with MHC class I. In some embodiments the disease/condition is associated with expression of a peptide of p53 in complex with MHC class I comprising an α-chain encoded by an HLA-A*24 allele. In some embodiments the disease/condition is associated with expression of p53₁₂₅₋₁₃₄ in complex with MHC class I comprising an α-chain encoded by an HLA-A*24 allele.

As discussed herein and reviewed for example in Royd et al. 2006;, Cell Death Differ. 13(6):1017-26 and Vousden and Lane 2007, Nat Rev Mol Cell Biol 8, 275-283, p53 is a tumour suppressor, and mutation in TP53 is implicated in the development/progression of a wide variety of cancers. Cancerous cells encoding or expressing a mutant p53 peptide/polypeptide often present of peptides of p53 in complex with MHC class I at the cell surface.

In some embodiments, the disease/condition to be treated/prevented according to the present invention is a cancer. In some embodiments, the cancer is associated with mutation in TP53. In some embodiments, the cancer comprises cells encoding/expressing a mutant p53 peptide/polypeptide.

A cancer may be determined to encode or express a mutant p53 peptide/polypeptide by any suitable means, which are well known to the skilled person, e.g. based on analysis of a biological sample. A cancer encoding a mutant p53 polypeptide may be identified on the basis of detection of nucleic acid encoding the mutation, e.g. by DNA sequencing etc. A cancer expressing a mutant p53 polypeptide may be identified by detection of expression of a mutant p53 peptide/polypeptide. Expression may be gene expression or protein expression. Gene expression can be determined e.g. by detection of mRNA encoding a mutant p53 peptide/polypeptide, for example by quantitative real-time PCR (qRT-PCR). Protein expression can be determined e.g. by detection of mutant p53 peptide/polypeptide, for example by antibody-based methods, for example by western blot, immunohistochemistry, immunocytochemistry, flow cytometry, ELISA.

In embodiments herein, the cell or cells of a cancer may be of a tumor.

In some embodiments the cancer comprises cells expressing a peptide of p53 in complex with MHC class I. In some embodiments the cancer comprises cells expressing MHC class I comprising an α-chain encoded by an HLA-A*24 allele. In some embodiments the cancer comprises cells expressing a peptide of p53 in complex with MHC class I comprising an α-chain encoded by an HLA-A*24 allele. In some embodiments the cancer comprises cells expressing p53₁₂₅₋₁₃₄ in complex with MHC class I comprising an α-chain encoded by an HLA-A*24 allele.

In some embodiments, the antibodies/fragments of the present invention are able to bind to and cause ADCC of cells expressing the target peptide-MHC complex. In some embodiments, the antibodies/fragments bind to and are internalised by cells expressing the target peptide-MHC complex, and are therefore useful for the targeted delivery e.g. of a drug moiety, e.g. a cytotoxic small molecule, to cells expressing the target peptide-MHC complex.

In some embodiments, the treatment may be aimed at reducing the number of cells expressing the target antigen for the antibody/fragment/CAR according to the present invention.

In some embodiments, the treatment may comprise modifying a cell or population of cells to comprise/express an antibody/antigen binding fragment, CAR, nucleic acid or vector of the present invention, which may then be useful to reduce the number of cells expressing the target antigen for the antibody/fragment/CAR. In some embodiments, the treatment may comprise administering to a subject a cell or population of cells modified to comprise/express an antibody/antigen binding fragment, CAR, nucleic acid or vector of the present invention.

The cancer may be any unwanted cell proliferation (or any disease manifesting itself by unwanted cell proliferation), neoplasm or tumor or increased risk of or predisposition to the unwanted cell proliferation, neoplasm or tumor. The cancer may be benign or malignant and may be primary or secondary (metastatic). A neoplasm or tumor may be any abnormal growth or proliferation of cells and may be located in any tissue. Examples of tissues include the adrenal gland, adrenal medulla, anus, appendix, bladder, blood, bone, bone marrow, brain, breast, cecum, central nervous system (including or excluding the brain) cerebellum, cervix, colon, duodenum, endometrium, epithelial cells (e.g. renal epithelia), gallbladder, oesophagus, glial cells, heart, ileum, jejunum, kidney, lacrimal glad, larynx, liver, lung, lymph, lymph node, lymphoblast, maxilla, mediastinum, mesentery, myometrium, nasopharynx, omentum, oral cavity, ovary, pancreas, parotid gland, peripheral nervous system, peritoneum, pleura, prostate, salivary gland, sigmoid colon, skin, small intestine, soft tissues, spleen, stomach, testis, thymus, thyroid gland, tongue, tonsil, trachea, uterus, vulva, white blood cells.

Tumors to be treated may be nervous or non-nervous system tumors. Nervous system tumors may originate either in the central or peripheral nervous system, e.g. glioma, medulloblastoma, meningioma, neurofibroma, ependymoma, Schwannoma, neurofibrosarcoma, astrocytoma and oligodendroglioma. Non-nervous system cancers/tumors may originate in any other non-nervous tissue, examples include melanoma, mesothelioma, lymphoma, myeloma, leukemia, Non-Hodgkin's lymphoma (NHL), Hodgkin's lymphoma, chronic myelogenous leukemia (CML), acute myeloid leukemia (AML), myelodysplastic syndrome (MDS), cutaneous T-cell lymphoma (CTCL), chronic lymphocytic leukemia (CLL), hepatoma, epidermoid carcinoma, prostate carcinoma, breast cancer, lung cancer, colon cancer, ovarian cancer, pancreatic cancer, thymic carcinoma, NSCLC, haematologic cancer and sarcoma.

In some embodiments, methods are for the treatment/prevention of a cancer, e.g. an epithelial cell cancer, breast cancer, gastrointestinal cancer (e.g. esophageal cancer, stomach cancer, pancreatic cancer, liver cancer (e.g. HCC), gallbladder cancer, colorectal cancer, anal cancer, gastrointestinal carcinoid tumor), and lung cancer (e.g. non-small cell lung cancer (NSCLC) or small cell lung cancer (SCLC))).

In some embodiments, the cancer to be treated is one or more of nasopharyngeal carcinoma (NPC; e.g. Epstein-Barr Virus (EBV)-positive NPC), cervical carcinoma (CC; e.g. human papillomavirus (HPV)-positive CC), oropharyngeal carcinoma (OPC; e.g. HPV-positive OPC), gastric carcinoma (GC; e.g. EBV-positive GC), hepatocellular carcinoma (HCC; e.g. Hepatitis B Virus (HBV)-positive HCC), lung cancer (e.g. non-small cell lung cancer (NSCLC)) and head and neck cancer (e.g. cancer originating from tissues of the lip, mouth, nose, sinuses, pharynx or larynx, e.g. head and neck squamous cell carcinoma (HNSCC)).

In some embodiments, the cancer may be associated with increased p53 gene or protein expression. For example, cells of the cancer may have increased expression of p53 as compared to comparable, non-cancerous cells, or may be associated with increased expression of p53 by other cells (e.g. non-cancerous cells) as compared to the level of expression by comparable cells in the absence of a cancer (e.g. in a healthy control subject).

In embodiments of the present invention, a method of treatment or prophylaxis may comprise adoptive cell transfer of immune cells. Adoptive cell transfer (ACT) generally refers to a process by which cells (e.g. immune cells) are obtained from a subject, typically by drawing a blood sample from which the cells are isolated. The cells are then typically treated or altered in some way, and then administered either to the same subject or to a different subject. The treatment is typically aimed at providing population of cells with certain desired characteristics to a subject, or increasing the frequency of cells with such characteristics in that subject. Adoptive transfer of T cells is described, for example, in Kalos and June 2013, Immunity 39(1): 49-60, which is hereby incorporated by reference in its entirety.

In the present invention, adoptive transfer may performed with the aim of introducing a cell or population of cells into a subject, and/or increasing the frequency of a cell or population of cells in a subject. In some embodiments, adoptive transfer may be of a cell comprising/expressing an antibody, fragment or CAR according to the present invention.

The cell may e.g. be a neutrophil, eosinophil, basophil, dendritic cell, lymphocyte, or monocyte. The lymphocyte may be e.g. a T cell, B cell, NK cell, NKT cell or innate lymphoid cell (ILC), or a precursor thereof. In some embodiments, the cell is a T cell. In some embodiments, the T cell is a CD3+ T cell. In some embodiments, the T cell is a CD3+, CD8+ T cell. In some embodiments, the T cell is a cytotoxic T cell (e.g. a cytotoxic T lymphocyte (CTL)). In some embodiments, the T cell is a virus-specific T cell. In some embodiments, the T cell is specific for EBV, HPV, HBV, HCV or HIV.

The present invention provides a method of treating or presenting a disease or condition in a subject, the method comprising modifying at least one cell obtained from a subject to express or comprise an antibody, fragment, CAR, nucleic acid or vector according to the present invention, optionally expanding the modified at least one cell, and administering the modified at least one cell to a subject.

The present invention also provides a method of killing a tumour cell, the method comprising administering to the cell a therapeutically effective amount of an antibody, antigen binding fragment, CAR, nucleic acid, vector or composition according to the present invention. The method may be performed *in* vitro or *in vivo.* Also provided is a method of killing a tumour cell in a subject, the method comprising administering to the subject a therapeutically effective amount of an antibody, antigen binding fragment, CAR, nucleic acid, vector or composition according to the present invention. In some embodiments the method of killing a tumour cell comprises administering the antibody, fragment, CAR, nucleic acid, vector or composition in combination with a therapeutic agent as described herein. Killing of a tumour cell may, for example, be as a result of membrane disruption, cell lysis, induction of apoptosis, antibody dependent cell-mediated cytotoxicity (ADCC), complement dependent cytotoxicity (CDC), or through the action of a drug conjugated to the antibody or antigen binding fragment.

In some embodiments, the method comprises:
(a) isolating at least one cell from a subject;
(b) modifying the at least one cell to express or comprise an antibody, antigen binding fragment, CAR, nucleic acid or vector according to the present invention,
(c) optionally expanding the modified at least one cell, and;
(d) administering the modified at least one cell to a subject.

In some embodiments, the subject from which the cell is isolated is the subject administered with the modified cell (i.e., adoptive transfer is of autologous cells). In some embodiments, the subject from which the cell is isolated is a different subject to the subject to which the modified cell is administered (i.e., adoptive transfer is of allogenic cells).

The at least one cell modified according to the present invention can be modified according to methods well known to the skilled person. The modification may comprise nucleic acid transfer for permanent or transient expression of the transferred nucleic acid.

Any suitable genetic engineering platform may be used to modify a cell according to the present invention. Suitable methods for modifying a cell include the use of genetic engineering platforms such as gammaretroviral vectors, lentiviral vectors, adenovirus vectors, DNA transfection, transposon-based gene delivery and RNA transfection, for example as described in Maus et al., Annu Rev Immunol (2014) 32:189-225, incorporated by reference hereinabove.

In some embodiments the method may comprise one or more of the following steps: taking a blood sample from a subject; isolating and/or expanding at least one cell from the blood sample; culturing the at least one cell in *in vitro* or *ex vivo* cell culture; introducing into the at least one cell an antibody, antigen binding fragment, CAR, nucleic acid, or vector according to the present invention, thereby modifying the at least one cell; expanding the at least one modified cell; collecting the at least one modified cell; mixing the modified cell with an adjuvant, diluent, or carrier; administering the modified cell to a subject.

In some embodiments, the methods may additionally comprise treating the cell to induce/enhance expression of the antibody/fragment, CAR, nucleic acid, or vector. For example, the nucleic acid/vector may comprise a control element for inducible upregulation of expression of the antibody/fragment or CAR, from the nucleic acid/vector in response to treatment with a particular agent. In some embodiments, treatment may be *in vivo* by administration of the agent to a subject having been administered with a modified cell according to the invention. In some embodiments, treatment may be *ex vivo* or *in vitro* by administration of the agent to cells in culture *ex vivo* or *in vitro.*

The skilled person is able to determine appropriate reagents and procedures for adoptive transfer of cells according to the present invention, for example by reference to Dai et al., 2016 J Nat Cancer Inst 108(7): djv439, which is incorporated by reference in its entirety.

In a related aspect, the present invention provides a method of preparing a modified cell, the method comprising introducing into a cell a CAR, nucleic acid or vector according to the present invention, thereby modifying the at least one cell. The method is preferably performed *in vitro* or *ex vivo.*

In one aspect, the present invention provides a method of treating or preventing a disease or condition in a subject, comprising:
(a) isolating at least one cell from a subject;
(b) introducing into the at least one cell the nucleic acid or vector according to the present invention, thereby modifying the at least one cell; and
(c) administering the modified at least one cell to a subject.

In some embodiments, the method additionally comprises therapeutic or prophylactic intervention, e.g. for the treatment or prevention of a cancer. In some embodiments, the therapeutic or prophylactic intervention is selected from chemotherapy, immunotherapy, radiotherapy, surgery, vaccination and/or hormone therapy.

Methods of treatment described herein may optionally include the co-administration of biological adjuvants (e.g., interleukins, cytokines, Bacillus Comette-Guerin, monophosphoryl lipid A, etc.) in combination with conventional therapies for treating cancer such as treatment with an agent for treating cancer (e.g. chemotherapy), radiation, or surgery. Methods of medical treatment may also involve *in vivo, ex vivo,* and adoptive immunotherapies, including those using autologous and/or heterologous cells or immortalized cell lines. Methods of treatment described herein may optionally involve boosting MHC class I expression, for example by interferon treatment or HDAC inhibitor treatment.

Antibodies, fragments, CARs nucleic acids, vectors and cells according to the present invention may be formulated as pharmaceutical compositions or medicaments for clinical use and may comprise a pharmaceutically acceptable carrier, diluent, excipient or adjuvant. The composition may be formulated for topical, parenteral, systemic, intracavitary, intravenous, intra-arterial, intramuscular, intrathecal, intraocular, intraconjunctival, intratumoral, subcutaneous, intradermal, intrathecal, oral or transdermal routes of administration which may include injection or infusion. Suitable formulations may comprise the Antibody, fragment, CAR, nucleic acid, vector, or cell in a sterile or isotonic medium. Medicaments and pharmaceutical compositions may be formulated in fluid, including gel, form. Fluid formulations may be formulated for administration by injection or infusion (e.g. via catheter) to a selected region of the human or animal body.

In accordance with the present invention methods are also provided for the production of pharmaceutically useful compositions, such methods of production may comprise one or more steps selected from: isolating an antibody, fragment, CAR, nucleic acid, vector, or cell as described herein; and/or mixing an antibody, fragment, CAR, nucleic acid, vector, or cell as described herein with a pharmaceutically acceptable carrier, adjuvant, excipient or diluent.

For example, a further aspect of the present invention relates to a method of formulating or producing a medicament or pharmaceutical composition for use in a method of medical treatment, the method comprising formulating a pharmaceutical composition or medicament by mixing an antibody, fragment, CAR, nucleic acid, vector, or cell as described herein with a pharmaceutically acceptable carrier, adjuvant, excipient or diluent.

Administration of an antibody, fragment, CAR, nucleic acid, vector, cell or composition according to the invention is preferably in a "therapeutically effective" or "prophylactically effective" amount, this being sufficient to show benefit to the subject. The actual amount administered, and rate and time-course of administration, will depend on the nature and severity of the disease or disorder. Prescription of treatment, e.g. decisions on dosage etc., is within the responsibility of general practitioners and other medical doctors, and typically takes account of the disease/disorder to be treated, the condition of the individual subject, the site of delivery, the method of administration and other factors known to practitioners. Examples of the techniques and protocols mentioned above can be found in Remington's Pharmaceutical Sciences, 20th Edition, 2000, pub. Lippincott, Williams & Wilkins.

Administration may be alone or in combination with other treatments, either simultaneously or sequentially dependent upon the condition to be treated. The antibody, fragment, CAR, nucleic acid, vector, cell or composition according to the present invention and a therapeutic agent may be administered simultaneously or sequentially.

In some embodiments, treatment with an antibody, fragment, CAR, nucleic acid, vector, cell or composition of the present invention is used in a method of prophylactic intervention, e.g. vaccination.

In some embodiments, treatment with an antibody, fragment, CAR, nucleic acid, vector, cell or composition of the present invention may be accompanied by other therapeutic or prophylactic intervention, e.g. chemotherapy, immunotherapy, radiotherapy, surgery, vaccination and/or hormone therapy.

Simultaneous administration refers to administration of the antibody, fragment, CAR, nucleic acid, vector, cell or composition and therapeutic agent together, for example as a pharmaceutical composition containing both agents (combined preparation), or immediately after each other and optionally via the same route of administration, e.g. to the same artery, vein or other blood vessel. Sequential administration refers to administration of one of the antibody, fragment, CAR, nucleic acid, vector, cell or composition or therapeutic agent followed after a given time interval by separate administration of the other agent. It is not required that the two agents are administered by the same route, although this is the case in some embodiments. The time interval may be any time interval.

Chemotherapy and radiotherapy respectively refer to treatment of a cancer with a drug or with ionising radiation (e.g. radiotherapy using X-rays or γ-rays). The drug may be a chemical entity, e.g. small molecule pharmaceutical, antibiotic, DNA intercalator, protein inhibitor (e.g. kinase inhibitor), or a biological agent, e.g. antibody, antibody fragment, nucleic acid or peptide aptamer, nucleic acid (e.g. DNA, RNA), peptide, polypeptide, or protein. The drug may be formulated as a pharmaceutical composition or medicament. The formulation may comprise one or more drugs (e.g. one or more active agents) together with one or more pharmaceutically acceptable diluents, excipients or carriers.

A treatment may involve administration of more than one drug. A drug may be administered alone or in combination with other treatments, either simultaneously or sequentially dependent upon the condition to be treated. For example, the chemotherapy may be a co-therapy involving administration of two drugs, one or more of which may be intended to treat the cancer.

The chemotherapy may be administered by one or more routes of administration, e.g. parenteral, intravenous injection, oral, subcutaneous, intradermal or intratumoral.

The chemotherapy may be administered according to a treatment regime. The treatment regime may be a pre-determined timetable, plan, scheme or schedule of chemotherapy administration which may be prepared by a physician or medical practitioner and may be tailored to suit the patient requiring treatment.

The treatment regime may indicate one or more of: the type of chemotherapy to administer to the patient; the dose of each drug or radiation; the time interval between administrations; the length of each treatment; the number and nature of any treatment holidays, if any etc. For a co-therapy a single treatment regime may be provided which indicates how each drug is to be administered.

Chemotherapeutic drugs and biologics may be selected from: alkylating agents such as cisplatin, carboplatin, mechlorethamine, cyclophosphamide, chlorambucil, ifosfamide; purine or pyrimidine anti-metabolites such as azathiopurine or mercaptopurine; alkaloids and terpenoids, such as vinca alkaloids (e.g. vincristine, vinblastine, vinorelbine, vindesine), podophyllotoxin, etoposide, teniposide, taxanes such as paclitaxel (TaxolTM), docetaxel; topoisomerase inhibitors such as the type I topoisomerase inhibitors camptothecins irinotecan and topotecan, or the type II topoisomerase inhibitors amsacrine, etoposide, etoposide phosphate, teniposide; antitumor antibiotics (e.g. anthracyline antibiotics) such as dactinomycin, doxorubicin (AdriamycinTM), epirubicin, bleomycin, rapamycin; antibody based agents, such as anti-PD-1 antibodies, anti-PD-L1 antibodies, anti-TIM-3 antibodies, anti-CTLA-4, anti-4-1 BB, anti-GITR, anti-CD27, anti-BLTA, anti-OX43, anti-VEGF, anti-TNFa, anti-IL-2, antiGpIIb/IIIa, anti-CD-52, anti-CD20, anti-RSV, anti-HER2/neu(erbB2), anti-TNF receptor, anti-EGFR antibodies, monoclonal antibodies or antibody fragments, examples include: cetuximab, panitumumab, infliximab, basiliximab, bevacizumab (Avastin®), abciximab, daclizumab, gemtuzumab, alemtuzumab, rituximab (Mabthera®), palivizumab, trastuzumab, etanercept, adalimumab, nimotuzumab; EGFR inihibitors such as erlotinib, cetuximab and gefitinib; anti-angiogenic agents such as bevacizumab (Avastin®); cancer vaccines such as Sipuleucel-T (Provenge®).

Further chemotherapeutic drugs may be selected from: 13-cis-Retinoic Acid, 2-Chlorodeoxyadenosine, 5-Azacitidine 5-Fluorouracil, 6-Mercaptopurine, 6-Thioguanine, Abraxane, Accutane®, Actinomycin-D Adriamycin®, Adrucil®, Afinitor®, Agrylin®, Ala-Cort®, Aldesleukin, Alemtuzumab, ALIMTA, Alitretinoin, Alkaban-AQ®, Alkeran®, All-transretinoic Acid, Alpha Interferon, Altretamine, Amethopterin, Amifostine, Aminoglutethimide, Anagrelide, Anandron®, Anastrozole, Arabinosylcytosine, Aranesp®, Aredia®, Arimidex®, Aromasin®, Arranon®, Arsenic Trioxide, Asparaginase, ATRA Avastin®, Azacitidine, BCG, BCNU, Bendamustine, Bevacizumab, Bexarotene, BEXXAR®, Bicalutamide, BiCNU, Blenoxane®, Bleomycin, Bortezomib, Busulfan, Busulfex®, Calcium Leucovorin, Campath®, Camptosar®, Camptothecin-11, Capecitabine, Carac™, Carboplatin, Carmustine, Casodex®, CC-5013, CCI-779, CCNU, CDDP, CeeNU, Cerubidine®, Cetuximab, Chlorambucil, Cisplatin, Citrovorum Factor, Cladribine, Cortisone, Cosmegen®, CPT-11, Cyclophosphamide, Cytadren®, Cytarabine Cytosar-U®, Cytoxan®, Dacogen, Dactinomycin, Darbepoetin Alfa, Dasatinib, Daunomycin, Daunorubicin, Daunorubicin Hydrochloride, Daunorubicin Liposomal, DaunoXome®, Decadron, Decitabine, Delta-Cortef®, Deltasone®, Denileukin, Diftitox, DepoCyt™, Dexamethasone, Dexamethasone Acetate, Dexamethasone Sodium Phosphate, Dexasone, Dexrazoxane, DHAD, DIC, Diodex, Docetaxel, Doxil®, Doxorubicin, Doxorubicin Liposomal, Droxia™, DTIC, DTIC-Dome®, Duralone®, Eligard™, Ellence™, Eloxatin™, Elspar®, Emcyt®, Epirubicin, Epoetin Alfa, Erbitux, Erlotinib, Erwinia L-asparaginase, Estramustine, Ethyol Etopophos®, Etoposide, Etoposide Phosphate, Eulexin®, Everolimus, Evista®, Exemestane, Faslodex®, Femara®, Filgrastim, Floxuridine, Fludara®, Fludarabine, Fluoroplex®, Fluorouracil, Fluoxymesterone, Flutamide, Folinic Acid, FUDR®, Fulvestrant, Gefitinib, Gemcitabine, Gemtuzumab ozogamicin, Gleevec™, Gliadel® Wafer, Goserelin, Granulocyte - Colony Stimulating Factor, Granulocyte Macrophage Colony Stimulating Factor, Herceptin ®, Hexadrol, Hexalen®, Hexamethylmelamine, HMM, Hycamtin®, Hydrea®, Hydrocort Acetate®, Hydrocortisone, Hydrocortisone Sodium Phosphate, Hydrocortisone Sodium Succinate, Hydrocortone Phosphate, Hydroxyurea, Ibritumomab, Ibritumomab Tiuxetan, Idamycin®, Idarubicin, Ifex®, IFN-alpha, Ifosfamide, IL-11, IL-2, Imatinib mesylate, Imidazole Carboxamide, Interferon alfa, Interferon Alfa-2b (PEG Conjugate), Interleukin - 2, Interleukin-11, Intron A® (interferon alfa-2b), Iressa®, Irinotecan, Isotretinoin, Ixabepilone, Ixempra™, Kidrolase, Lanacort®, Lapatinib, L-asparaginase, LCR, Lenalidomide, Letrozole, Leucovorin, Leukeran, Leukine™, Leuprolide, Leurocristine, Leustatin™, Liposomal Ara-C, Liquid Pred®, Lomustine, L-PAM, L-Sarcolysin, Lupron®, Lupron Depot®, Matulane®, Maxidex, Mechlorethamine, Mechlorethamine Hydrochloride, Medralone®, Medrol®, Megace®, Megestrol, Megestrol Acetate, Melphalan, Mercaptopurine, Mesna, Mesnex™, Methotrexate, Methotrexate Sodium, Methylprednisolone, Meticorten®, Mitomycin, Mitomycin-C, Mitoxantrone, M-Prednisol®, MTC, MTX, Mustargen®, Mustine, Mutamycin®, Myleran®, Mylocel™, Mylotarg®, Navelbine®, Nelarabine, Neosar®, Neulasta™, Neumega®, Neupogen®, Nexavar®, Nilandron®, Nilutamide, Nipent®, Nitrogen Mustard, Novaldex®, Novantrone®, Octreotide, Octreotide acetate, Oncospar®, Oncovin®, Ontak®, Onxal™, Oprevelkin, Orapred®, Orasone®, Oxaliplatin, Paclitaxel, Paclitaxel Protein-bound, Pamidronate, Panitumumab, Panretin®, Paraplatin®, Pediapred®, PEG Interferon, Pegaspargase, Pegfilgrastim, PEG-INTRON™, PEG-L-asparaginase, PEMETREXED, Pentostatin, Phenylalanine Mustard, Platinol®, Platinol-AQ®, Prednisolone, Prednisone, Prelone®, Procarbazine, PROCRIT®, Proleukin®, Prolifeprospan 20 with Carmustine Implant Purinethol®, Raloxifene, Revlimid®, Rheumatrex®, Rituxan®, Rituximab, Roferon-A® (Interferon Alfa-2a), Rubex®, Rubidomycin hydrochloride, Sandostatin® Sandostatin LAR®, Sargramostim, Solu-Cortef®, Solu-Medrol®, Sorafenib, SPRYCEL™, STI-571, Streptozocin, SU11248, Sunitinib, Sutent®, Tamoxifen, Tarceva®, Targretin®, Taxol®, Taxotere®, Temodar®, Temozolomide, Temsirolimus, Teniposide, TESPA, Thalidomide, Thalomid®, TheraCys®, Thioguanine, Thioguanine Tabloid®, Thiophosphoamide, Thioplex®, Thiotepa, TICE®, Toposar®, Topotecan, Toremifene, Torisel®, Tositumomab, Trastuzumab, Treanda®, Tretinoin, Trexall™, Trisenox®, TSPA, TYKERB®, VCR, Vectibix™, Velban®, Velcade®, VePesid®, Vesanoid®, Viadur™, Vidaza®, Vinblastine, Vinblastine Sulfate, Vincasar Pfs®, Vincristine, Vinorelbine, Vinorelbine tartrate, VLB, VM-26, Vorinostat, VP-16, Vumon®, Xeloda®, Zanosar®, Zevalin™, Zinecard®, Zoladex®, Zoledronic acid, Zolinza, Zometa®.

Multiple doses of the antibody/fragment, CAR, nucleic acid, vector, cell or composition may be provided. One or more, or each, of the doses may be accompanied by simultaneous or sequential administration of another therapeutic agent.

Multiple doses may be separated by a predetermined time interval, which may be selected to be one of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31 days, or 1, 2, 3, 4, 5, or 6 months. By way of example, doses may be given once every 7, 14, 21 or 28 days (plus or minus 3, 2, or 1 days).

### Diagnostic applications

Antibodies and antigen binding fragments described herein may be used in methods that involve the binding of the antibody or antigen binding fragment to target antigen. Such methods include diagnosing a disease or condition in a subject. Such methods may involve detection of the bound complex of antibody, or antigen binding fragment, and target. As such, in one embodiment a method is provided, the method comprising contacting a sample containing, or suspected to contain, peptide-MHC complex (e.g. of a peptide of p53 in complex with MHC class I molecule, such as p53₁₂₅₋₁₃₄ in complex with MHC class I comprising an α-chain encoded by an HLA-A*24 allele) with an antibody or antigen binding fragment as described herein, and detecting the formation of a complex of antibody, or antigen binding fragment, and the peptide-MHC complex.

Suitable method formats are well known in the art, including immunoassays such as sandwich assays, e.g. ELISA. The method may involve labelling the antibody/antigen binding fragment or the peptide-MHC complex, or both, with a detectable label, e.g. fluorescent, luminescent or radio-label. Expression of the peptide-MHC complex may be measured by immunohistochemistry (IHC), for example of a tissue sample obtained by biopsy. In some embodiments, the label may be selected from: a radio-nucleotide, positron-emitting radionuclide (e.g. for positron emission tomography (PET)), MRI contrast agent or fluorescent label.

Analysis of peptide-MHC complex may be *in vitro* or *in vivo,* and may involve analysis e.g. by fluorescence imaging, positron emission tomography (PET), or magnetic resonance imaging (MRI), by detection of appropriately labelled species.

Methods of this kind may provide the basis of a method of diagnosis of a disease or condition requiring detection and/or quantitation of the peptide-MHC complex. Such methods may be performed *in vitro* on a subject sample, or following processing of a subject sample. Once the sample is collected, the subject is not required to be present for the *in vitro* method of diagnosis to be performed and therefore the method may be one which is not practised on the human or animal body. The present invention provides the use of antibody or antigen binding fragment described herein for the detection of peptide-MHC complex *in vitro.*

The term *"in vitro"* is intended to encompass experiments with cells in culture whereas the term *"in vivo"* is intended to encompass experiments with and/or treatment of intact multicellular organisms.

Such methods may also be performed *in vivo,* e.g. following administration to a subject of an antibody, antigen binding fragment, CAR, nucleic acid, vector, cell or composition according to the present invention.

Such methods may involve detecting the presence of, and/or determining the amount of, the peptide-MHC complex present in a subject or subject sample. The method may further comprise comparing the determined amount against a standard or reference value as part of the process of reaching a diagnosis. Other diagnostic tests may be used in conjunction with those described here to enhance the accuracy of the diagnosis or prognosis or to confirm a result obtained by using the tests described here.

In some cases, *in vitro* or *in vivo* methods for the detection of the presence of the peptide-MHC complex may involve boosting MHC class I expression, for example by interferon treatment or HDAC inhibitor treatment.

The presence of the peptide-MHC complex or the level of the peptide-MHC complex present in a subject sample may be indicative that a subject has a disease/condition, e.g. a disease/or condition described herein. Detection (e.g. in a sample obtained from a subject) of the peptide-MHC complex may be used for the purpose of diagnosis of a cancerous condition in the subject, diagnosis of a predisposition to a cancerous condition or for providing a prognosis (prognosticating) of a cancerous condition. The diagnosis or prognosis may relate to an existing (previously diagnosed) cancerous condition.

The presence of the peptide-MHC complex or the level of the peptide-MHC complex present in a subject sample may be indicative that a subject may respond to treatment with an antibody, antigen binding fragment, CAR, nucleic acid, vector, cell or composition according to the present invention. Detection of the presence of the peptide-MHC complex, or detection of a particular level of the peptide-MHC complex, may be used to select a subject for treatment with antibody, antigen binding fragment, CAR, nucleic acid, vector, cell or composition according to the present invention. Detection of the presence of the peptide-MHC complex, or detection of a particular level of the peptide-MHC complex, may be used to select a subject for treatment with another agent for the treatment of a disease/condition.

A sample may be taken from any tissue or bodily fluid. The sample may comprise or may be derived from: a quantity of blood; a quantity of serum derived from the individual's blood which may comprise the fluid portion of the blood obtained after removal of the fibrin clot and blood cells; a tissue sample or biopsy; pleural fluid; cerebrospinal fluid (CSF); or cells isolated from said individual. In some embodiments, the sample may be obtained or derived from a tissue or tissues which are affected by the disease/disorder (e.g. tissue or tissues in which symptoms of the disease manifest, or which are involved in the pathogenesis of the disease/disorder). In some embodiments the sample may be obtained or derived from a cancerous cell or tumour biopsy.

### Protein Expression

Molecular biology techniques suitable for producing the proteins (e.g. the antibodies, antigen binding fragments and CARs) according to the invention in cells are well known in the art, such as those set out in Sambrook et al., Molecular Cloning: A Laboratory Manual, New York: Cold Spring Harbor Press, 1989. Polypeptides may be expressed from a nucleic acid sequence. The nucleic acid sequence may be contained in a vector present in a cell, or may be incorporated into the genome of the cell.

Any cell suitable for the expression of polypeptides may be used for producing proteins according to the invention. The cell may be a prokaryote or eukaryote. Suitable prokaryotic cells include *E.coli.* Examples of eukaryotic cells include a yeast cell, a plant cell, insect cell or a mammalian cell (e.g. Chinese Hamster Ovary (CHO) cells). In some cases the cell is not a prokaryotic cell because some prokaryotic cells do not allow for the same post-translational modifications as eukaryotes. In addition, very high expression levels are possible in eukaryotes and proteins can be easier to purify from eukaryotes using appropriate tags. Specific plasmids may also be utilised which enhance secretion of the protein into the media.

Methods of producing a polypeptide of interest may involve culture or fermentation of a cell modified to express the polypeptide. The culture or fermentation may be performed in a bioreactor provided with an appropriate supply of nutrients, air/oxygen and/or growth factors. Secreted proteins can be collected by partitioning culture media/fermentation broth from the cells, extracting the protein content, and separating individual proteins to isolate secreted polypeptide. Culture, fermentation and separation techniques are well known to those of skill in the art.

Bioreactors include one or more vessels in which cells may be cultured. Culture in the bioreactor may occur continuously, with a continuous flow of reactants into, and a continuous flow of cultured cells from, the reactor. Alternatively, the culture may occur in batches. The bioreactor monitors and controls environmental conditions such as pH, oxygen, flow rates into and out of, and agitation within the vessel such that optimum conditions are provided for the cells being cultured.

Following culture of cells that express the polypeptide of interest, that polypeptide is preferably isolated. Any suitable method for separating polypeptides from cell culture known in the art may be used. In order to isolate a polypeptide of interest from a culture, it may be necessary to first separate the cultured cells from media containing the polypeptide of interest. If the polypeptide of interest is secreted from the cells, the cells may be separated from the culture media that contains the secreted polypeptide by centrifugation. If the polypeptide of interest collects within the cell, it will be necessary to disrupt the cells prior to centrifugation, for example using sonification, rapid freeze-thaw or osmotic lysis. Centrifugation will produce a pellet containing the cultured cells, or cell debris of the cultured cells, and a supernatant containing culture medium and the polypeptide of interest.

It may then be desirable to isolate the polypeptide of interest from the supernatant or culture medium, which may contain other protein and non-protein components. A common approach to separating polypeptide components from a supernatant or culture medium is by precipitation. Polypeptides/proteins of different solubility are precipitated at different concentrations of precipitating agent such as ammonium sulfate. For example, at low concentrations of precipitating agent, water soluble proteins are extracted. Thus, by adding increasing concentrations of precipitating agent, proteins of different solubility may be distinguished. Dialysis may be subsequently used to remove ammonium sulfate from the separated proteins.

Other methods for distinguishing different polypeptides/proteins are known in the art, for example ion exchange chromatography and size chromatography. These may be used as an alternative to precipitation, or may be performed subsequently to precipitation.

Once the polypeptide of interest has been isolated from culture it may be necessary to concentrate the protein. A number of methods for concentrating a protein of interest are known in the art, such as ultrafiltration or lyophilisation.

### Kits

In some aspects of the present invention a kit of parts is provided. In some embodiments the kit may have at least one container having a predetermined quantity of an antibody, antigen binding fragment, CAR, composition, nucleic acid, vector or cell according to the present invention.

The kit may provide the antibody, antigen binding fragment, CAR, composition, nucleic acid, vector or cell together with instructions for administration to a patient in order to treat a specified disease/condition, e.g. a cancer). The antibody, antigen binding fragment, CAR, composition, nucleic acid, vector or cell may be formulated so as to be suitable for injection or infusion to a tumor or to the blood.

In some embodiments the kit may comprise materials for producing a cell according to the present invention. For example, the kit may comprise materials for modifying a cell to express or comprise an antibody, antigen binding fragment, CAR, nucleic acid or vector according to the present invention, or materials for introducing into a cell the nucleic acid or vector according to the present invention.

In some embodiments the kit may further comprise at least one container having a predetermined quantity of another therapeutic agent (e.g. anti-infective agent or chemotherapy agent). In such embodiments, the kit may also comprise a second medicament or pharmaceutical composition such that the two medicaments or pharmaceutical compositions may be administered simultaneously or separately such that they provide a combined treatment for the specific disease or condition. The therapeutic agent may also be formulated so as to be suitable for injection or infusion to a tumor or to the blood.

### Sequence Identity

Pairwise and multiple sequence alignment for the purposes of determining percent identity between two or more amino acid or nucleic acid sequences can be achieved in various ways known to a person of skill in the art, for instance, using publicly available computer software such as ClustalOmega (Söding, J. 2005, Bioinformatics 21, 951-960), T-coffee (Notredame et al. 2000, J. Mol. Biol. (2000) 302, 205-217), Kalign (Lassmann and Sonnhammer 2005, BMC Bioinformatics, 6(298)) and MAFFT (Katoh and Standley 2013, Molecular Biology and Evolution, 30(4) 772-780 software. When using such software, the default parameters, e.g. for gap penalty and extension penalty, are preferably used.

The invention includes the combination of the aspects and preferred features described except where such a combination is clearly impermissible or expressly avoided.

The section headings used herein are for organizational purposes only and are not to be construed as limiting the subject matter described.

Aspects and embodiments of the present invention will now be illustrated, by way of example, with reference to the accompanying figures. Further aspects and embodiments will be apparent to those skilled in the art. All documents mentioned in this text are incorporated herein by reference.

Throughout this specification, including the claims which follow, unless the context requires otherwise, the word "comprise," and variations such as "comprises" and "comprising," will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment.

### Brief Description of the Figures

Embodiments and experiments illustrating the principles of the invention will now be discussed with reference to the accompanying figures.
**Figure 1****.** Light chain variable domain sequences for anti-p53-A24 antibody clones. CDRs are underlined and shown separately.
**Figure 2****.** Heavy chain variable domain sequences for anti-p53-A24 antibody clones. CDRs are underlined and shown separately.
**Figure 3****.** Table showing light chain CDR sequences for anti-p53-A24 antibody clones.
**Figure 4****.** Table showing heavy chain CDR sequences for anti-p53-A24 antibody clones.

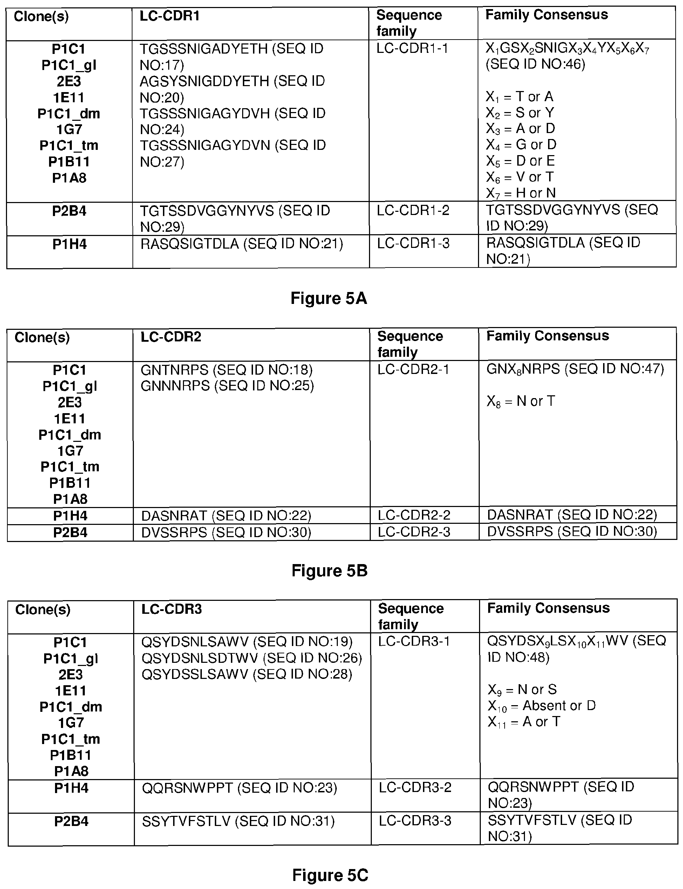

**Figure 5****.** Tables showing light chain CDR sequences for anti-p53-A24 antibody clones and consensus sequences, for (A) LC-CDR1, (B) LC-CDR2 and (C) LC-CDR3.

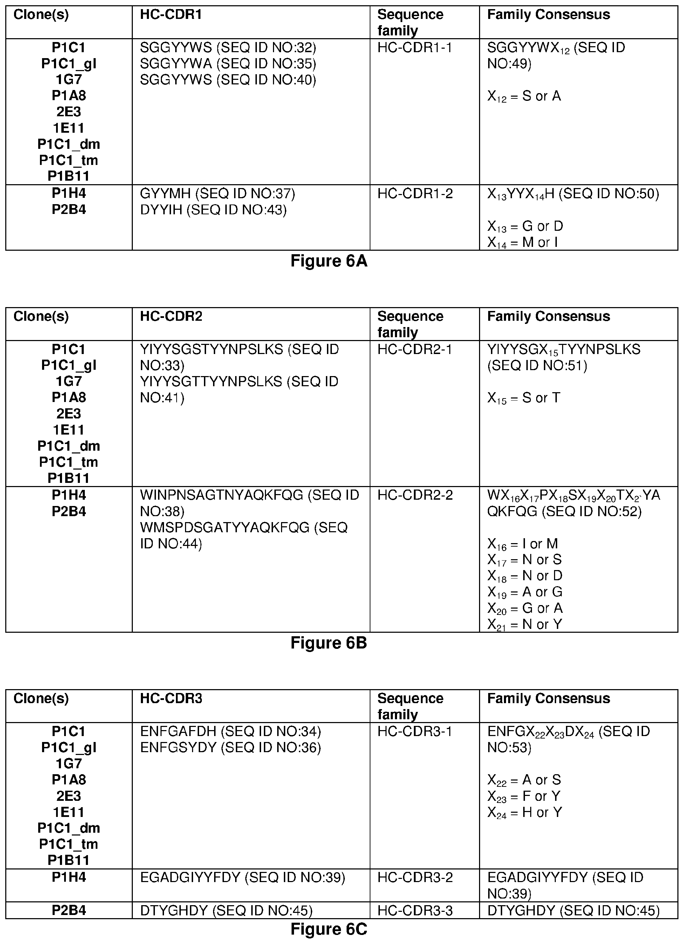

**Figure 6****.** Tables showing heavy chain CDR sequences for anti-p53-A24 antibody clones and consensus sequences, for (A) HC-CDR1, (B) HC-CDR2 and (C) HC-CDR3.

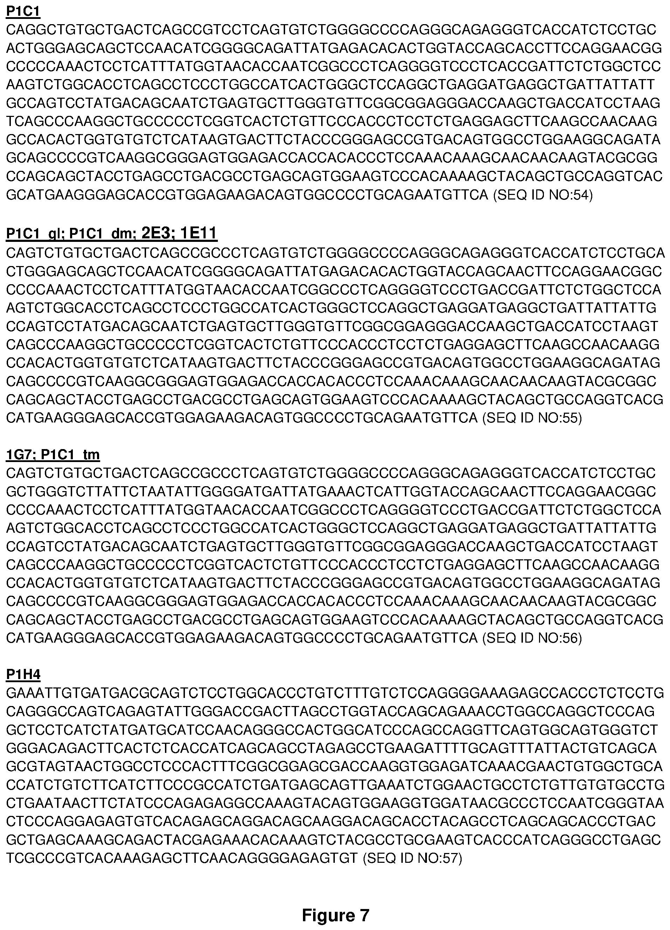

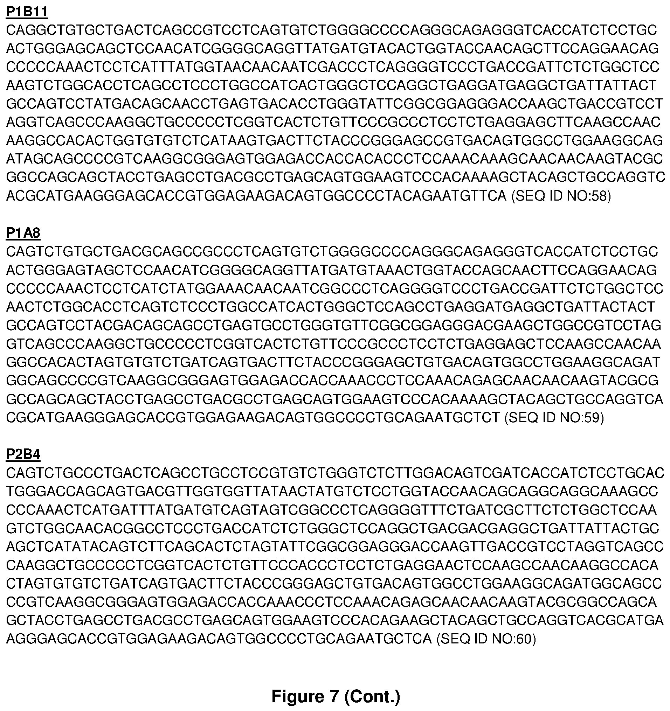

**Figure 7****.** Nucleotide sequences encoding VL regions for the anti-p53-A24 antibody clones.

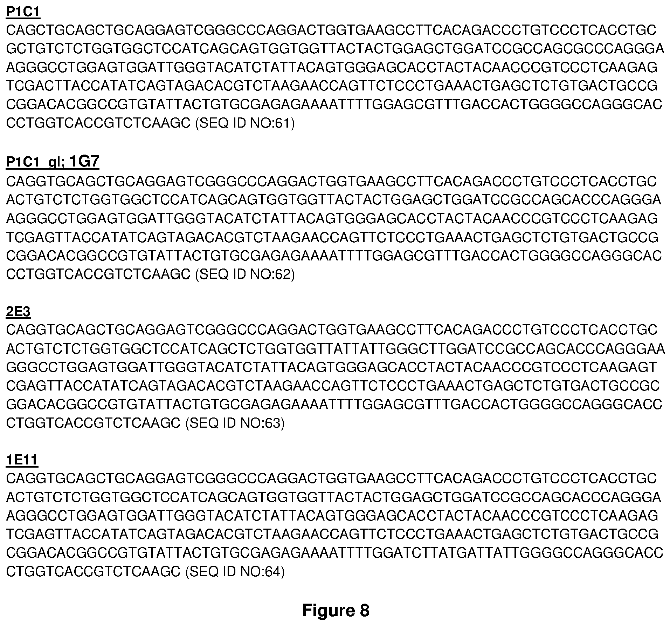

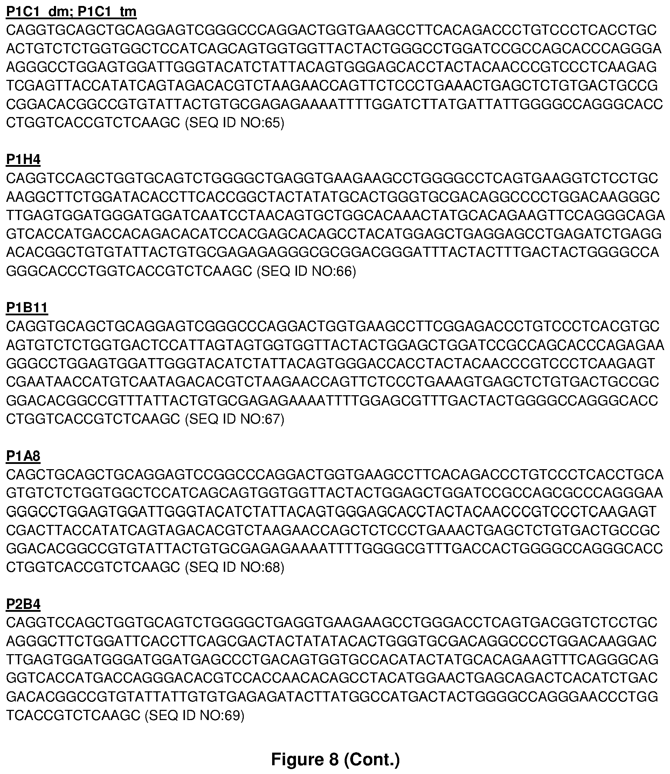

**Figure 8****.** Nucleotide sequences encoding VH regions for the anti-p53-A24 antibody clones.
**Figure 9****.** Graphs showing the results of ELISA analysis of binding of anti-p53-A24 antibody clones to (**A**) p53125-134-A24 monomers, and (**B**) negative control antigen.
**Figure 10****.** Graphs showing binding of (**A**) P1C1, (**B**) P1H4 and (**C**) P2B4 to HLA*A24-expressing HT29 cells, unpulsed (1), pulsed with irrelevant peptides hTERT324-332, hTERT₄₆₁₋₄₆₉, WT1 2₃₅₋₂₄₃, WT1₄₁₇₋₄₂₅ or p53₂₀₄₋₂₁₂ (2 to 6), or pulsed with p53₁₂₅₋₁₃₄ (7).
**Figure 11****.** Graphs showing binding of P1C1 to (**A**) wild-type MDA-MB-231 cells (left panels) or HLA*A24-transduced MDA-MB-231 cells (right panels), either pulsed with p53 peptide (top panels) or unpulsed (bottom panels), and (**B**) HLA*A24+ SoaS2 cells, before or after pulsing with various hTERT, WT1 or p53 peptides. MFI histograms are shown.
**Figure 12****.** Table showing binding affinity of antibody P1C1-derived antibody clones to p53-A24.
**Figure 13****.** Graphs showing binding of P1C1-derived clones, the germline version (P1C1_gl) and 2 affinity-matured clones (P1C1_dm and P1C1_tm) to unpulsed HT29 cells. MFI histograms are shown; the narrow, sharp peak to the left represents the negative control (secondary antibody only) and the broader peak to the right represents the test clone.
**Figure 14****.** Bar charts showing specific killing of HLA*A24 positive cells in an ADCC assay in the presence of P1C1_gl or P1C1_tm. Mean cytotoxicity in triplicate ±SD is shown.
**Figure 15****.** Graphs and bar chart showing internalisation of the antibody complexed to p53-A24 by HT29 pulsed cells. (**A**) MFI curves of HT29 cells incubated with pH-sensitive dye-labelled P1C1_tm (left panels) or a labelled isotype control antibody (right panels) on ice (top panels) or at 37°C (bottom panels). (**B**) MFI values of cells for cells incubated with pH-sensitive dye-labelled P1C1_tm or a labelled isotype control antibody at different time points.
**Figure 16****.** Bar chart showing increased cytotoxic effect of PNU and PBD on HT29 cells in the absence (PNU/PBD 2dary only) or presence (PNU/PBD) of P1C1_tm antibody. Cells were incubated with P1C1_tm and PNU or PBD cytotoxic drugs conjugated to anti-Fc antibodies. Three concentrations of antibodies were tested, for each of them the ratio P1C1_tm/anti-Fc antibody was 1:1. Shown is the proportion of surviving cells after 72 hours of incubation compared to the number of cells in wells left untreated.

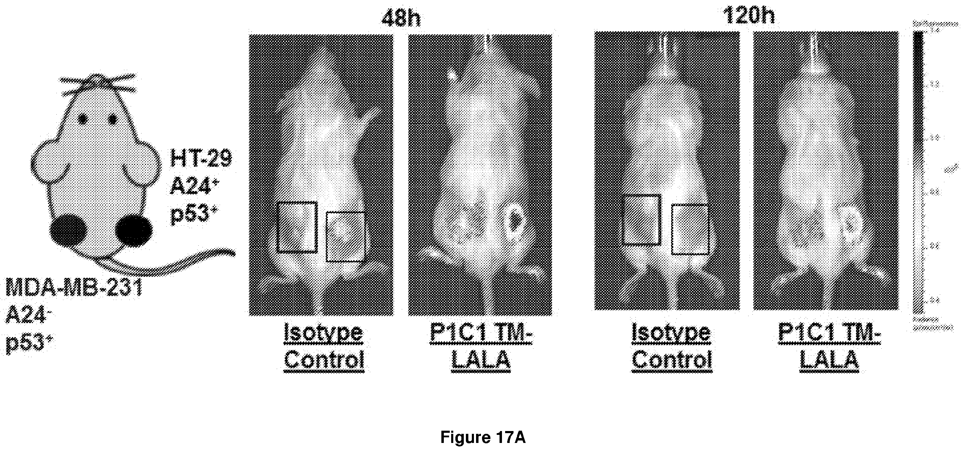

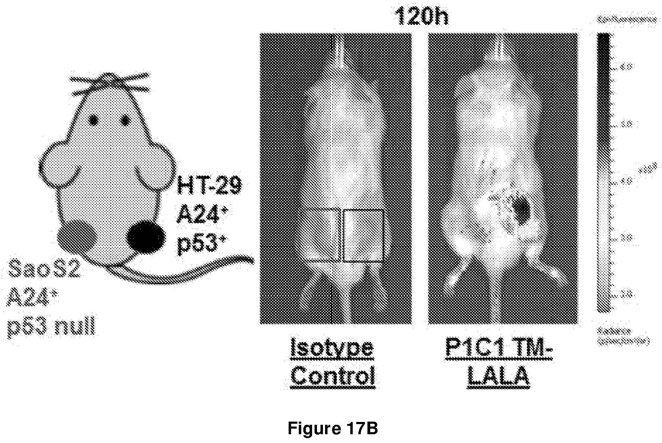

**Figure 17****.** Photographs showing *in vivo* specificity of P1C1_tm antibody. The antibody was used to track human tumour cell lines injected in NSG mice. (**A**) HT29 cells expressing p53 and HLA*A24 were seeded in the right flank of the animals, and HLA*A24-/p53+ control cells were seeded in the left flank. (**B**) HT29 cells expressing p53 and HLA*A24 were seeded in the right flank of the animals, and HLA*A24+/p53- control cells were seeded in the left flank. Representative images from *in vivo* imaging of established tumours at the indicated number of hours after injection of fluorescently-labelled P1C1_tm antibody are shown.
**Figure 18****.** Graph showing % cytolysis of HT29 tumour cells by p53 CAR T cells, control T cells, or no T cells. % cytolysis was measured over 40 hours using an impedance-based T cell-mediated cytotoxicity assay (xCELLigence).
**Figure 19****.** Anti-p53-A24/CD3 bispecific antibodies were evaluated for their ability to induce tumour cell killing via human primary T cells. (A) Two different formats of bispecific antibody were tested: BsAb1 and BsAb2. (**B**) The target-specific cytotoxicity of BsAb1 and BsAb2 was tested against HLA-A24+/p53 mutant+ cell line HT29 (BsAb1: right hand curve, BsAb2: left hand curve) and HLA-A24+/p53null cell line SaOS2.

### Examples

In the following Examples, the inventors describe isolation and characterisation of antibodies capable of binding to p53 peptide:MHC class I complex.

### Example 1: Isolation of anti-p53:MHC class I complex antibodies

The p53₁₂₅₋₁₃₄ peptide was attached to soluble HLA*A2402 (p53-A24) to form a soluble peptide MHC complex (pMHC). Antibodies capable of binding to this pMHC complex were then isolated from a human antibody phage display library via *in vitro* selection. Out of 190 clones screened, the 36 clones showing the highest binding to the pMHC by ELISA assay were isolated, and 4 amongst them were cloned into IgG format for further characterisation: P1C1, P1H4, P1A8 and P1B11.

The amino acid sequences for the light chain and heavy chain variable regions are shown in Figures 1 and 2, respectively.

### Example 2: Avidity and specificity for p53-A24

Antibody clones P1C1, P1H4, P1A8 and P1B11 were analysed by ELISA assay for binding to p53-A24 monomers, and for binding to irrelevant antigen, to determine avidity and specificity.

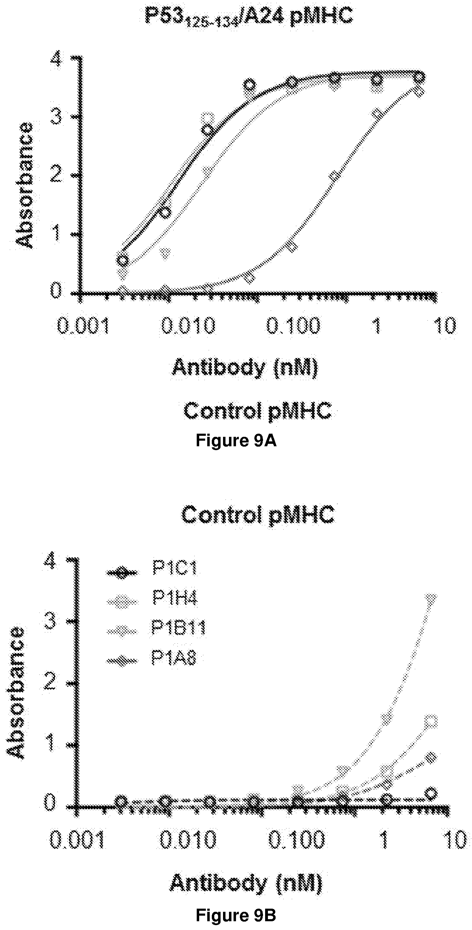

With the exception of P1A8, all clones were shown to bind to p53-A24 with high affinity (Figure 9A). The antibodies also displayed zero or limited non-specific binding (Figure 9B).

### Example 3: Specific binding to p53 on HLA*A24-expressing cells

The ability to recognise and bind p53-A24 pMHC expressed at the cell surface was measured using HT29 cells. These cells constitutively express HLA*A24.

Briefly, HT29 cells were pulsed with the p53₁₂₅₋₁₃₄ peptide, peptide selected from a panel of irrelevant peptides, or were unpulsed for 1 hour at room temperature. Cells were then incubated with P1C1, P1H4 or P2B4, and binding was measured by flow cytometry using a secondary labelled antibody.

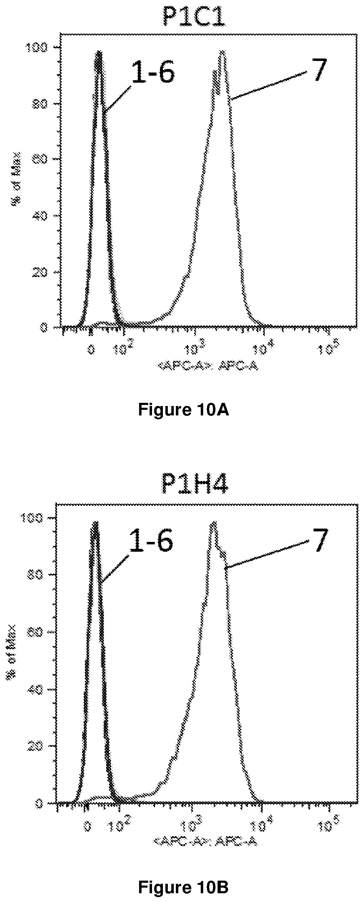

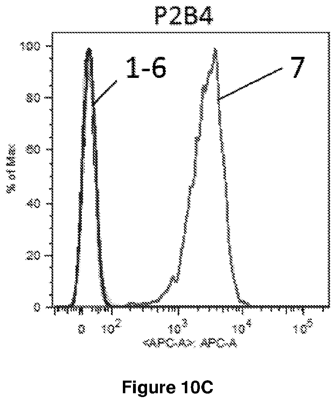

The results are shown in Figures 10A to 10C. P1C1, P1H4 or P2B4 antibodies only bound to cells that had been pulsed with the p53₁₂₅₋₁₃₄ peptide, demonstrating their specificity for the p53₁₂₅₋₁₃₄ antigen (Figures 10A to 10C). Moreover, the fact that the antibodies did not bind to unpulsed HT29 cells suggests they only bind to HLA*A24 presenting p53 antigen, and does not bind to HLA*A24 not presenting p53.

### Example 4: Specificity for HLA*A24 MHC Class I molecule and for p53-A24

To confirm the specificity for the HLA*A24 haplotype, binding was assessed on cells expressing a different HLA*A type: MDA-MB-231 cells, which constitutively express HLA*A02 and p53. MDA-MB-231 cells were also transduced to express HLA*A24.

Binding of P1C1 was then assessed on transduced and nontransduced MDA-MB-231 cells, which were either pulsed with the p53₁₂₅₋₁₃₄ peptide or unpulsed. Binding was measured by flow cytometry using a secondary labelled antibody.

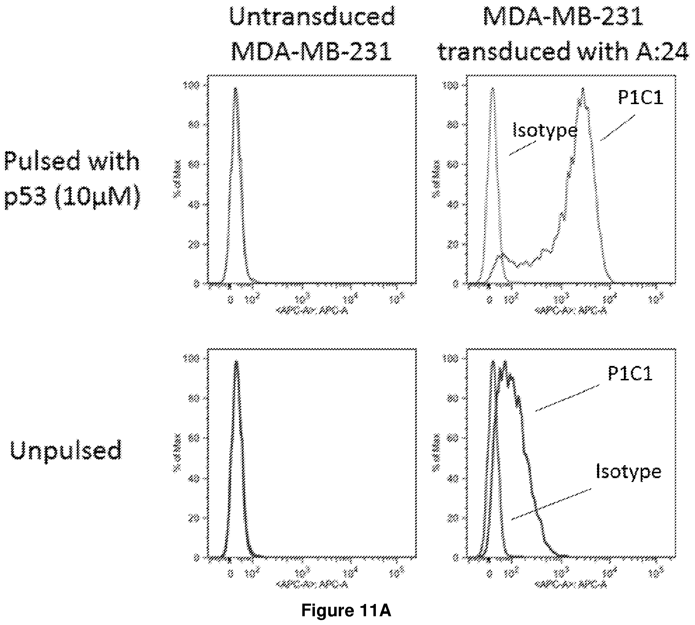

The results are shown in Figure 11A. P1C1 bound only to cells expressing HLA*A24 (i.e. transduced MDA-MB-231), and no binding was observed on nontransduced MDA-MB-231 cells expressing HLA*A02, confirming the specificity for p53₁₂₅₋₁₃₄ peptide presented by HLA*A24.

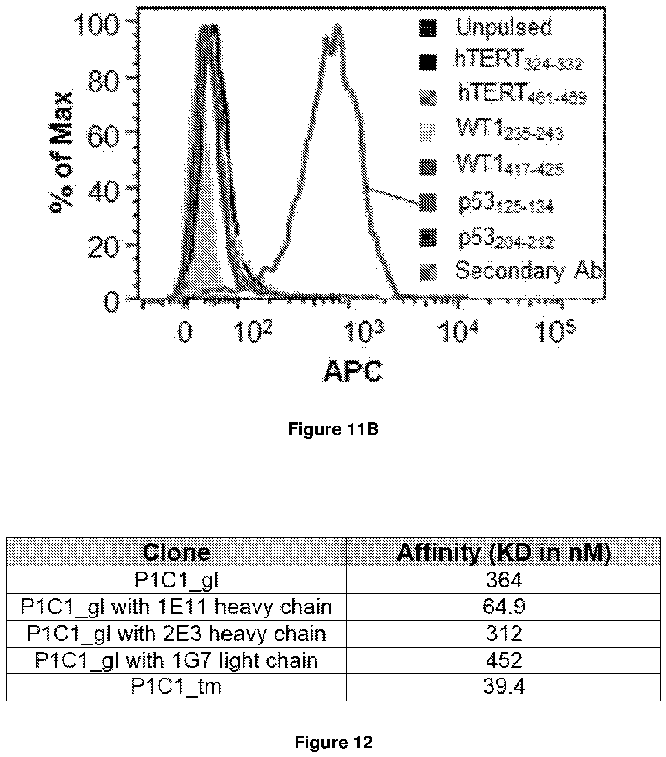

Similar experiments were conducted using SaoS2 cells which are p53-negative. These cells constitutively express HLA*A24. The cells were pulsed with various peptides of p53, WT1, hTERT or unpulsed. Analysis of binding of P1C1 antibody to the cells by bound exclusively cells that were pulsed with p53₁₂₅₋₁₃₄, confirming the specificity of the antibody for this antigen (Figure 11B).

Taken together, these results demonstrate the specificity of the antibody for p53₁₂₅₋₁₃₄ presented by HLA*A24.

### Example 5: Affinity Matured Antibodies

The P1C1 sequence was reverted to a germline framework to give clone P1C1_gl, which subsequently underwent affinity maturation. 2 affinity matured clones were retained for the heavy chain (2E3 and 1E11), and 1 affinity matured clone was retained for the light chain (1G7).

A double-mutant comprising the substitutions present in both 2E3 and 1E11 was constructed, designated clone P1C1_dm. A triple-mutant comprising the substitutions present in 2E3, 1E11 and 1G7 was generated, designated clone P1C1_tm.

The affinity of different P1C1-derived clones for p53-A24 complex was measured via Surface Plasmon Resonance analysis. The results are shown in Figure 12, and show that P1C1_tm has ∼10-fold higher affinity for p53-A24 as compared to P1C1_gl.

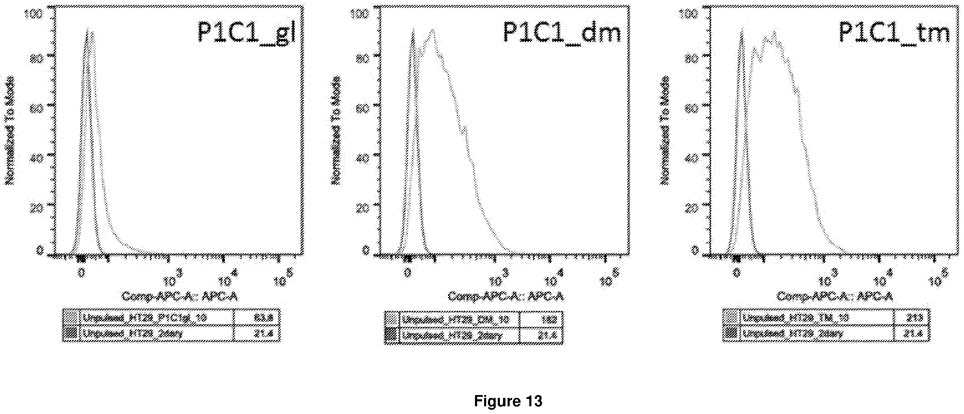

The ability of P1C1_dm, P1C1_tm and P1C1-gl to bind to unpulsed HT29 cells was analysed by flow cytometry. P1C1_dm and P1C1_tm clones showed a higher binding to unpulsed HT29 cells than the original germline clone (Figure 13).

### Example 6: In vitro activity: induction of Antibody-Dependent Cell-mediated Cytotoxicity (ADCC)

To assess the ability of the antibodies to induce ADCC, HLA*A24 positive HT29 were mixed with PBMCs at a ratio of 1:10, and incubated overnight in the presence or absence of P1C1_gl or P1C1_tm, and cell killing was measured. Cells were either unpulsed, or pulsed with the p53 peptide.

The specificity of induction of ADCC by the antibodies was also assessed using HLA*A24-negative MDA-MB-231 cells, nontransduced or transduced with HLA*A24.

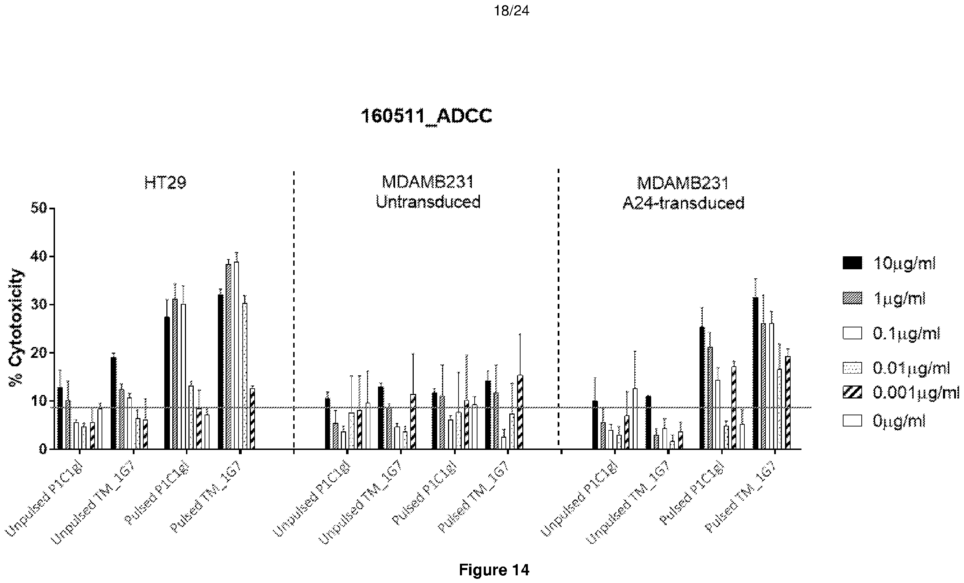

The results are shown in Figure 14. Dose-dependent killing was observed to be more efficient in p53 peptide pulsed HLA*A24-positive cells as compared to unpulsed cells. No significant ADCC was observed in HLA*A24-negative MDA-MB-231 cells. P1C1_tm exhibits more potent ADCC as compared to P1C1_gl.

### Example 7: Internalisation of P1C1 tm bv HT29 cells

P1C1_tm antibody was labelled with pH-sensitive pHrodo-Red dye and incubated with p53 peptide-pulsed HT29 cells at 37°C or on ice for various periods of time, before internalisation was analysed by flow cytometry. Internalised antibodies produce significant fluorescence as compared to surface bound antibodies due to the acidic environment in the endosomes.

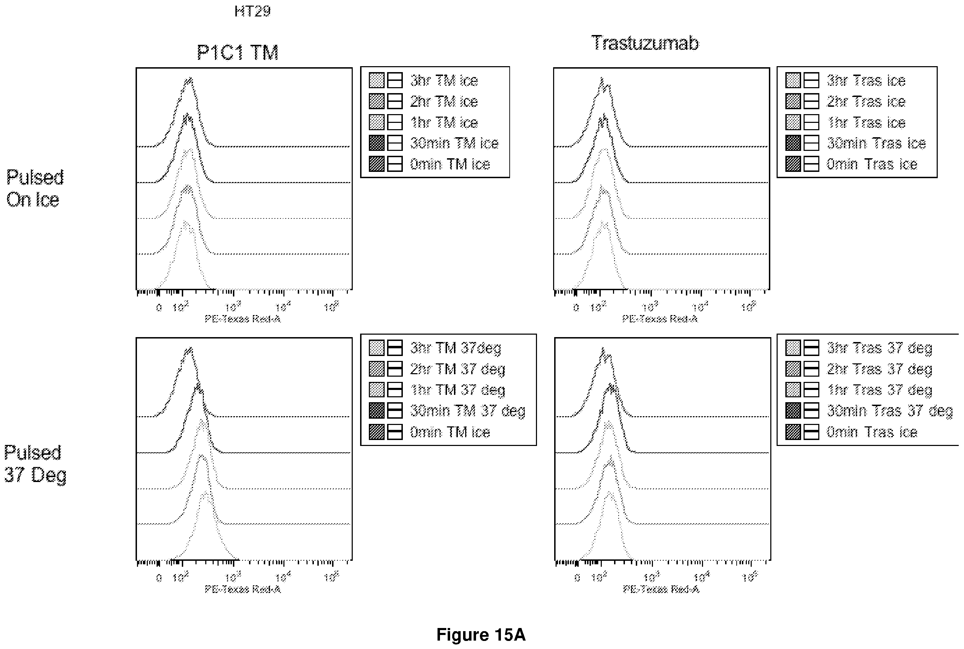

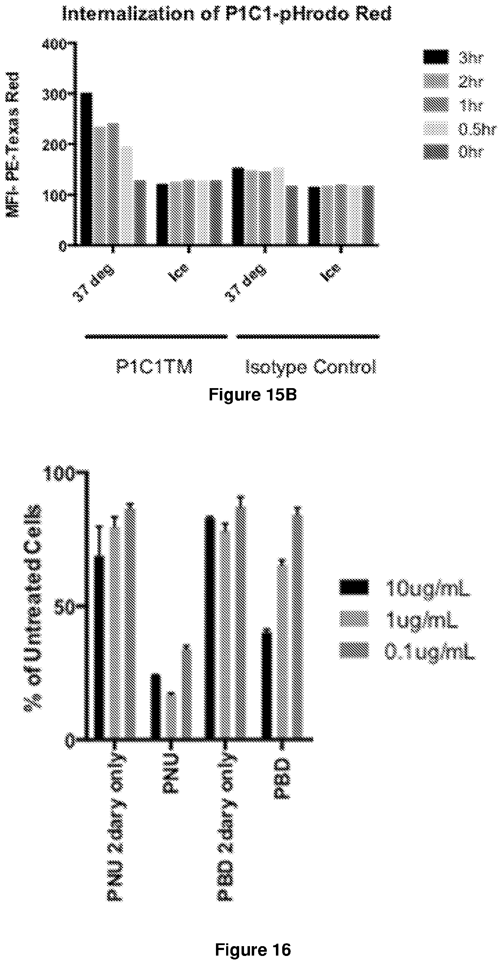

The results are shown in Figures 15A and 15B. Upon binding to p53-A24 pMHC, the antibody is internalised with the complex when the complex is recycled, as evidenced by fluorescence shift in HT29 cells pulsed with p53 peptide incubated at 37°C in the presence of P1C1_tm antibody (Figure 15A, bottom left panel). This shift was not observed with a non-specific control antibody (Figure 15A, bottom right panel), or in cells incubated on ice and hence not recycling the p53-A24 pMHC (Figure 15A, top left panel).

### Example 8: Drug delivery to tumour cells

Internalisation of the antibody with the p53-A24 complex may be used as a tool for drug delivery to specifically target tumour cells. This was tested using drug-conjugated secondary antibodies binding to P1C1_tm. Briefly, HT29 cells were incubated with P1C1_tm and anti-human Fc specific secondary antibodies conjugated with cytotoxic drugs PNU159682 (PNU) or pyrrolobenzodiazepine (PBD). After 72 hours, cell viability was analysed by MTT assay. Some cells were incubated only with the drug-conjugated antibodies, in the absence of P1C1_tm, as controls.

The results of the experiments are shown in Figure 16. P1C1_tm was found to dramatically improve the cytotoxic effect of PNU and PBD. The increased effect is likely due to the internalisation of the drugs as part of the immune complex of P1C1_tm/drug-conjugated anti-Fc antibody bound to the p53-A24 complex.

These data suggest that P1C1_tm could be used in the treatment of cancer for targeted drug delivery of drugs to tumours, and to increase the efficacy of drugs by forcing their internalisation into the targeted cells.

### Example 9: In vivo imaging of HT29 in NSG mice

One of the applications of the TCR like antibodies will be to help diagnose cancer by identifying tumour cells which cross-present intracellular molecules via MHC class I, in the present case p53.

To assess the usefulness of P1C1_tm as a diagnostic antibody, HT29 cells (positive for both A24 and p53), and nontransduced MDA-MB-231 cells (expressing p53 but negative for A24) or SaoS2 cells (expressing A24 but not p53) were implanted into flanks of NSG mice, as shown schematically in Figures 17A and 17B and explained in the Figure legend.

Tumours were allowed to establish and grow up to 100-200 mm³ before 50µg of AF680-labelled P1C1_tm antibody was administered intravenously. Tumour labelling was captured 48 and 120 hours later by *in vivo* fluorescent imaging.

As shown in Figures 17A and 17B, the antibody allowed the detection of the HLA*A24+ HT29 cells but did not track the HLA*A02 expressing tumour (Figure 9A), nor the HLA*A24+ tumour cells not expressing p53 (Figure 9B)

### Example 10: T cell-mediated cytotoxicity against HT29 tumour cells

T cells expressing a chimeric antigen receptor (CAR) comprising a TCR like antigen binding fragment were assessed for their ability to kill HT29 tumour cells.

T cells from healthy donors were activated by addition of TransAct™ (CD3/CD28 agonists) and maintained at a density of 1-2×10⁶ cells/ml in TexMACS™ medium supplemented with 50 ng/mL IL2 for a duration of 72 hr. Upon stimulation, cells were electroporated with or without lentiviral-based p53 CAR plasmid (2 µg per million cells). Due to a considerable amount of cell death caused by plasmid DNA electroporation, apoptotic cells (Annexin V+) were depleted from cell culture by immunomagnetic negative selection at 48 hr post-electroporation.

Cells were rested in culture for additional 24 hr before being assessed in a T cell-mediated cytotoxicity assay (xCELLigence). In this assay, cell index values of tumour cells are measured. Cell index is determined by the impedance of current across the transistor plate caused by tumour cell adherence.

Using the xCELLigence impedance-based system, continuous tumour cell killing was evaluated over 40 hours. HT29 tumour cells were plated in a 96-well, resistor-bottomed plate at 15,000 cells per well in complete growth media. After 18-24 hours, 3,750 effector T cells (1:4 seeding ratio) were added, at which point cell index values correlating to HT29 adherence were normalized. Impedance-based measurements of the normalized cell index were recorded every 10 minutes and converted into % cytolysis. Data present the mean (± standard deviation) of triplicates.

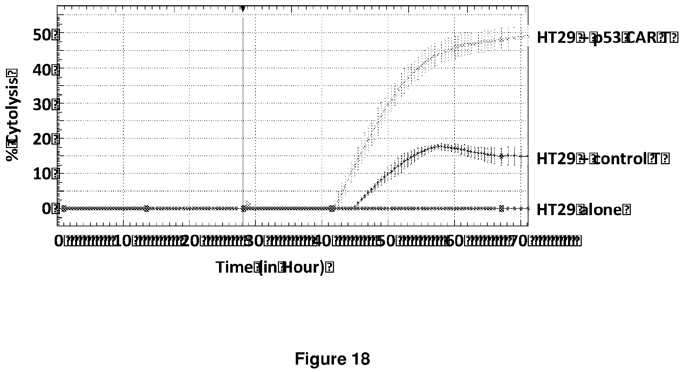

The results are shown in Figure 18. The p53 CAR T cells were able to kill significantly more of the HLA*A24 positive HT29 tumour cells compared to control T cells.

### Example 11: Anti-p53-A24/CD3 bispecific antibody cytotoxicity in vitro against HT29 tumour cells

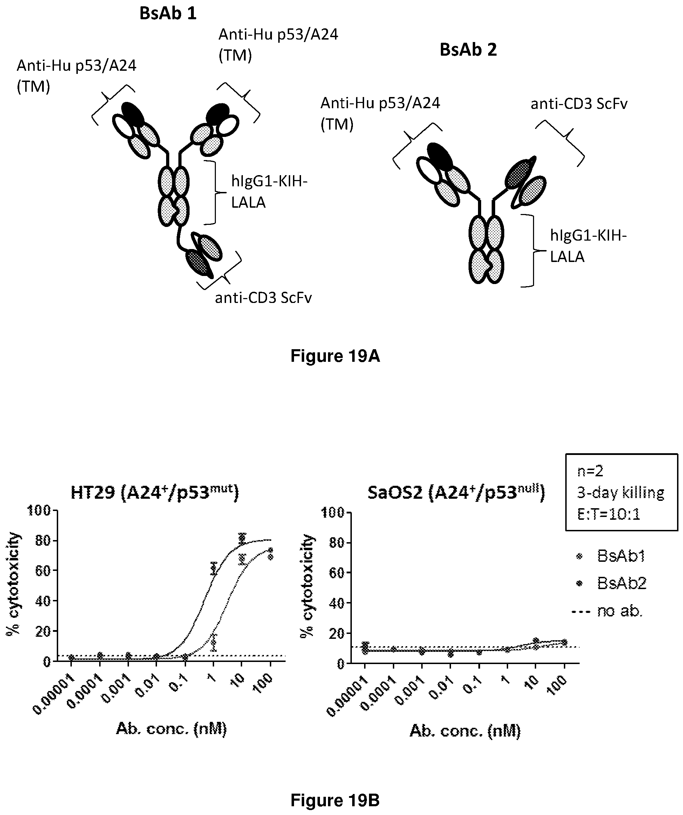

Two different formats of anti-p53-A24/CD3 bispecific antibody (BsAb) cocultured with human primary T cells and tumour cells and assessed for their ability to induce tumour cell killing. The two BsAb formats (1 and 2) are shown in Figure 19A.

Tumour cells (target cells) were pre-labelled with Oregon Green and cultured in 96-well half-area flat-bottom plate overnight for cell adhesion. The following day, primary T cells isolated from human PBMCs were added with Effector to Target ratio 10 to 1 (E:T=10:1). BsAbs, with 10-fold titration for 8 points, were also prepared and added to each well to reach to the indicated final concentration. The plate was then incubated in 37C, 5% CO2, for 3 days. For FACS readout, tumour cells were gently detached with Accutase and stained with Propidium iodide (PI) to label the dead cells. The BsAb-induced cytotoxicity was measured with FACS readout after 3-day coculture using MACSQuant Analyzer. % cytotoxicity equals to the cell count of dead cells divided by the cell count of target cells.

The results are shown in Figure 19B. In HLA-A24+/p53 mutant+ cell line HT29, target-specific cytotoxicity is observed with both bispecific constructs. BsAb2 (EC50=0.48nM; left hand curve) shows higher potency than BsAb1 (EC50=3.05nM; right hand curve). On the other hand, no non-specific cytotoxicity is observed in both constructs in the cell killing of HLA-A24+/p53null cell line SaOS2, showing the specificity of the bispecific constructs for targeting the p53-A24 complex.

The following numbered clauses, describing aspects of our proposals, are part of the description
1. An antibody or antigen binding fragment, optionally isolated, which is capable of binding to a peptide-MHC complex comprising a peptide of p53 and an MHC class I molecule.
2. The antibody or antigen binding fragment according to clause 1, wherein the MHC class I molecule comprises an MHC class I α-chain encoded by an HLA-A*24 allele.
3. The antibody or antigen binding fragment according to clause 1 or clause 2, wherein the peptide of p53 comprises, or consists of, the amino acid sequence of SEQ ID NO:75, or a variant having thereof having one or two or three amino acid substitutions in the amino acid sequence.
4. The antibody or antigen binding fragment according to any one of clauses 1 to 3, comprising the amino acid sequences i) to vi):

| | |
|---|---|
| i) LC-CDR1: | X₁GSX₂SNIGX₃X₄YX₅X₆X₇ (SEQ ID NO:46); |
| | TGTSSDVGGYNYVS (SEQ ID NO:29); or |
| | RASQSIGTDLA (SEQ ID NO:21); |
| ii) LC-CDR2: | GNX₈NRPS (SEQ ID NO:47); |
| | DASNRAT (SEQ ID NO:22); or |
| | DVSSRPS (SEQ ID NO:30) |
| iii) LC-CDR3: | QSYDSX₉LSX_{1O}X₁₁WV (SEQ ID NO:48); |
| | QQRSNWPPT (SEQ ID NO:23); or |
| | SSYTVFSTLV (SEQ ID NO:31); |
| iv) HC-CDR1: | SGGYYWX₁₂ (SEQ ID NO:49); or |
| | X₁₃YYX₁₄H (SEQ ID NO:50); |
| v) HC-CDR2: | YIYYSGX₁₅TYYNPSLKS (SEQ ID NO:51); or |
| | WX₁₆X₁₇PX₁₈SX₁₉X₂₀TX_{2'}YAQKFQG (SEQ ID NO:52); |
| vi) HC-CDR3: | ENFGX₂₂X₂₃DX₂₄ (SEQ ID NO:53); |
| | EGADGIYYFDY (SEQ ID NO:39); or |
| | DTYGHDY (SEQ ID NO:45); |

or a variant thereof in which one or two or three amino acids in one or more of the sequences i) to vi) are replaced with another amino acid;
wherein X₁ = T or A, X₂ = S or Y, X₃ = A or D, X₄ = G or D, X₅ = D or E, X₆ = V or T, X₇ = H or N, X₈ = N or T, X₉ = N or S, X₁₀ = Absent or D, X₁₁ = A or T, X₁₂ = S or A, X₁₃ = G or D, X₁₄ = M or I, X₁₅ = S or T, X₁₆ = I or M, X₁₇ = N or S, X₁₈ = N or D, X₁₉ = A or G, X₂₀ = G or A, X₂₁ = N or Y, X₂₂ = A or S, X₂₃ = For Y, and X₂₄ = H or Y.
5. The antibody or antigen binding fragment according to clause 4, wherein LC-CDR1 is one of TGSSSNIGADYETH (SEQ ID NO:17), AGSYSNIGDDYETH (SEQ ID NO:20), TGSSSNIGAGYDVH (SEQ ID NO:24), TGSSSNIGAGYDVN (SEQ ID NO:27), TGTSSDVGGYNYVS (SEQ ID NO:29) or RASQSIGTDLA (SEQ ID NO:21).
6. The antibody or antigen binding fragment according to clause 4 or clause 5, wherein LC-CDR2 is one of GNTNRPS (SEQ ID NO:18), GNNNRPS (SEQ ID NO:25), DASNRAT (SEQ ID NO:22) or DVSSRPS (SEQ ID NO:30).
7. The antibody or antigen binding fragment according to any one of clauses 4 to 6, wherein LC-CDR3 is one of QSYDSNLSAWV (SEQ ID NO:19), QSYDSNLSDTWV (SEQ ID NO:26), QSYDSSLSAWV (SEQ ID NO:28), QQRSNWPPT (SEQ ID NO:23) or SSYTVFSTLV (SEQ ID NO:31).
8. The antibody or antigen binding fragment according to any one of clauses 4 to 7, wherein HC-CDR1 is one of SGGYYWS (SEQ ID NO:32), SGGYYWA (SEQ ID NO:35), SGGYYWS (SEQ ID NO:40), GYYMH (SEQ ID NO:37), or DYYIH (SEQ ID NO:43).
9. The antibody or antigen binding fragment according to any one of clauses 4 to 8, wherein HC-CDR2 is one of YIYYSGSTYYNPSLKS (SEQ ID NO:33), YIYYSGTTYYNPSLKS (SEQ ID NO:41), WINPNSAGTNYAQKFQG (SEQ ID NO:38) or WMSPDSGATYYAQKFQG (SEQ ID NO:44).
10. The antibody or antigen binding fragment according to any one of clauses 4 to 9, wherein HC-CDR3 is one of ENFGAFDH (SEQ ID NO:34), ENFGSYDY (SEQ ID NO:36), EGADGIYYFDY (SEQ ID NO:39), or DTYGHDY (SEQ ID NO:45).
11. The antibody or antigen binding fragment according to any one of clauses 1 to 10, having at least one light chain variable region incorporating the following CDRs:

| | |
|---|---|
| LC-CDR1: | TGSSSNIGADYETH (SEQ ID NO:17) |
| LC-CDR2: | GNTNRPS (SEQ ID NO:18) |
| LC-CDR3: | QSYDSNLSAWV (SEQ ID NO:19); |

or

| | |
|---|---|
| LC-CDR1: | AGSYSNIGDDYETH (SEQ ID NO:20) |
| LC-CDR2: | GNTNRPS (SEQ ID NO:18) |
| LC-CDR3: | QSYDSNLSAWV (SEQ ID NO:19); |

or

| | |
|---|---|
| LC-CDR1: | RASQSIGTDLA (SEQ ID NO:21) |
| LC-CDR2: | DASNRAT (SEQ ID NO:22) |
| LC-CDR3: | QQRSNWPPT (SEQ ID NO:23); |

or

| | |
|---|---|
| LC-CDR1: | TGSSSNIGAGYDVH (SEQ ID NO:24) |
| LC-CDR2: | GNNNRPS (SEQ ID NO:25) |
| LC-CDR3: | QSYDSNLSDTWV (SEQ ID NO:26); |

or

| | |
|---|---|
| LC-CDR1: | TGSSSNIGAGYDVN (SEQ ID NO:27) |
| LC-CDR2: | GNNNRPS (SEQ ID NO:25) |
| LC-CDR3: | QSYDSSLSAWV (SEQ ID NO:28); |

or

| | |
|---|---|
| LC-CDR1: | TGTSSDVGGYNYVS (SEQ ID NO:29) |
| LC-CDR2: | DVSSRPS (SEQ ID NO:30) |
| LC-CDR3: | SSYTVFSTLV (SEQ ID NO:31). |

12. The antibody or antigen binding fragment according to any one of clauses 1 to 11, having at least one heavy chain variable region incorporating the following CDRs:

| | |
|---|---|
| HC-CDR1: | SGGYYWS (SEQ ID NO:32) |
| HC-CDR2: | YIYYSGSTYYNPSLKS (SEQ ID NO:33) |
| HC-CDR3: | ENFGAFDH (SEQ ID NO:34); |

or

| | |
|---|---|
| HC-CDR1: | SGGYYWA (SEQ ID NO:35) |
| HC-CDR2: | YIYYSGSTYYNPSLKS (SEQ ID NO:33) |
| HC-CDR3: | ENFGAFDH (SEQ ID NO:34); |

or

| | |
|---|---|
| HC-CDR1: | SGGYYWS (SEQ ID NO:32) |
| HC-CDR2: | YIYYSGSTYYNPSLKS (SEQ ID NO:33) |
| HC-CDR3: | ENFGSYDY (SEQ ID NO:36); |

or

| | |
|---|---|
| HC-CDR1: | SGGYYWA (SEQ ID NO:35) |
| HC-CDR2: | YIYYSGSTYYNPSLKS (SEQ ID NO:33) |
| HC-CDR3: | ENFGSYDY (SEQ ID NO:36); |

or

| | |
|---|---|
| HC-CDR1: | GYYMH (SEQ ID NO:37) |
| HC-CDR2: | WINPNSAGTNYAQKFQG (SEQ ID NO:38) |
| HC-CDR3: | EGADGIYYFDY (SEQ ID NO:39); |

or

| | |
|---|---|
| HC-CDR1: | SGGYYWS (SEQ ID NO:40) |
| HC-CDR2: | YIYYSGTTYYNPSLKS (SEQ ID NO:41) |

| | |
|---|---|
| HC-CDR3: | ENFGAFDY (SEQ ID NO:42); |

or

| | |
|---|---|
| HC-CDR1: | DYYIH (SEQ ID NO:43) |
| HC-CDR2: | WMSPDSGATYYAQKFQG (SEQ ID NO:44) |
| HC-CDR3: | DTYGHDY (SEQ ID NO:45). |

13. An antibody or antigen binding fragment, optionally isolated, which is capable of binding to a peptide-MHC complex comprising a peptide of p53 and an MHC class I molecule, comprising a light chain and a heavy chain variable region sequence, wherein:
the light chain comprises a LC-CDR1, LC-CDR2, LC-CDR3, having at least 85% overall sequence identity to LC-CDR1: one of X₁GSX₂SNIGX₃X₄YX₅X₆X₇ (SEQ ID NO:46), TGTSSDVGGYNYVS (SEQ ID NO:29) or RASQSIGTDLA (SEQ ID NO:21); LC-CDR2: one of GNX₈NRPS (SEQ ID NO:47), DASNRAT (SEQ ID NO:22) or DVSSRPS (SEQ ID NO:30); LC-CDR3: one of QSYDSX₉LSX₁₀X₁₁WV (SEQ ID NO:48), QQRSNWPPT (SEQ ID NO:23) or SSYTVFSTLV (SEQ ID NO:31); and
the heavy chain comprises a HC-CDR1, HC-CDR2, HC-CDR3, having at least 85% overall sequence identity to HC-CDR1: one of SGGYYWX₁₂ (SEQ ID NO:49) or X₁₃YYX₁₄H (SEQ ID NO:50); HC-CDR2: one of YIYYSGX₁₅TYYNPSLKS (SEQ ID NO:51) or WX₁₆X₁₇PX₁₈SX₁₉X₂₀TX_{2'}YAQKFQG (SEQ ID NO:52); HC-CDR2: one of ENFGX₂₂X₂₃DX₂₄ (SEQ ID NO:53), EGADGIYYFDY (SEQ ID NO:39) or DTYGHDY (SEQ ID NO:45);
wherein X₁ = T or A, X₂ = S or Y, X₃ = A or D, X₄ = G or D, X₅ = D or E, X₆ = V or T, X₇ = H or N, X₈ = N or T, X₉ = N or S, X₁₀ = Absent or D, X₁₁ = A or T, X₁₂ = S or A, X₁₃ = G or D, X₁₄ = M or I, X₁₅ = S or T, X₁₆ = I or M, X₁₇ = N or S, X₁₈ = N or D, X₁₉ = A or G, X₂₀ = G or A, X₂₁ = N or Y, X₂₂ = A or S, X₂₃ = F or Y, and X₂₄ = H or Y.
14. An antibody or antigen binding fragment, optionally isolated, which is capable of binding to a peptide-MHC complex comprising a peptide of p53 and an MHC class I molecule, comprising a light chain and a heavy chain variable region sequence, wherein:
the light chain sequence has at least 85% sequence identity to the light chain sequence of one of SEQ ID NOs:1 to 7, and;
the heavy chain sequence has at least 85% sequence identity to the heavy chain sequence of one of SEQ ID NOs:8 to 16.
15. The antibody or antigen binding fragment according to any one of clauses 1 to 14, which displays antibody-dependent cell-mediated cytotoxicity (ADCC) to cells comprising or expressing peptide-MHC complex comprising a peptide of p53 and an MHC class I molecule.
16. The antibody or antigen binding fragment according to any one of clauses 1 to 15, which is internalised by cells comprising or expressing peptide-MHC complex comprising a peptide of p53 and an MHC class I molecule.
17. The antibody or antigen binding fragment according to any one of clauses 1 to 16, which is a fully human antibody or a fully human antibody fragment.
18. The antibody or antigen binding fragment according to any one of clauses 1 to 17, conjugated to a drug moiety or a detectable moiety.
19. The antibody or antigen binding fragment according to any one of clauses 1 to 18, further comprising an antibody or antigen binding fragment specific for a target other than a peptide-MHC complex.
20. The antibody or antigen binding fragment according to clause 19, wherein the target other than a peptide-MHC complex is an immune cell surface molecule.
21. A chimeric antigen receptor (CAR) comprising an antigen binding fragment according to any one of clauses 1 to 20.
22. An *in vitro* complex, optionally isolated, comprising an antibody, antigen binding fragment or CAR according to any one of clauses 1 to 21 bound to a peptide-MHC complex comprising a peptide of p53 and an MHC class I molecule.
23. A composition comprising the antibody, antigen binding fragment or CAR according to any one of clauses 1 to 21 and at least one pharmaceutically-acceptable carrier.
24. An isolated nucleic acid encoding the antibody, antigen binding fragment or CAR according to any one of clauses 1 to 21.
25. A vector comprising the nucleic acid of clause 24.
26. A cell comprising the nucleic acid according to clause 24 or the vector according to clause 25.
27. A method for making an antibody, antigen binding fragment or CAR according to any one of clauses 1 to 21, comprising culturing the cell of clause 26 under conditions suitable for the expression of the antibody or antigen binding fragment or CAR.
28. An antibody, antigen binding fragment, CAR, composition, nucleic acid, vector or cell according to any one of clauses 1 to 21, or 23 to 26 for use in therapy, or in a method of medical treatment.
29. An antibody, antigen binding fragment, CAR, composition, nucleic acid, vector or cell according to any one of clauses 1 to 21, or 23 to 26 for use in the treatment or prevention of a cancer.
30. Use of an antibody, antigen binding fragment, CAR, composition, nucleic acid, vector or cell according to any one of clauses 1 to 21, or 23 to 26 in the manufacture of a medicament for treating or preventing a cancer.
31. A method of treating or preventing a cancer, comprising administering to a subject a therapeutically or prophylactically effective amount of the antibody, antigen binding fragment, CAR, composition, nucleic acid, vector or cell according to any one of clauses 1 to 21, or 23 to 26.
32. A method of treating or preventing a cancer in a subject, comprising:
(a) isolating at least one cell from a subject;
(b) modifying the at least one cell to express or comprise the antibody, antigen binding fragment, CAR, nucleic acid or vector according to any one of clauses 1 to 21, or 24 to 26 and;
(c) administering the modified at least one cell to a subject.
33. A method of treating or preventing a cancer in a subject, comprising:
(a) isolating at least one cell from a subject;
(b) introducing into the at least one cell the nucleic acid according to clause 24 or the vector according to clause 25, thereby modifying the at least one cell and;
(c) administering the modified at least one cell to a subject.
34. A kit of parts comprising a predetermined quantity of the antibody, antigen binding fragment, CAR, composition, nucleic acid, vector or cell according to any one of clauses 1 to 21, or 23 to 26.
35. A method of diagnosing a disease or a condition in a subject, the method comprising contacting a sample containing, or suspected to contain, peptide-MHC complex with an antibody or antigen binding fragment according to any one of clauses 1 to 21 and detecting the formation of a complex of antibody, or antigen binding fragment, and the peptide-MHC complex.

## Claims

1. A chimeric antigen receptor (CAR) comprising an antigen-binding domain comprising an antibody or antigen-binding fragment which is capable of binding to a peptide-MHC complex comprising a peptide of p53 and an MHC class I molecule.

2. The CAR according to claim 1, wherein the MHC class I molecule comprises an MHC class I α-chain encoded by an HLA-A*24 allele.

3. The CAR according to claim 1 or claim 2, wherein the peptide of p53 comprises, or consists of, the amino acid sequence of SEQ ID NO:75, or a variant having thereof having one or two or three amino acid substitutions in the amino acid sequence.

4. The CAR according to any one of claims 1 to 3, wherein the antibody or antigen-binding fragment comprises the amino acid sequences i) to vi):
| | |
|---|---|
| i) LC-CDR1: | X₁GSX₂SNIGX₃X₄YX₅X₆X₇ (SEQ ID NO:46); |
| | TGTSSDVGGYNYVS (SEQ ID NO:29); or |
| | RASQSIGTDLA (SEQ ID NO:21); |
| ii) LC-CDR2: | GNX₈NRPS (SEQ ID NO:47); |
| | DASNRAT (SEQ ID NO:22); or |
| | DVSSRPS (SEQ ID NO:30) |
| iii) LC-CDR3: | QSYDSX₉LSX₁₀X₁₁VW (SEQ ID N0:48); |
| | QQRSNWPPT (SEQ ID NO:23); or |
| | SSYTVFSTLV (SEQ ID NO:31); |
| iv) HC-CDR1: | SGGYYWX₁₂ (SEQ ID NO:49); or |
| | X₁₃YYX₁₄H (SEQ ID NO:50); |
| v) HC-CDR2: | YIYYSGX₁₅TYYNPSLKS (SEQ ID NO:51 ); or |
| | WX₁₆X₁₇PX₁₈SX₁₉X₂₀TX₂₁YAQKFQG (SEQ ID NO:52); |
| vi) HC-CDR3: | ENFGX₂₂X₂₃DX₂₄ (SEQ ID NO:53); |
| | EGADGIYYFDY (SEQ ID NO:39); or |
| | DTYGHDY (SEQ ID NO:45); |
or a variant thereof in which one or two or three amino acids in one or more of the sequences i) to vi) are replaced with another amino acid;
wherein X₁ = T or A, X₂ = S or Y, X₃ = A or D, X₄ = G or D, X₅ = D or E, X₆ = V or T, X₇ = H or N, X₈ =N or T, X₉ =N or S, X₁₀ =Absent or D, X₁₁ =A or T, X₁₂ = S or A, X₁₃ = G or D, X₁₄ =M or I, X₁₅ =S or T, X₁₅ = I or M, X₁₁ =N or S, X₁₈ =N or D, X₁₉ =A or G, X₂₀ = G or A, X₂₁ = N or Y, X₂₂ = A or S, X₂₃ = F or Y, and X₂₄ = H or Y.

5. The CAR according to any one of claims 1 to 4, wherein the antibody or antigen-binding fragment comprises:
(i) at least one light chain variable region incorporating the following CDRs:
LC-CDR1: TGSSSNIGADYETH (SEQ ID NO:17)
LC-CDR2: GNTNRPS (SEQ ID NO:18)
LC-CDR3: QSYDSNLSAWV (SEQ ID NO:19); and
at least one heavy chain variable region incorporating the following CDRs:
HC-CDR1: SGGYYWS (SEQ ID NO:32)
HC-CDR2: YIYYSGSTYYNPSLKS (SEQ ID NO:33)
HC-CDR3: ENFGAFDH (SEQ ID NO:34);
or
(ii) at least one light chain variable region incorporating the following CDRs:
LC-CDR1: TGSSSNIGADYETH (SEQ ID NO:17)
LC-CDR2: GNTNRPS (SEQ ID NO:18)
LC-CDR3: QSYDSNLSAWV (SEQ ID NO:19); and
at least one heavy chain variable region incorporating the following CDRs:
HC-CDR1: SGGYYWA (SEQ ID NO:35)
HC-CDR2: YIYYSGSTYYNPSLKS (SEQ ID NO:33)
HC-CDR3: ENFGSYDY (SEQ ID NO:36);
or
(iii) at least one light chain variable region incorporating the following CDRs:
LC-CDR1: TGSSSNIGADYETH (SEQ ID NO:17)
LC-CDR2: GNTNRPS (SEQ ID NO:18)
LC-CDR3: QSYDSNLSAWV (SEQ ID NO:19); and
at least one heavy chain variable region incorporating the following CDRs:
HC-CDR1: SGGYYWA (SEQ ID NO:35)
HC-CDR2: YIYYSGSTYYNPSLKS (SEQ ID NO:33)
HC-CDR3: ENFGAFDH (SEQ ID NO:34);
or
(iv) at least one light chain variable region incorporating the following CDRs:
LC-CDR1: TGSSSNIGADYETH (SEQ ID NO:17)
LC-CDR2: GNTNRPS (SEQ ID NO:18)
LC-CDR3: QSYDSNLSAWV (SEQ ID NO:19); and
at least one heavy chain variable region incorporating the following CDRs:
HC-CDR1: SGGYYWS (SEQ ID NO:32)
HC-CDR2: YIYYSGSTYYNPSLKS (SEQ ID NO:33)
HC-CDR3: ENFGSYDY (SEQ ID NO:36);
or
(v) at least one light chain variable region incorporating the following CDRs:
LC-CDR1: AGSYSNIGDDYETH (SEQ ID NO:20)
LC-CDR2: GNTNRPS (SEQ ID NO:18)
LC-CDR3: QSYDSNLSAWV (SEQ ID NO:19); and
at least one heavy chain variable region incorporating the following CDRs:
HC-CDR1: SGGYYWS (SEQ ID NO:32)
HC-CDR2: YIYYSGSTYYNPSLKS (SEQ ID NO:33)
HC-CDR3: ENFGAFDH (SEQ ID NO:34);
or
(vi) at least one light chain variable region incorporating the following CDRs:
LC-CDR1: AGSYSNIGDDYETH (SEQ ID NO:20)
LC-CDR2: GNTNRPS (SEQ ID NO:18)
LC-CDR3: QSYDSNLSAWV (SEQ ID NO:19); and
at least one heavy chain variable region incorporating the following CDRs:
HC-CDR1: SGGYYWA (SEQ ID NO:35)
HC-CDR2: YIYYSGSTYYNPSLKS (SEQ ID NO:33)
HC-CDR3: ENFGSYDY (SEQ ID NO:36);
or
(vii) at least one light chain variable region incorporating the following CDRs:
LC-CDR1: RASQSIGTDLA (SEQ ID NO:21)
LC-CDR2: DASNRAT (SEQ ID NO:22)
LC-CDR3: QQRSNWPPT (SEQ ID NO:23); and
at least one heavy chain variable region incorporating the following CDRs:
HC-CDR1: GYYMH (SEQ ID NO:37)
HC-CDR2: WINPNSAGTNYAQKFQG (SEQ ID NO:38)
HC-CDR3: EGADGIYYFDY (SEQ ID NO:39);
or
(viii) at least one light chain variable region incorporating the following CDRs:
LC-CDR1: TGSSSNIGAGYDVH (SEQ ID NO:24)
LC-CDR2: GNNNRPS (SEQ ID NO:25)
LC-CDR3: QSYDSNLSDTWV (SEQ ID NO:26); and
at least one heavy chain variable region incorporating the following CDRs:
HC-CDR1: SGGYYWS (SEQ ID NO:40)
HC-CDR2: YIYYSGTTYYNPSLKS (SEQ ID NO:41)
HC-CDR3: ENFGAFDY (SEQ ID NO:42);
or
(ix) at least one light chain variable region incorporating the following CDRs:
LC-CDR1: TGSSSNIGAGYDVN (SEQ ID NO:27)
LC-CDR2: GNNNRPS (SEQ ID NO:25)
LC-CDR3: QSYDSSLSAWV (SEQ ID NO:28); and
at least one heavy chain variable region incorporating the following CDRs:
HC-CDR1: SGGYYWS (SEQ ID NO:32)
HC-CDR2: YIYYSGSTYYNPSLKS (SEQ ID NO:33)
HC-CDR3: ENFGAFDH (SEQ ID NO:34);
or
(x) at least one light chain variable region incorporating the following CDRs:
LC-CDR1: TGTSSDVGGYNYVS (SEQ ID NO:29)
LC-CDR2: DVSSRPS (SEQ ID NO:30)
LC-CDR3: SSYTVFSTLV (SEQ ID NO:31); and
at least one heavy chain variable region incorporating the following CDRs:
HC-CDR1: DYYIH (SEQ ID NO:43)
HC-CDR2: WMSPDSGATYYAQKFQG (SEQ ID NO:44)
HC-CDR3: DTYGHDY (SEQ ID NO:45).

6. The CAR according to any one of claims 1 to 5, wherein the antibody or antigen-binding fragment comprises:
the light chain variable region sequence has at least 85% sequence identity to the light chain variable region sequence of one of SEQ ID NOs:1 to 7; and
the heavy chain variable region sequence has at least 85% sequence identity to the heavy chain variable region sequence of one of SEQ ID NOs:8 to 16.

7. The CAR according to any one of claims 1 to 6, wherein the antibody or antigen-binding fragment comprises:
(i) the light chain variable region sequence has at least 85% sequence identity to the light chain sequence of SEQ ID NO:1; and
the heavy chain variable region sequence has at least 85% sequence identity to the heavy chain sequence of SEQ ID NO:8;
or
(ii) the light chain variable region sequence has at least 85% sequence identity to the light chain sequence of SEQ ID NO:2; and
the heavy chain variable region sequence has at least 85% sequence identity to the heavy chain sequence of SEQ ID NO:9;
or
(iii) the light chain variable region sequence has at least 85% sequence identity to the light chain sequence of SEQ ID NO:2; and
the heavy chain variable region sequence has at least 85% sequence identity to the heavy chain sequence of SEQ ID NO:12;
or
(iv) the light chain variable region sequence has at least 85% sequence identity to the light chain sequence of SEQ ID NO:2; and
the heavy chain variable region sequence has at least 85% sequence identity to the heavy chain sequence of SEQ ID NO:10;
or
(v) the light chain variable region sequence has at least 85% sequence identity to the light chain sequence of SEQ ID NO:2; and
the heavy chain variable region sequence has at least 85% sequence identity to the heavy chain sequence of SEQ ID NO:11;
or
(vi) the light chain variable region sequence has at least 85% sequence identity to the light chain sequence of SEQ ID NO:3; and
the heavy chain variable region sequence has at least 85% sequence identity to the heavy chain sequence of SEQ ID NO:9;
or
(vii) the light chain variable region sequence has at least 85% sequence identity to the light chain sequence of SEQ ID NO:3; and
the heavy chain variable region sequence has at least 85% sequence identity to the heavy chain sequence of SEQ ID NO:12;
or
(viii) the light chain variable region sequence has at least 85% sequence identity to the light chain sequence of SEQ ID NO:4; and
the heavy chain variable region sequence has at least 85% sequence identity to the heavy chain sequence of SEQ ID NO:13;
or
(ix) the light chain variable region sequence has at least 85% sequence identity to the light chain sequence of SEQ ID NO:5; and
the heavy chain variable region sequence has at least 85% sequence identity to the heavy chain sequence of SEQ ID NO:14;
or
(x) the light chain variable region sequence has at least 85% sequence identity to the light chain sequence of SEQ ID NO:6; and
the heavy chain variable region sequence has at least 85% sequence identity to the heavy chain sequence of SEQ ID NO:15;
or
(xi) the light chain variable region sequence has at least 85% sequence identity to the light chain sequence of SEQ ID NO:7; and
the heavy chain variable region sequence has at least 85% sequence identity to the heavy chain sequence of SEQ ID NO:16.

8. The CAR according to any one of claims 1 to 7, further comprising an antibody or antigen-binding fragment specific for a target other than a peptide-MHC complex.

9. The CAR according to claim 8, wherein the target other than a peptide-MHC complex is an immune cell surface molecule.

10. A nucleic acid encoding the CAR according to any one of claims 1 to 9.

11. A cell comprising the CAR according to any one of claims 1 to 9, or the nucleic acid according to claim 10.

12. The cell according to claim 11, for use in the treatment or prevention of a cancer.

13. The cell for use according to claim 12, wherein the cancer comprises cells expressing a peptide of p53 and an MHC class I molecule.

14. The cell for use according to claim 13, wherein the MHC class I molecule comprises an MHC class I α-chain encoded by an HLA-A*24 allele.

15. The cell for use according to claim 13 or claim 14, wherein the peptide of p53 comprises, or consists of, the amino acid sequence of SEQ ID NO:75, or a variant having thereof having one or two or three amino acid substitutions in the amino acid sequence.
